# EUROPEAN PATENT APPLICATION

(11) **EP 2 314 320 A2**
(43) Date of publication of application: **27.04.2011**
(21) Application number: 10183132.9
(22) Date of filing: 31.03.2006
(51) Int. Cl.: A61K 47/48, C07K 14/16

(54) **Method for shielding functional sites or epitopes on proteins**

(30) Priority: 05.04.2005 US 668354 P
(62) Divisional of application: 06724272.7
(71) Applicant: Istituto di Ricerche di Biologia Molecolare P. Angeletti S.p.A., 00040 Pomezia (Rome) (IT)
(72) Inventor: Bianchi, Elisabetta, i-00040, Rome (IT); Pessi, Antonello, i-00040, Rome (IT)
(74) Representative: Horgan, James Michael Frederic

(57) **Abstract**

Methods of site-specifically shielding one or more binding sites within a polypeptide are disclosed, comprising attaching at least one small molecular weight, water-soluble polymer to said polypeptide such that the binding site is masked by said polymer. The shielding of a binding site (*e.g*., epitope) as per the disclosed methods acts to either eliminate or substantially reduce the biological response induced by the interaction between said binding site and its cognate receptor, helping to refocus the biological response toward unmasked portions of the polypeptide. Pharmaceutical products generated as per the methods described herein (*e.g*., polymer-modified antigens and vaccine compositions comprising them), as well as the use thereof, induce a specific immune response against unmasked portions of the polypeptides when directly introduced into living vertebrate tissue, preferably a mammalian host such as a human or a non-human mammal of commercial or domestic veterinary importance, generating selective immunoprotection in said mammal.

## Description

### CROSS-REFERENCE TO RELATED APPLICATIONS

The present application claims the benefit of U.S. Provisional Application No. 60/668,354, filed April 5, 2005, hereby incorporated by reference herein.

### FIELD OF THE INVENTION

The present invention relates to a method of shielding one or more binding sites within a polypeptide comprising site-specifically attaching at least one small molecular weight, water-soluble polymer to said polypeptide such that the binding site is selectively masked by said polymer. In one embodiment, the masked binding site is an epitope within a polypeptide antigen. The shielding of a binding site (*e.g.*, epitope) as per the disclosed methods acts to either eliminate or substantially reduce the biological response induced by the interaction between said binding site and its cognate receptor, helping to refocus the biological response toward unmasked portions of the polypeptide. The present invention further relates to pharmaceutical products generated as per the methods described herein (*e.g.*, polymer-modified antigens and vaccine compositions comprising them), as well as the use thereof, which when directly introduced into living vertebrate tissue, preferably a mammalian host such as a human or a non-human mammal of commercial or domestic veterinary importance, induce a specific immune response against unmasked portions of the polypeptides, generating selective immunoprotection in said mammal.

### BACKGROUND OF THE INVENTION

The natural occurrence of selectively masking, or "shielding," a biologically active site or domain (*e.g.*, an immunogenic epitope) within a protein has been described as a strategy used by pathogens to subvert host immune responses. For example, in the hemagglutinin (HA) of influenza virus, the epitope of a monoclonal antibody is shielded by an N-linked oligosaccharide chain (Wiley, D.C. et al., 1981, Nature 289:373-8). Skehel and co-workers showed that growth of the virus in the presence of the antibody selected for variants whose HA bears an additional N-linked oligosaccharide in close proximity to the antibody epitope (1984, Proc. Natl. Acad. Sci. U.S.A. 81:1779-83). Occlusion of critical epitopes by glycosylation as a mechanism of evading neutralization by the host immune system is also well described for the gp120 subunit of the envelope glycoprotein of HIV (see, *e.g.*, Burton, D.R. et al., 2004, Nat. Immunol. 5:233-6; Johnson, W.E. and R.C. Desrosiers, 2002, Annu. Rev. Med. 53:499-518; Kwong, P.D. et al., 1998, Nature 393:648-59; and, Wei, X. et al., 2003, Nature 422:307-12); while the same effect is achieved for other HIV epitopes by conformational masking (Kwong, P.D. et al., 2002, Nature 420:678-82). This mechanism of "immune escape" often results in the chronic presentation of immunodominant epitopes which act as decoys, keeping the immune system from focusing on more protective epitopes that would otherwise ensure optimal protection ("neutralizing" epitopes). Ultimately, this mechanism may also contribute to limited vaccine efficacy.

Carbohydrates can also mask non-antigenic receptor binding sites. For example, Goto, H. and K. Kawaoka (1998, Proc. Natl. Acad. Sci. U.S.A. 95:10224-8) have proposed the masking of a specific location in the influenza viral neuraminidase protein by an oligosaccharide side chain, preventing binding of plasminogen to the protein. Since plasminogen is required to cleave the spatially contiguous viral hemagglutinin, making it fusion competent, the carbohydrate side chain profoundly alters infectivity of the virus.

Polymer shielding is a widely used method to reduce the immunogenicity of pharmaceutical proteins, ultimately improving their therapeutic effect by helping to minimize undesired immunogenic side effects and decreasing clearance via kidney filtration (for a review, see Schellekens, H., 2002, Clinical Therapeutics 24:1720-40). A widely applied method for shielding therapeutic proteins is the attachment of high molecular weight ("HMW"), linear or branched, polyethylene glycol ("PEG") chains to said proteins (Harris, J.M. and R.B. Chess, 2003, Nat. Rev. Drug Discov. 2:214-21; U.S. Patent No. 4,179,337 issued to Davis et al.). Since the desired effect is a global masking of the protein, the size of the PEG moieties used to modify the therapeutic protein is usually comparable to the size of the protein to which they are attached.

More recently, the notion of protein shielding in a selected, epitope-specific manner has been proposed, termed "immunofocusing." One result of this strategy is the selective shielding of an immunodominant yet non-neutralizing epitope within a protein antigen or immunogen, directing and/or re-focusing immune responses to more neutralizing epitopes (see Burton, D.R. et al., 2004, Nat. Immunol. 5:233-6; Delves, P.J. et al., 1997, Mol. Med Today 3:55-60; and, Pantophlet, R. and D.R. Burton, 2003, Trends Mol. Med. 9:468-73). This method was developed to circumvent the complex immune-evading strategies evolved to favor persistent pathogenic infections, as described above. In a particular example, Garrity, R.R. et al. incorporated an extra N-linked glycosylation site in the V3 hypervariable loop of.HIV gp120, causing a considerable shift in the antibody response to said protein (1997, J. Immunol. 159:279-89). A more systematic study performed by Pantophlet, R. et al. showed that the addition of seven N-glycosylation motifs within monomeric gp120 abolished binding to a broad panel of non-neutralizing antibodies, while binding to a neutralizing monoclonal antibody, mAB b12, was retained with reduced efficiency (Pantophlet, R. and D.R. Burton, 2003, supra 9:468-73; Pantophlet, R. et al., 2003, J. Virol. 77:5889-901). Importantly, however, glycosylation will not always abrogate T-cell recognition. Indeed, the binding of glycosylated peptides by T-cells is well documented (see, *e.g.*, Cudic, M. et al., 2002, Bioorg. Med. Chem. 10:3859-70; Glithero, A.J. et al., 1999, Immunity 10:63-74; Haurum, J.S. et al., 1999, J. Exp. Med. 190:145-50; Jackson, D.C. et al., 1994, Virology 199:422-30; and, Rudd, P.M. et al., 2001, Science 291:2370-6), especially if the carbohydrate side chain is small (Cudic et al, 2002, *supra;* Jackson et al., 1994, *supra).* Thus, the present invention relates to the selective shielding of a binding site of a receptor within a peptide or protein via the attachment of a small molecular weight ("SMW") polymer moiety other than a carbohydrate side chain, including but not limited to SMW PEG moieties (*i.e.*, "PEGylation"), to said peptide/protein either within or in the proximity of said binding site.

U.S. Patent No. 5,041,376, issued to Gething et al. on Aug. 20, 1991, discloses a general method for shielding epitopes of proteins by incorporating supernumeracy N-linked oligosaccharide side chains at site-specific locations using oligonucleotide mutagenesis.

U.S. Patent Nos. 5,585,250 and 5,853,724, issued to Garrity et al. on Dec. 17, 1996 and Dec. 29, 1998, respectively, disclose a method of epitope dampening the V3 loop ofMV-1 gp120/160 to redirect a neutralizing antibody response away from said V3 loop and toward more neutralizing portion of the protein.

### SUMMARY OF THE INVENTION

The present invention relates to methods of shielding one or more binding sites within a polypeptide comprising site-specifically attaching at least one small molecular weight ("SMW"), water-soluble polymer to said polypeptide such that the binding site is selectively masked by said polymer. In one embodiment of the present invention, the binding site (*i.e.*, functional site) that is shielded as per the methods described herein is an immunogenic or antigenic epitope within a polypeptide antigen. The masking of selected binding sites (*e.g.*, epitopes) helps to eliminate or suppress unwanted biological responses (*e.g.*, immune reactivity) induced by the interaction between said binding sites and their cognate receptors, refocusing the biological response to unmasked portions of said polymer-modified polypeptide. Thus, the present invention relates to a method of shielding a binding site or epitope of a peptide or polypeptide comprising site-specifically attaching at least one SMW water-soluble polymer to said peptide/polypeptide such that the binding site/epitope is selectively masked by said polymer, wherein the peptide/polypeptide contains one or more binding sites/epitopes. The SMW water-soluble polymer is attached to the polypeptide at a location (*e.g.*, an amino acid) either within or in close proximity to the binding site/epitope to be shielded. In one embodiment of the present invention, said small molecular weight polymer used to selectively shield a binding site within a polypeptide by site-specific attachment to said polypeptide is selected from the group consisting of polyalkylene oxide, polyamide alkylene oxide and derivatives thereof. In a further embodiment, said small molecular weight, water-soluble polymer is PEG.

The present invention further relates to a methods of immunodampening an immunodominant and/or unfavorable epitope present within a polypeptide antigen in efforts to direct a more favorable response (*e.g.*, a neutralizing response) to an alternative epitope within said protein/peptide, wherein said immunodominant and/or unfavorable epitope is selectively masked by at least one small molecular weight, water-soluble polymer attached to said antigen. Thus, the methods described herein may be used to focus the immune response toward one or more unmasked epitopes and away from the masked epitopes which have been rendered largely unrecognizable by immune-derived cells or proteins. Said small molecular weight polymers used to selectively shield an unfavorable epitope within an antigen by site-specific attachment to said antigen can be selected from the group consisting of polyalkylene oxide, polyamide alkylene oxide and derivatives thereof. In a further embodiment, said small molecular weight, water-soluble polymer is PEG.

One particular embodiment of the present invention relates to a method of shielding an epitope of a polypeptide antigen comprising site-specifically attaching at least one SMW water-soluble polymer, including but not limited to SMW PEG, to at least one amino acid residue within said antigen such that the epitope is selectively masked by said polymer, wherein said epitope is located within a scaffold domain of a covalently-stabilized, trimeric coiled-coil structure that mimics all or a portion of an internal, trimeric coiled-coil motif of an enveloped virus membrane-fusion protein. Said coiled-coil structure comprises three chimeric peptides covalently-stabilized by one or more covalent bonds between said peptides, each chimeric peptide comprising a scaffold domain which comprises a soluble, trimeric form of a coiled-coil fused in helical phase to all or a portion of a NH₂-terminal heptad repeat domain, or a modified form thereof, of said enveloped virus membrane-fusion protein. In a specific embodiment of this portion of the present invention, said enveloped virus membrane-fusion protein is HIV gp41.

Therefore, one embodiment of the present invention relates to a method of shielding an epitope of a covalently-stabilized, trimeric coiled-coil structure that mimics all or a portion of an internal, trimeric coiled-coil motif of HIV gp41 comprising site-specifically attaching at least one small molecular weight, water-soluble polymer to said coiled-coil structure such that said epitope is selectively masked by said polymer. In a further embodiment, the masked epitope is located within the scaffold domain of the coiled-coil structure, wherein said coiled-coil structure comprises three chimeric peptides, each having a "scaffold domain" which comprises a soluble, trimeric form of a coiled-coil that is fused in helical phase to all or a portion of an HIV gp41 NH₂-terminal heptad repeat domain, or a modified form thereof, and wherein said peptides are covalently-stabilized by one or more covalent bonds, including but not limited to disulfide bonds or an any covalent bond resulting from a chemoselective ligation reaction.

The present invention also relates to the polymer-modified peptides or proteins generated as per the methods described herein, pharmaceutical/vaccine compositions that contain them, and methods of immunizing an animal, including but not limited to a mammal, against a disease condition with said pharmaceutical/vaccine compositions to induce selective immunoprotection in said mammal.

Therefore, one embodiment of the present invention relates to chimeric peptides that are polymer-modified as per the methods described herein, wherein said peptides can be covalently-stabilized to generate trimeric coiled-coil structures and comprise an alpha("α")-helical domain comprising an α-helical scaffold protein capable of forming a trimeric coiled-coil (the "scaffold" portion) fused in helical phase to all or a portion of a NH₂-terminal heptad repeat domain of an enveloped virus membrane-fusion protein (the "N-peptide" portion), including but not limited to HTV gp41. It is this scaffold domain, containing at least one or more epitopes, which is site-specifically shielded via the attachment of one or more small molecular weight polymers, including but not limited to PEG, to selected amino acid residues located within the scaffold domain sequence. The present invention further relates to the covalently-stabilized, trimeric coiled-coil structures which are comprised of the polymer-modified chimeric peptides generated as per the methods described herein, wherein said polymer-modified coiled-coils include but not limited to (CCIPNI7)₃, C(thioEPN17)₃ and C(thioEP17GluN17)₃.

The present invention further relates to a pharmaceutical product (*e.g.*, vaccine), as well as the production and use thereof, which can be directly introduced into living vertebrate tissue, preferably a mammalian host such as a human or a non-human mammal of commercial or domestic veterinary importance, to induce an immune response which specifically recognizes a particular disease or condition (including but not limited to human immunodeficiency virus-1 infection/A>DS), wherein said pharmaceutical products and/or antigenic conjugates thereof comprise a polymer-modified polypeptide antigen generated by the methods described herein.

As used herein, "HIV" is meant to represent either HIV-1, HIV-2, or HIV-1 and/or HIV-2.

As used herein, "neutralizing" is used as in the art, namely to denote the ability of an antibody to prevent viral infection in an *in vitro* cell/virus-based assay. Neutralizing activity may be measured quantitatively as the IC₅₀ value for that specific antibody. A "neutralizing antibody" or a "HIV neutralizing antibody" can be shown in an art accepted infectivity assay to neutralize at least one HIV isolate.

As used herein, "PEG" refers to - polyethylene glycol -.

As used herein, "SMW" refers to - small molecular weight -; "HMW" refers to - high molecular weight -; and, "MW" refers to - molecular weight -.

As used herein, "SPPS" refers to - Solid-Phase Peptide Synthesis -.

As used herein, the term "Michael acceptor" refers to any chemical moiety that contains a polarized, electrophilic carbon-carbon double bond that is able to react with a nucleophilic species (*e.g.*, O⁻, C⁻, or S⁻).

### BRIEF DESCRIPTION OF THE DRAWINGS

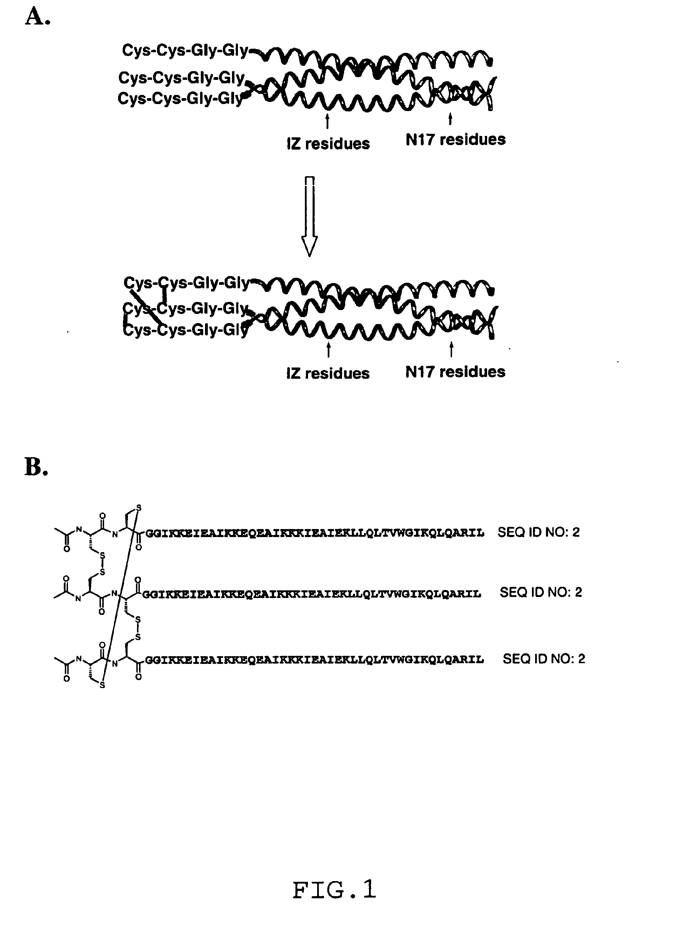

Figure 1A shows a schematic representation of the oxidation reaction yielding the covalently-stabilized, homotrimeric coiled-coil (CCIZN17)₃. There are three disulfide bonds stabilizing the trimeric coiled-coil, one bond between each peptide chain. Figure 1B shows a chemical, structural representation of the three disulfide bonds stabilizing the (CCIZN17)₃ ([SEQ ID NO:2]₃) trimeric coiled-coil. The three disulfide bonds are located between thiol (-SH) groups of the NH₂-terminal cysteine amino acid residues, the chemical structure of which is depicted. The amino acids located downstream of the two cysteine residues are in bold with single letter nomenclature.

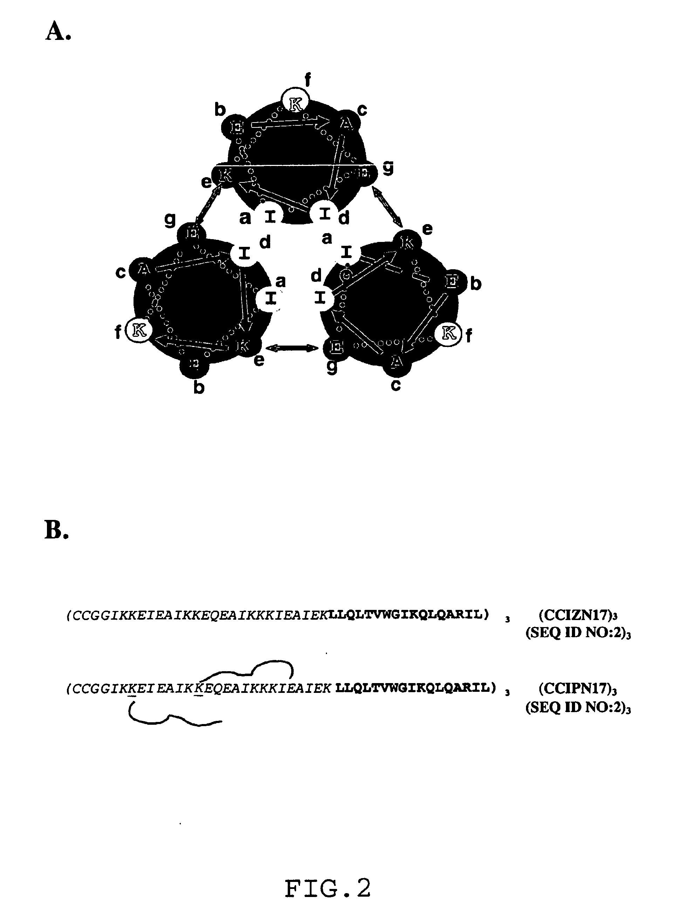

Figure 2A shows a helical wheel representation of the trimeric "IZ" scaffold. The three lysine amino acids ("K") located at the "f" positions of the heptad repeat are outlined, residing in more external positions of the coiled-coil. Two of these lysine residues were selected for derivatization at the ε-amino group of the side chain with m-dPEG™ acid (MW=236) to generate the selectively-shielded trimeric coiled-coil (CCIPN17)₃, as seen in the sequence representation of (CCIPN17)₃ in Figure 2B. The PEGylated lysine residues in the IZ scaffold domain (italicized) are underlined. The HTV N17 domain is bolded.

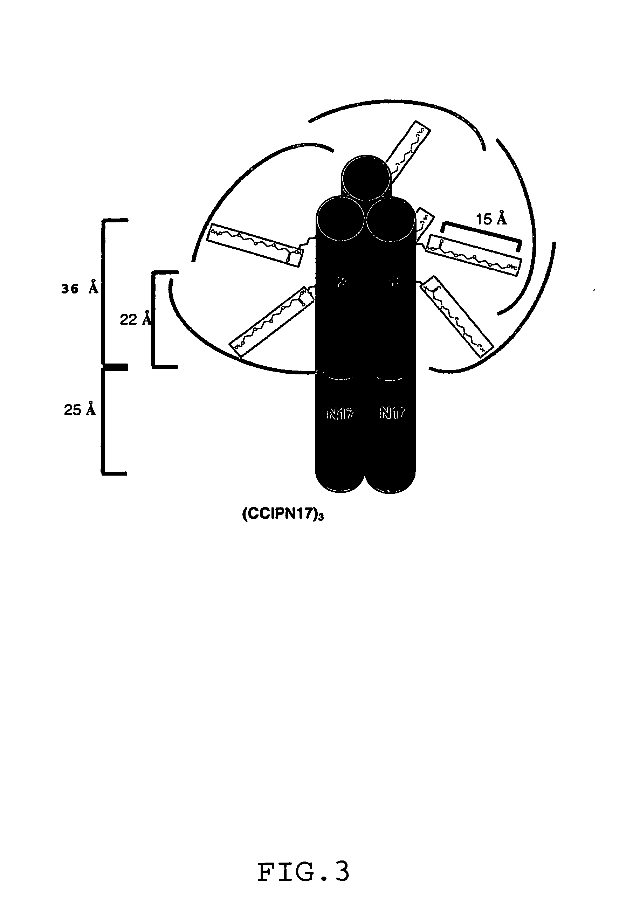

Figure 3 shows a schematic model of the (CCIPN17)₃ peptide vaccine. The individual chimeric peptides, CCIPN17, are represented as cylinders of approximately 62 Å in length. The dark gray portion represents the IZ scaffold domain, about 36 Å long, while the light gray portion represents the N17 region of the HIV gp41 N-peptide, about 25 Å long. The m-dPEG™ acid (MW=236) moieties are represented as rectangles of about 15 Å in length.

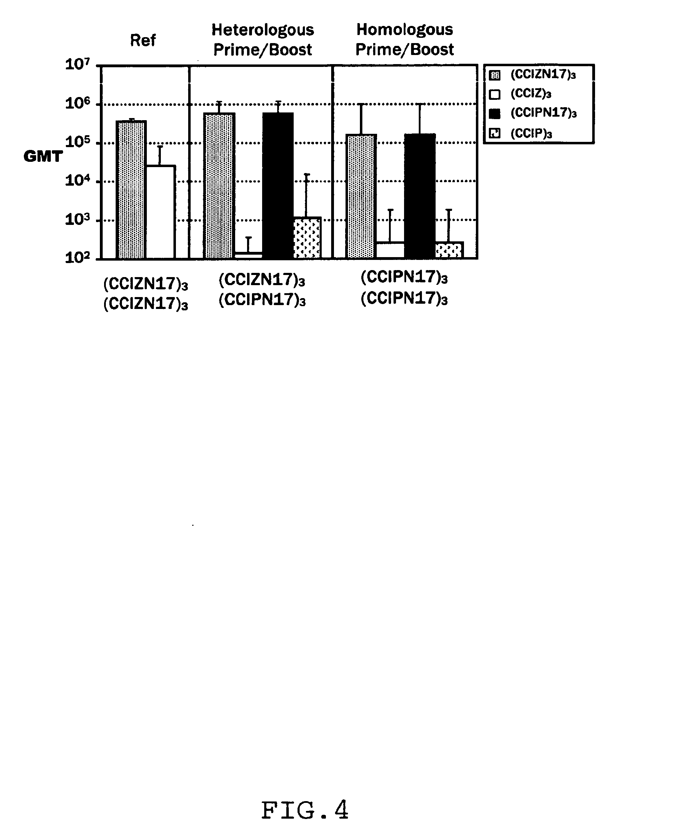

Figure 4 shows ELISA results from guinea pig studies in performed under homologous and heterologous Prime/Boost immunization regimens with (CCIPN17)₃. The Prime/Boost pair is shown below each group of bars. The ELISA geometric mean titers (GMT) against each antigen are a true reflection of the relative amount of antibodies with the corresponding specificity present in the serum.

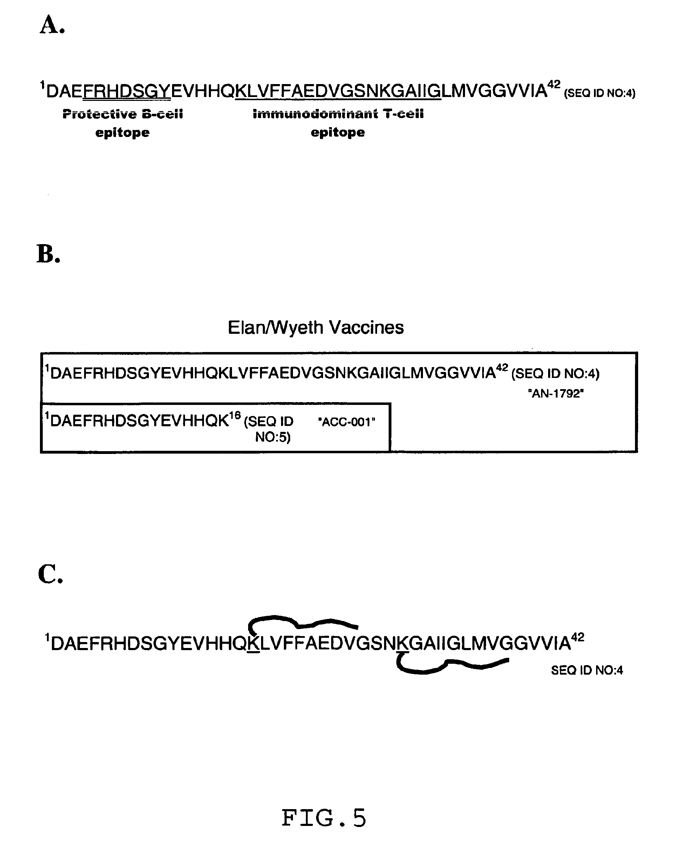

Figure 5A shows the amino acid sequence of the amyloid β-peptide. (Aβ) 1-42 peptide. An immunodominant T-cell epitope (Monsogeo, A. et al., 2003, J. Clin. Invest. 112:415-22) is single underlined, while a protective B-cell epitope (McLaurin, J. et al., 2002, Nat. Med. 8:1263-9) is double underlined. Figure 5B shows the amino acid sequences of two Aβ peptide vaccines from Elan/Wyeth, AN-1792 (Aβ 1-42) and ACC-001 ((Aβ 1-16). Figure 5C shows a schematic representation of a proposed Aβ 1-42 peptide vaccine wherein two lysine amino acid residues (underlined) are derivatized with m-dPEG™ acid (MW=236) moieties in order to selectively shield the immunodominant T-cell epitope in favor of the protective B-cell epitope in the NH₂-terminal portion of the peptide.

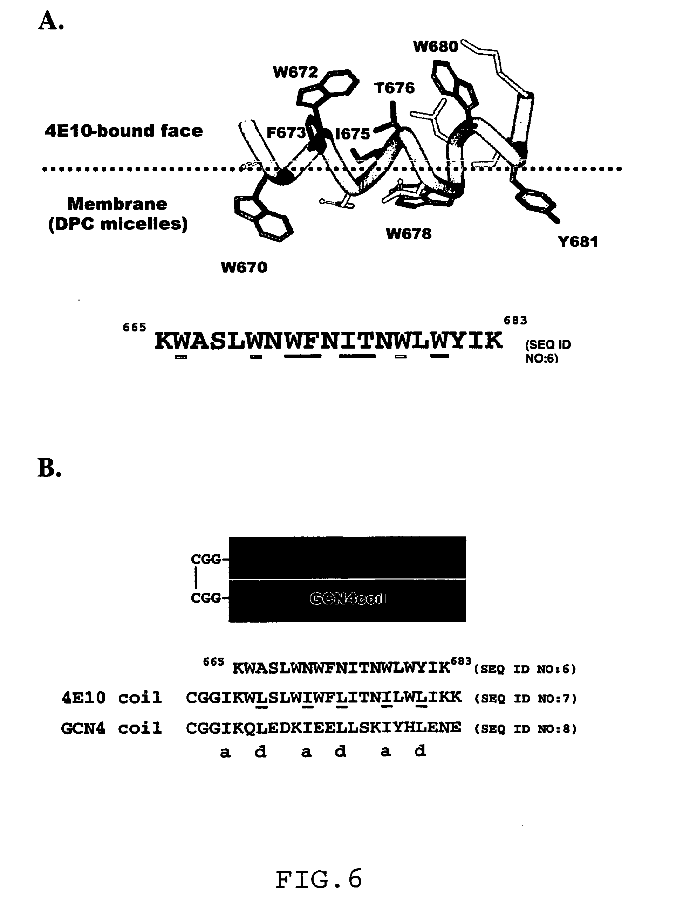

Figure 6A shows the amino acid sequence of the tryptophan (Trp)-rich motif in the HIV-1 gp41 ectodomain (SEQ ID NO:6) and a schematic representation of its secondary structure as identified by Schibli , D.J. et al. (2001, Biochemistry 40:9570-8). Recently, the single-underlined amino acid residues (W672, F673,1675, T676 and W680) have been identified to interact with the HIV-neutralizing antibody 4E10 (Cardoso, R.M.F. et al., 2005, Immunity vol.22:163-173). The double-underlined amino acid residues (W666, W370, and W678) are membrane-facing Trp residues. Figure 6B shows a schematic representation of a proposed 4E10-targeted vaccine based on the leucine zipper GCN4. The underlined amino acid residues represent mutations introduced in some membrane-facing residues of the Trp-rich region containing the 4E10-binding amino acids to produce the peptide "4E10 coil" (SEQ ID NO:7) having a dimerization surface compatible to the "GCN4 coil" peptide (SEQ ID NO:8). Disulfide bonds further enhance the GCN4/4E10 coiled-coil.

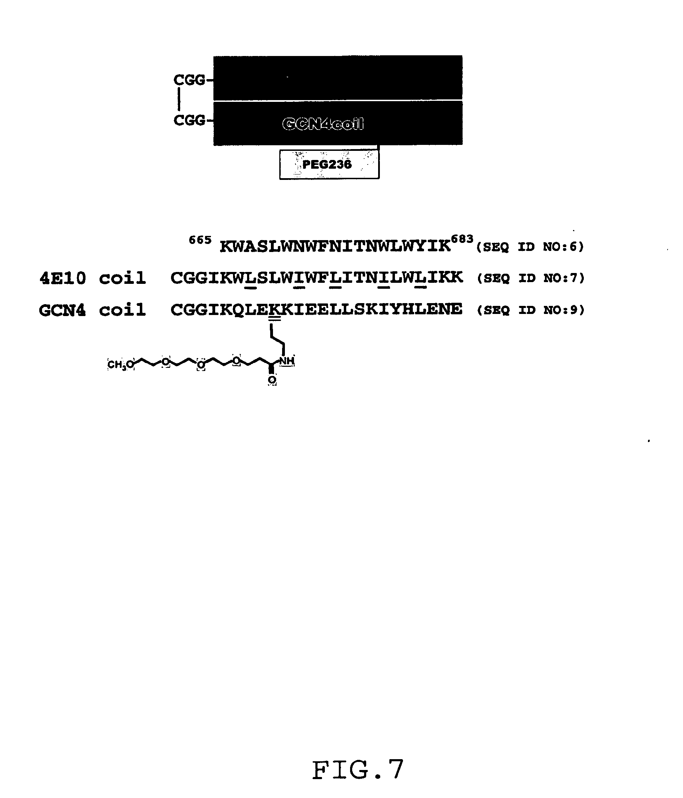

Figure 7 shows a schematic representation of the proposed 4E10-targeted vaccine depicted in Figure 6B that is PEG-modified by the attachment of one m-dPEG™ acid (MW=236) moiety to a lysine residue (double-underlined).

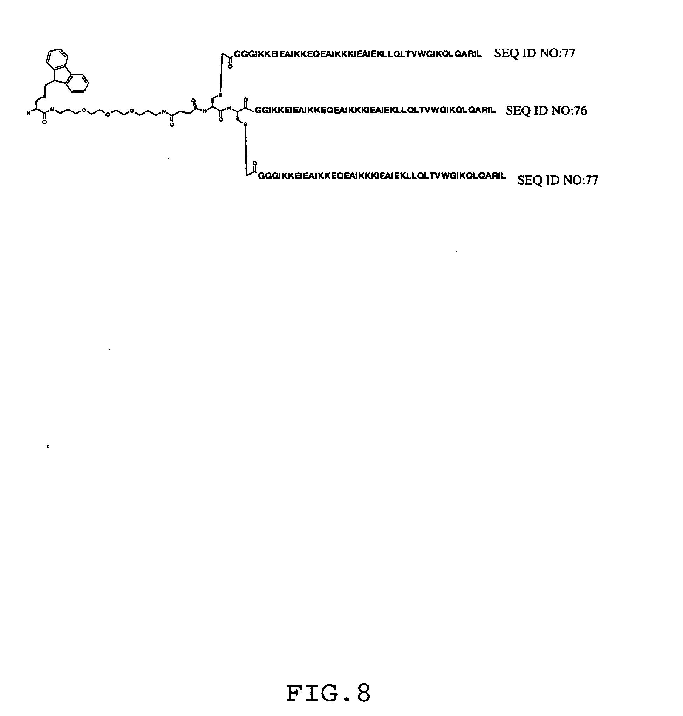

Figure 8 shows the chemical, structural representation of a trimeric coiled-coil structure, NH_{Z}-C(Fm)(thioIZN17)₃, which can be polymer-modified as per the present invention which are covalently-stabilized by thioether chemoselective bonds. The trimeric coiled-coil is by two thioether bonds located between a thiol (-SH) group on each of the two cysteine amino acid residues of the CC-chimeric N-peptide, NH₂-C(Fm)Ttds-CCIZN17 (SEQ ID NO:76), and the electrophilic bromo-moiety of each of the two derivatized-chimeric N peptides, Br-acetyl-GGGIZIV17 (SEQ ID NO:77). The amino acids located at the COOH-terminus of the bonded cysteine residues are in single letter nomenclature. This coiled-coil structure has a fluoroenylmethoxy ("Fm") protecting group shielding the thiol of the NH₂-teminal cysteine residue of the CC-chimeric N-peptide, as well as a free amino group at the NH₂-terminus.

### DETAILED DESCRIPTION OF THE INVENTION

The present invention describes, in general, a method of selectively masking, or shielding, a functional site within a peptide or protein that is normally recognized by a particular receptor. In one embodiment of the present invention, the functional site (*i.e.,* binding site) to be masked via the disclosed methods is an immunogenic or antigenic epitope, *e.g.*, a T-cell or B-cell epitope, and the receptor which is inhibited from recognizing said epitope is an antigen receptor of a T-cell or an immunoglobulin molecule, respectively. The masking of selected epitopes helps to eliminate unwanted immune reactivity to a portion of the peptide/protein antigen without affecting the immunogenicity of both alternative epitopes within the peptide/protein and the remainder of the peptide/protein in general. The selective masking of one or more binding sites (*e.g.*, epitopes) within a peptide or protein is achieved, as described herein, by the attachment of one or more small molecular weight ("SMW") polymers (*e.g.*, polyethylene glycol) to one or more suitable locations within the peptide/protein, *i.e.*, either within or in close proximity to the binding site to be masked. In one embodiment of the present invention, the SMW polymer can be attached to one or more amino acid residues within or in close proximity to the binding site to be masked, wherein said amino acids can be coded or non-coded residues. Thus, the present invention relates to a method of shielding a binding site within a polypeptide, including but not limited to an epitope of a polypeptide antigen, comprising site-specifically attaching at least one small molecular weight, water-soluble polymer to said polypeptide such that the binding site is selectively masked by said polymer.

As noted above, while the methods described herein are broadly applicable to the selective shielding of any binding site for a receptor located within a peptide or protein, the present invention is described in detail herein, and later exemplified with specific examples, with the selective shielding of epitopes (*i.e.*, B- or T-cell epitopes) within peptides or proteins which are recognized by either immunoglobulin molecules or T-cell receptors. In no way, however, is the current description intended to limit the present invention to methods of selectively masking said epitopes. One of skill in the art will appreciate how the methods described in detail herein can be modified to selectively shield any specific binding site within a peptide or polypeptide of interest with the intent of preventing the recognition of that binding site by its cognate receptor. Thus, a "binding site," as used herein, refers to a region of a peptide or polypeptide comprising either amino acid residues that are recognized by and/or interact with other proteins (*e.g.*, antibodies or cellular receptors) or sugar side chains attached thereto. Thus, an antigenic/immunogenic epitope masked as per methods described as part of the present invention represents a "binding site" as defined herein. Proteineous binding sites may comprise either a three-dimensional, conformational region of a properly folded peptide/polypeptide generated by selective, non-contiguous amino acids (*e.g.*, as often seen for catalytic active sites) or a linear epitope of contiguous amino acids (*e.g.*, T-cell epitopes).

The present invention relates to a method of shielding a functional site (*i.e.*, binding site) of a peptide or polypeptide comprising site-specifically attaching at least one small molecular weight, water-soluble polymer to said peptide/polypeptide such that the functional site is selectively masked by said polymer. In one embodiment of the present invention, said functional site is an epitope within a polypeptide antigen. Thus, one embodiment of the present invention relates to a method of shielding an epitope of a polypeptide antigen comprising site-specifically attaching at least one small molecular weight, water-soluble polymer to said antigen such that the epitope is selectively masked by said polymer. As used herein, an "epitope" encompasses a protein determinant capable of inducing classic immunogenic (*i.e.*, ability to induce a humoral or cell-mediated response) and/or antigenic (*i.e.*, ability to combine specifically with an antibody of T-cell receptor) responses. Thus, an immunogenic epitope is merely one of the many types of binding sites which can be selectively masked as per the methods described as part of the present invention. It is well known that epitopes usually consist of chemically active surface groupings of molecules such as amino acids or sugar side chains and often have specific three dimensional structural characteristics, as well as specific charge characteristics. Conformational and nonconformational epitopes are distinguished in that the binding of the former but not the latter is lost in the presence of denaturing solvents.

Methods of selectively shielding an epitope of a peptide/polypeptide is particularly useful if said peptide/polypeptide contains more than one epitope, wherein a specific immune response is desired against one or more of said epitopes and away from others. Thus, the methods described herein may be used to focus the immune response toward one or more desired, unmasked epitopes and away from the masked epitope which has been rendered unrecognizable to immune-derived cells. By hindering the association of the masked epitope with its immune cell receptor (*e.g.,* an immunoglobulin molecule) via attaching a small molecular weight polymer molecule in a suitable location within the peptide/polypeptide, the epitope is effectively both physically and functionally shielded. For example, a polypeptide antigen (*e.g.*, a synthetic peptide or a pathogenic protein) may contain one or more immunodominant epitopes that selectively provoke an immune response in a host organism to the substantial exclusion of other epitopes within that antigen. If said immunodominant epitopes induce an unfavorable immune responses (*e.g.*, production of either non-neutralizing antibodies or non-conserved, neutralizing antibodies) at the expense of potential immune responses generated against less immunostimulating epitopes located within the same peptide/protein, it is important to focus the immune response to the less immunostimulating epitopes and away from the immunodominant epitope. Thus, one embodiment of the present invention relates to the immunodampening of an immunodominant, unfavorable epitope(s) in efforts to direct a more favorable immune response (*e.g.*, a neutralizing response or a more conserved, neutralizing response) to an alternative epitope within the protein/peptide. Importantly, immunodampening a disfavored immune response by shielding the epitope which induces said response does not necessarily include the complete removal of the undesired response.

The present invention further relates to said polymer-modified peptides or proteins generated as per the methods described herein, pharmaceutical/vaccine compositions that contain them, and a method of immunizing a mammal against a disease condition with said pharmaceutical/vaccine compositions to induce selective immunoprotection in said mammal. Thus, the present invention relates to a method of focusing an immune response directed against a polypeptide, wherein said polypeptide contains more than one epitope capable of inducing an immune response, comprising site-specifically attaching at least one small molecular weight, water-soluble polymer to said polypeptide such that at least one epitope is selectively masked by said polymer, directing the immune response against said polypeptide to unshielded regions within said polypeptide. Thus, the present invention relates to a method of altering an immune response directed against a polypeptide by selectively shielding one or more epitopes within said polypeptide by site-specifically attaching at least one small molecular weight, water-soluble polymer to said polypeptide as per the methods described herein.

As demonstrated with the use of polyethylene glycol ("PEG") in the Examples section infra, the polymer used to selectively mask binding sites or epitopes as described herein are substantially straight-chain polymers and substantially non-immunogenic, as well as non-toxic, highly soluble and devoid of biological activity. PEG has been selected as the preferred embodiment of polymer used to selectively shield the binding sites or epitopes as per the methods described herein; however, this polymer is employed solely for purposes of illustration and not limitation. Examples of water-soluble polymers include: polyalkylene glycol and derivatives thereof, including methoxypolyethylene glycol, polyethylene glycol homopolymers, polypropylene glycol homopolymers, copolymers of ethylene glycol with propylene glycol, wherein said homopolymers and copolymers are unsubstituted or substituted at one end with an alkyl group, heparin and fragments of heparin, polyvinyl alcohol and polyvinyl ethyl ethers, polyvinylpyrrolidone, aspartamide, and polyoxyethylated polyols, with the dextran and dextran derivatives, dextrine and dextrine derivatives. It will be appreciated that various derivatives of the specifically recited water-soluble polymers are also contemplated for use in the methods described herein. Water-soluble polymers such as those described above are well known, particularly the polyalkylene oxide based polymers such as PEG (see, *e.g.,* Poly(ethylene glycol) Chemistry: Biotechnical and Biomedical Applications, Ed. J.M. Harris, New York:Plenum Press, 1992; Poly(ethylene glycol) Chemistry and Biological Applications, Eds. J.M. Harris and S. Zalipsky, ACS, 1997; International Patent Applications with the following publication numbers: WO 90/13540, WO 92/00748, WO 92/16555, WO 94/04193,WO 94/14758, WO 94/17039, WO 94/18247, WO 94/28937, WO 95/11924, WO 96/00080, WO 96/23794, WO 98/07713, WO 98/41562, WO 98/48837, WO 99/30727, WO 99/32134, WO 99/33483, WO 99/53951, WO 01/26692, WO 95/13312, WO 96/21469, WO 97/03106, WO 99/45964; and the following U.S. Patents: 4,179,337; 5,075,046; 5,089,261; 5,100,992; 5,134,192; 5,166,309; 5,171,264; 5,213,891; 5,219,564; 5,275,838; 5,281,698; 5,298,643; 5,312,808; 5,321,095; 5,324,844; 5,349,001; 5,352,756; 5,405,877; 5,455027; 5,446,090; -5,470, 829; 5,478,805; 5,567,422; 5,605,976; 5,612,460; 5,614,549; 5,618, 528; 5,672,662; 5,637,749; 5,643,575; 5,650,388; 5,681,567; 5,686,110; 5,730,990; 5,739,208; 5,756,593; 5,808,096; 5,824,778; 5,824,784; 5,840,900; 5,874, 500; 5,880,131; 5,900,461; 5,902,588; 5,919,442; 5,919,455; 5,932, 462; 5,965,119; 5,965,566; 5,985,263; 5,990,237; 6,011,042; 6,013,283; 6,077,939; 6,113,906; 6,127,355; 6,177,087; 6,180,095; 6,194,580; and 6,214,966).

In one embodiment of the present invention, the small molecular weight polymer used to selectively shield a binding site within a polypeptide, including but not limited to an epitope of a polypeptide antigen, by site-specific attachment to said polypeptide is selected from the group consisting of polyalkylene oxide, polyamide alkylene oxide and derivatives thereof. In a further embodiment, said small molecular weight, water-soluble polymer is PEG, having a water-soluble repeat unit comprising of -(CH₂-CH₂-O)-. PEG is favored because the polyether backbone is inert in both biological environments and under most chemical reaction conditions, making the termini easily accessible for chemical modification needed to generate a PEG derivative comprising a functional group that is capable of covalent attachment to a. particular reactive group on the peptide/protein to be modified (*i.e.,* "activated" PEG). There are a variety of activated PEG polymers known in the art and having functional groups which include, but are not limited to, carbonate, active ester, aldehyde, tresylate, acrylate, ketone, aminooxy, amine, carboxylic acid, ester, thioester, halogen, thiol, cyanoacetate, dipalmitoyl phosphatidylethanolamine, distearoyl phosphatidylethanolamine, epoxide, hydrazide, azide, isocyanate, maleimide, methacrylate, nitrophenyl, orthopyridyl disulfide, silane, sulfhydryl, vinyl sulfones, succinimidyl glutarate, succimidyl succinate and succinic acid. A functional group can be chosen based on the type of available reactive group on the polypeptide to be modified. In addition, monofunctional PEG derivatives ("mPEG"), having only one derivatizable end, can be utilized to minimize cross-linking of polypeptides resulting from a single PEG polymer attaching to more than one polypeptide, thus providing improved homogeneity. The most common reactive sites of polypeptides are the α- or ε-amino groups of lysine residues or the NH₂-terminal amino group of other amino acids. A list of other typical reactive sites includes the amino acids cysteine, histidine, arginine, aspartic acid, glutamic acid, serine, threonine, tyrosine, as well as the COOH-terminal carboxylic acid of amino acids. Alternatively, the SMW polymer molecules of the present invention, including but not limited to PEG, can be site-specifically attached to target polypeptides or peptides via an interaction with a reactive group other than one present within a coded or non-coded amino acid. For example, said SMW polymer may be attached via interaction with a reactive group within a carbohydrate moiety or synthetic linker, wherein said moiety or linker is a component of the target polypeptide/peptide structure.

The variety of polymers used to selectively modify a polypeptide as per the methods described herein, including but not limited to PEG, are intended to encompass those having a small molecular weight. Generally, a polymer of a "small molecular weight," as used herein, is considered to represent a polymer, particular a PEG polymer, of a molecular weight less than or equal to about 1000 Da *(e.g.,* ≤ PEG 1000). The number of water-soluble repeat units of the attached polymer moiety can vary; however, for an attached PEG moiety, for example, the more preferred number of such units (-(CH₂-CH₂O)-) ranges up to 20 units. While it is anticipated that small molecular weight polymers, including PEG, used to mask selected binding sites or epitopes within a peptide or polypeptide as outlined in the present invention will have a molecular weight less than or equal to approximately 1000 Da, one of skill in the art will appreciate how the size range of polymer used to modify a peptide/protein will largely depend on the size of the binding site or epitope to be masked. To this end, the present invention is not limited to the use of polymers less than or equal to approximately 1000 Da in weight if, for example, full or substantial shielding of the binding site/epitope would require attachment of a polymer of a large molecular weight. However, it is important to remember that the goal of the methods described herein is to shield only a portion of the peptide/polypeptide, said portion comprising the binding site/epitope to be masked, rather than globally shielding all binding sites/epitopes present within the protein.

The present invention relates to polymer-modified, therapeutic peptides or polypeptide wherein one or more epitopes (*e.g.*, which generate an undesirable immune response) or binding sites is selectively masked, leaving the remainder of the therapeutic agent accessible for induction of an alternative and more favorable immune response or activity. Thus, the goal of the present invention is to shield the therapeutic protein only to the extent necessary to mask a particular binding site/epitope, making small molecular weight, unbranched polymers most attractive for use in the present methods. Although polymer-modified proteins have been previously described, the nature, manner and intent of the polymer-modifications of the present invention markedly differ from such prior efforts. A vast majority of the polymer-modified, biologically active conjugates disclosed in the prior art are modified by the attachment of PEG moieties of a size greater than about 1000 Da. For example, the six PEGylated products approved by the Food and Drug Administration (Adagen^{®}, Oncaspar^{®}, PEG-Intron^{®}, Pegasys^{®}, Somavert^{®} and Neulaste^{®}) are each modified with PEG polymers of a molecular weight ranging from 5,000 to 40,000 Da. Use of high molecular weight ("HMW") polymers are disfavored in the present invention due to the possibility of masking more of the peptide/polypeptide than desired, thus potentially compromising the recognition of favorable binding sites/epitopes. In the past, it has been accepted that coupling a therapeutic protein or chemical compound with HMW polymers accompanies a certain sacrifice in the biological activity provided by the therapeutic agent. While the attached, HMW PEG polymers often cover (*i.e.*, shield) all or a substantial portion of a therapeutic agent, the high flexibility of PEG molecules provides moments when the active sites of the agent are visible (*e.g.*, accessible) to effectuate the intended biological response. Thus, while the specific activity of the therapeutic agent is reduced as a result of the modification, the bioavailability of the agent increases due to both reduced kidney and reticuloendothelial system ("RES") clearance and shielding from proteolytic enzymes. Ultimately, this increase in bioavailability provides a more favorable biological response to the administration of the therapeutic agent as a whole. In comparison, the intent of the present invention is not to induce a general, improved response to a given activity of the protein/peptide. Rather, the given activity of a protein/polypeptide is selectively altered by the modifications described herein. Because of the high flexibility of the polyether backbone and its ability to bind water molecules, PEG molecules act as if they are 5-10X as large as a soluble protein of comparable molecular weight. Thus, the present inventors hypothesized that the selective shielding of epitopes or binding sites can be achieved by attachment of smaller molecular weight, water-soluble polymers (*e.g.*, with a molecular weight of less than about 1000 Da) in a site-specific manner, leaving the remainder of the protein, and the binding sites/epitopes within it, available to interact with cognate receptors without limitation and to generate and/or facilitate a desired response.

Thus, a preferred embodiment of the present invention relates to a method of shielding an epitope or binding site of a peptide or polypeptide comprising site-specifically attaching at least one small molecular weight, water-soluble polymer to said peptide/polypeptide such that the epitope/binding site is selectively masked by said polymer, wherein the peptide/polypeptide contains more than one epitope or binding site. As a result of the polymer-modification of the peptide/polypeptide, the biological response induced by said peptide/polypeptide when administered to an organism (*e.g.*, in the form of a vaccine) is a result of the activity of the unmasked epitopes or binding sites of the peptide/polypeptide. Thus, the present invention relates to a method of modifying a peptide or polypeptide by covalently attaching to said peptide/polypeptide a small molecular weight, water-soluble polymer that, when administered to an organism (*e.g.*, in the form of a vaccine), induces a specific biological response to one or more regions of the peptide/polypeptide that are not shielded by the attached polymer. In one embodiment of the present invention, the peptide or polypeptide of interest comprises one or more epitopes, either alone or in combination with, one or more binding sites for proteins or compounds other than those originating from the immune system. It is the intention of one embodiment of the present invention that particular epitopes are selectively shielded in order to reduce immunogenicity of a portion or region of an amino acid sequence (*i.e.*, to reduce a particular immune response in favor of another). The bioactivity of the peptide/polypeptide is thus refocused on a different (*i.e.*, unmasked) portion of the protein, which in turn changes the specific response to said peptide/protein. In one embodiment of the present invention, the selectively masked epitope is a "scaffold" domain located within a covalently-stabilized, trimeric coiled-coil structure that mimics all or a portion of an internal, trimeric coiled-coil motif of an enveloped virus membrane-fusion protein comprising three chimeric peptides covalently-stabilized by one or more covalent bonds between said peptides. Each chimeric peptide of said coiled-coil comprises a scaffold domain comprising a soluble, trimeric form of a coiled-coil fused in alpha-helical phase to all or a portion of a NH₂-terminal heptad repeat domain, or a modified form thereof, of said enveloped virus membrane-fusion protein. In one embodiment of this portion of the present invention, said enveloped virus membrane-fusion protein is HIV gp41.

Thus, the present invention relates to a method of shielding an epitope of a polypeptide antigen comprising site-specifically attaching at least one small molecular weight, water-soluble polymer, including but not limited to SMW PEG, to at least one amino acid residue within said antigen such that the epitope is selectively masked by said polymer, wherein said selectively-masked epitope is located within a scaffold domain of a covalently-stabilized, trimeric coiled-coil structure that mimics all or a portion of an internal, trimeric coiled-coil motif of an enveloped virus membrane-fusion protein comprising three chimeric peptides covalently-stabilized by one or more covalent bonds between said peptides, each chimeric peptide comprising a scaffold domain which comprises a soluble, trimeric form of a coiled-coil fused in helical phase to all or a portion of a NH₂-terminal heptad repeat domain, or a modified form thereof, of said enveloped virus membrane-fusion protein. In a specific embodiment of this portion of the present invention, said enveloped virus membrane-fusion protein is HIV gp41.

In an exemplary application of the present invention, the inventors have discovered that the selective masking of a portion of a vaccine construct comprising a covalently-stabilized, trimeric coiled-coil structure that mimics all or a portion of an internal, trimeric coiled-coil motif of HIV gp41 (described in detail in U.S. Provisional Patent Application Serial No. 60/576,062, filed June 1, 2004, and U.S. Provisional Patent Application Serial No. 60/636,724, filed December 16, 2004) via the attachment of two, small molecular weight PEG moieties is effective to selectively focus immune responses toward the HIV portion of the peptide mimetic (*i.e.,* reducing the immunogenicity of non-HIV portions of this mimetic of gp41 membrane fusion intermediates). This is described in detail in Example 1, *infra.* The coiled-coil structure consists of three alpha("α")-helical polypeptides, each comprising a non-HIV-derived scaffold domain, which helps to stabilize the α-helical structure, fused in helical phase to an HIV N-peptide domain. Two lysine amino acids located within the amino acid sequence of the scaffold domain of each of the three polypeptides comprised within the covalently-stabilized, coiled-coil structure are modified by the covalent attachment of a PEG 236 moiety (see Figure 3). The modification of each peptide results in a coiled-coil structure with a total of six, covalently-attached, SMW PEG molecules. As shown in Example 1, said SMW PEG molecules effectively shield the production of antibodies against the scaffold domain, focusing the immune response against the HIV portion of the structure. It is believed that this exemplification is the first disclosure of the use of SMW PEG molecules, in particular PEG 236, to selectively shield a region of a peptide/protein of interest.

Therefore, one embodiment of the present invention relates to a method of shielding an epitope of a covalently-stabilized, trimeric coiled-coil structure that mimics all or a portion of an internal, trimeric coiled-coil motif of HIV gp41 comprising site-specifically attaching at least one small molecular weight, water-soluble polymer to said coiled-coil structure such that said epitope is selectively masked by said polymer. In a further embodiment, the masked epitope is located within the scaffold domain of the coiled-coil structure, wherein said coiled-coil structure comprises three chimeric peptides, each having a "scaffold domain" which comprises a soluble, trimeric form of a coiled-coil that is fused in helical phase to all or a portion of an HIV gp41 NH₂-terminal heptad repeat domain, or a modified form thereof, and wherein said peptides are covalently-stabilized by one or more covalent bonds. The scaffold domain can be modified by the attachment of one or more small molecular weight, water-soluble polymers to one or more amino acid residues within said domain, wherein said water-soluble polymer is selected from the group consisting of polyalkylene oxide, polyamide alkylene oxide and derivatives thereof, and more specifically, a PEG polymer (*e.g.*, PEG 236).

The trimeric coiled-coil structures which can be selectively shielded as per the methods described herein have been described in detail in U.S. Provisional Patent Application Serial Nos. 60/576,062, filed June 1, 2004, and 60/636,724, filed December 16, 2004; both of which are incorporated by reference herein. The trimeric coiled-coil structures described in these applications are mimetics of enveloped virus membrane fusion, specifically representing mimetics of fusion intermediates of membrane proteins of enveloped virus, including but not limited to the HIV gp41. As an example of the membrane fusion event used by many enveloped viruses, HIV gp41-mediated membrane fusion is a complex process involving three essential components located in the ectodomain of the gp41 glycoprotein: an NH₂-terminal fusion peptide, an NH₂-terminal heptad repeat ("N-helix") and a COOH-terminal heptad repeat ("C-helix"). The two heptad repeat regions (NH₂:HR1 and COOH:HR2) impart periodic hydrophobicity to the glycoprotein and are predictive of α-helical structures that interact with each other to form a fusogenic (*i.e.,* fusion-active) conformation of gp41 called the "trimer-of hairpins," a common structural motif involved in the fusion mechanism of many enveloped viruses. The trimer-of-hairpins structure is a bundle of six α-helices: three α-helices formed by C-helix regions from three gp41 ectodomains packed in an anti-parallel manner against a central, three-stranded coiled-coil formed by N-helix regions from three gp41 ectodomains. The fusion process progresses via the formation of a "pre-hairpin" conformation that places the NH₂-terminal fusion peptide near/in the target cell membrane, exposing the N-helix coiled-coil. The trimer-of-hairpins forms when the three C-helices then fold back to associate with this central, N-helix coiled-coil, drawing the viral and host cell membranes into close proximity as a prelude to membrane fusion. Anti-HIV agents that prevent viral/host cell membrane fusion include neutralizing antibodies that bind to the pre-hairpin or trimer-of-hairpins complex. As such, a hydrophobic pocket within the heptad repeat 1 (HR1) region of the gp41 ectodomain, formed by the central, N-helix coiled-coil, is thought to be an attractive target for eliciting broadly cross-reactive, neutralizing antibodies to HIV (see U.S. Provisional Application Serial No. 60/576,012, filed June 1, 2004, describing a neutralizing antibody that binds to this hydrophobic pocket region). Since the "pre-hairpin" structure is a common feature of many enveloped viral membrane-fusion proteins (see, Singh et al., 1999, J. Mol. Biol. 290:1031-1041), polymer-modified mimetics of membrane fusion can be generated to produce vaccine constructs specific to a variety of enveloped viruses using the methods described herein. Thus, while the methods exemplified herein target the production of a vaccine construct specific to HIV, it will be known to one of skill in the art that this same basic strategy can be used to generate vaccine constructs targeting other enveloped viruses.

Thus, one embodiment of the present invention relates to a polymer-modified, covalently-stabilized, trimeric coiled-coil that mimics all or a portion of a HIV gp41 N-peptide coiled-coil comprising three chimeric peptides, each chimeric peptide comprising a scaffold domain, which comprises a soluble, trimeric form of a coiled-coil, fused in helical phase to all or a portion of a HIV gp41 N-peptide, or a modified form thereof, wherein an epitope located within the scaffold domain is selectively masked by at least one small molecular weight, water-soluble polymer site-specifically attached to at least one amino acid residue within or in close proximity to said scaffold domain. Said trimeric coiled-coil gp41 mimetic is stabilized via covalent bonds generated between said chimeric peptides including, for example, disulfide bonds or any covalent bond which results from a chemoselective ligation reaction, and can represent either a homotrimeric or heterotrimeric structure. Epitopes located within the scaffold portion of said coiled-coil structures can be shielded via the attachment of a SMW water-soluble polymer selected from the group consisting of polyalkylene oxide, polyamide alkylene oxide and derivatives thereof, including but not limited to a PEG polymer with a molecular weight of less than about 1000 Da.

Thus, in one embodiment of the present invention, a polymer-modified, trimeric coiled-coil structure that mimics all or a portion of an internal, trimeric coiled-coil motif of an enveloped virus membrane-fusion protein, including but not limited to HIV gp41, comprises a coiled-coil mimetic that is covalently-stabilized via the formation of covalent, disulfide bonds between cysteine residues on juxtaposed, α-helical peptides contained within the coiled-coil structure, wherein a region of the coiled-coil structure (containing an epitope) is shielded by the attachment of a SMW polymer to one or more amino acids within or near said region. In this embodiment of the present invention, the cysteine amino acid residues that participate in the covalent bond formation are engineered to reside outside of the α-helical domain of said individual α-helical peptides. Thus, the individual peptides contained within the coiled-coil structure each have an α-helical domain which comprises a soluble, trimeric form of a coiled-coil ("scaffold" domain) fused in helical phase to all or a portion of a NH₂-terminal heptad repeat domain ("N-peptide" domain), or a modified form thereof, of said enveloped virus membrane-fusion protein, including by not limited to HIV gp41, and at least two cysteine residues located outside of said α-helical domain. By incubating a plurality of said soluble chimeric peptides at a concentration at which a trimeric coiled-coil structure forms, and then oxidizing said structures, covalent disulfide bonds are generated between cysteine residues on juxtaposed chimeric peptides of the coiled-coil. As described previously, the preferred epitopes to be shielded as per the methods disclosed herein are located within the scaffold domain.

In a further embodiment of the present invention, a polymer-modified, trimeric coiled-coil structure that mimics all or a portion of an internal, trimeric coiled-coil motif of an enveloped virus membrane-fusion protein, including but not limited to HIV gp41, comprises a coiled-coil mimetic that is covalently-stabilized via the formation of covalent, chemical bonds between the α-helical peptides contained within the coiled-coil structure resulting from a chemoselective ligation reaction, wherein a region of the coiled-coil structure (containing at least one epitope) is shielded by the attachment of a SMW polymer to one or more amino acids within or near said region. In this embodiment, said coiled-coil structure is comprised of three, chimeric peptides, each having an α-helical domain which comprises a soluble, trimeric form of a coiled-coil ("scaffold" domain) fused in helical phase to all or a portion of a NH₂-terminal heptad repeat domain ("N-peptide" domain), or a modified form thereof, of said enveloped virus membrane-fusion protein, including but not limited to HIV gp41. The three chimeric peptides are stabilized in the coiled-coil structure via formation of covalent, chemical bonds generated by a chemoselective reaction, including but not limited to the formation of thioether bonds. For example, a heterotrimeric coiled-coil structure that can be polymer-modified as per the present invention can comprise one chimeric peptide which further comprises at least two cysteine residues located outside of the α-helical domain of said peptide, and two chimeric peptides which are each derivatized to incorporate an electrophilic moiety; wherein a nucleophilic sulfhydryl of each cysteine residue in the first chimeric peptide forms a thioether bond with the electrophilic moiety of each of the two derivatized chimeric peptides, as described in detail in U.S. Provisional Patent Application Serial No. 60/576,012; *supra*). Chemoselective ligation reactions that may be used to stabilize said coiled-coil structures include, but are not limited to, reactions between the following chemical species, wherein said chemical species are incorporated within the individual chimeric peptides contained within the resulting trimeric structure: (i) an aldehyde/ketone and a hydrazide to form a hydrazone; (ii) a ketone and a aminoxy group to form an oxime; (iii) a ketone and a thiosemicarbazide to form a thiosemicarbazone; (iv) an aldehyde and **a** β-amino thiol to form a thiazolidine; (v) a thiocarboxylate and a α-halo carbonyl to form a thioester; (vi) a thioester and a N-terminal peptide cysteine to form an amide; (vii) a alkyl halide and a thiol to form a thioether; and, (viii) a maleimide and a thiol to form a thioether.

Therefore, one embodiment of the present invention relates to chimeric peptides that are polymer-modified as per the methods described herein, wherein said peptides can be covalently-stabilized to generate trimeric coiled-coil structures. Said chimeric peptides comprise an alpha("α")-helical domain comprising an α-helical scaffold protein capable of forming a trimeric coiled-coil (the "scaffold" portion) fused in helical phase to all or a portion of a NH₂-terminal heptad repeat domain of an enveloped virus membrane-fusion protein (the "N-peptide" portion), including but not limited to HIV gp41. It is this scaffold domain, containing at least one or more epitopes, which is site-specifically shielded via the attachment of one or more small molecular weight polymers, including but not limited to PEG, to selected amino acid residues located either within or in close proximity to the scaffold domain sequence. As described previously, this polymer-modification focuses the immune response to the viral N-peptide portion of the trimeric coiled-coil.

As described in detail in U.S. Provisional Patent Applications 60/576,062 and 60/636,724, *supra,* chimeric peptides which comprise all or a portion of the NH₂-terminal heptad repeat domain (N-helix) of.HIV gp41 fused to an α-helical scaffold protein capable of acquiring a trimeric coiled-coil conformation are known in the art (see Eckert and Kim, 2001, Proc. Natl. Acad. Sci. USA 98:11187-11192). Chimeric peptides having this general structure are designated herein as "chimeric N-peptides" and are not, however, limited to FHV-derived peptides. An NH₂-terminal heptad repeat domain of any enveloped viral membrane fusion protein known to utilize a similar fusion mechanism as that used by HIV (*i.e.*, trimer-of-hairpins) can be used to generate a chimeric N-peptide to be used within the present invention. In one embodiment of the present invention, soluble, chimeric peptides which can be polymer-modified as per the methods described herein and covalently-stabilized within homotrimeric or heterotrimeric coiled-coil structures represent derivatives of these chimeric N-peptides. One example of such a chimeric N-peptide derivative which can be polymer-modified as described herein is referred to as a "CC-chimeric N-peptide." CC-chimeric N-peptides comprise the following components: (1) an α-helical scaffold protein capable of acquiring a trimeric coiled-coil conformation ("scaffold" portion); (2) all of a portion of the NH₂-terminal heptad repeat domain ("N-helix") of an enveloped viral membrane protein ("N-peptide" portion), including but not limited to all or a portion of the N-helix region of HIV gp41; and, (3) at least two cysteine residues located at either the NH₂- or COOH-terminus of the chimeric peptide sequence ("cysteine" portion), optionally separated from the core chimeric peptide sequence by a linker/spacer region for increased flexibility. The scaffold portion is fused in helical phase to the N-peptide portion described above, and the cysteine portion (optionally including the linker/spacer region) is located outside of this α-helical core of the chimeric peptide. Said peptides are capable of forming a soluble, trimeric coiled-coil structure wherein three, identical (*i.e.*, to form a homotrimeric coiled-coil) or substantially similar (*i.e.*, to form a heterotrimeric coiled-coil) chimeric peptide chains physically associate in a parallel orientation. It is the scaffold domain, and any epitopes located within it, that is selectively shielded via the attachment of one or more small molecular weight polymer moieties, including but not limited PEG, to one or more amino acid residues located within or near said domain in order to dampen the immunogenicity of this region and, in turn, focus the immune response to the N-peptide portion of the trimeric coiled-coil structure.

Thus, one embodiment of the present invention relates to a polymer-modified, soluble chimeric peptide which comprises: (a) a scaffold portion comprising a soluble, trimeric form of a coiled-coil; (b) a N-peptide portion comprising all or a portion of a N-peptide of HIV gp41, or a modified form thereof; and, (c) a cysteine portion comprising at least two cysteine residues; wherein said scaffold portion in (a) is fused in helical phase to said N-peptide portion in (b), forming an α-helical domain; said cysteine portion in (c) is located outside of said α-helical domain; and one or more epitopes located within said scaffold portion is selectively masked by at least one small molecular weight, water-soluble polymer site-specifically attached to at least one amino acid residue within said scaffold portion.

"CCIZN17" is one CC-chimeric N-peptide that can be polymer-modified as part of the present invention (*i.e.*, selective shielding of epitopes within the scaffold domain). The core α-helical domain of CCIZN17 is the chimeric N-peptide, "IZN17," disclosed in Eckert and Kim, 2001, *supra.* IZN17 (*IKKEIEAIKKEQEAIKKKIEAIEK*LLQLTVWGIKQLQARIL; SEQ ID NO:1) was designed by Eckert and Kim, 2001, *supra,* to enable the formation of nonaggregating, trimeric coiled-coils of a portion of the N-helix domain of HIV gp41. This chimeric peptide consists of a soluble α-helical domain capable of forming a trimeric coiled-coil (the "IZ" scaffold domain; SEQ ID NO:14; italicized above), fused in helical phase to the NH₂-terminus of a portion of the N-helix of HIV gp41 (the "N17" N-peptide domain; SEQ ID NO:32; underlined above), creating a continuous coiled-coil that is 41 amino acids in length. The IZ domain is a modified isoleucine zipper based on a design described by Suzuki et al. (1998, Protein Eng. 11:1051-1055) that is helical and trimeric in solution. The N17 domain is a truncated portion of a 36 amino acid peptide ("N36") derived from the N-helix region of HIV gp41 identified by protein dissection of the gp41 ectodomain. As described in detail in U.S. Provisional Patent Applications 60/576,062 and 60/636,724, *supra,* CCIZN17 was generated by adding the amino acid sequence Cys-Cys-Gly-Gly (SEQ ID NO: 10) to the NH₂-terminus of said IZN17. Thus, CCIZN17 consists of the following amino acid sequence:
CCGGIKKEIEAIKKEQEAIKKEQEAIEKLLQLTVWGLKQLQARIL (SEQ ID NO:2). The cysteine residues added to IZN17 to generate CCIZN17 impart the ability of said CC-chimeric N-peptide to form covalently-stabilized, trimeric coiled-coils (see Figure 1 for disulfide bond-stabilized, CCIZN17-derived coiled-coils; see Figure 8 for thioether bond-stabilized, CCIZN17-derived coiled-coils). The additional glycine residues in CCIZN17 represent a spacer region separating the cysteine residues from the α-helical core of the chimeric IZN17 peptide, giving said cysteine residues greater conformational freedom to participate in disulfide or chemoselective (*e.g.*, thioether) bond formation. The presence of covalent, disulfide bond linkages among three CCIZN17 chimeric peptides generates a covalently-stabilized, trimeric coiled-coil, (CCIZN17)₃ (see Figure 1), which is more stable than the original IZN17 trimeric coiled-coil, especially since the subsistence of the trimer is no longer dependent on the concentration of the monomeric chains (see U.S. Provisional Patent Applications 60/576,062 and 60/636,724, *supra).*

Chimeric N-peptides (*e.g.*, IZN17) can also be derivatized to incorporate an electrophilic moiety capable of participating in covalent, chemoselective reactions, designated as "derivatized-chimeric N-peptides." Thus, a further embodiment of the present invention relates to derivatized-chimeric N-peptides that are polymer-modified as per the methods described herein (*e.g.*, to selectively shield epitopes within the scaffold domain) that can be covalently-stabilized within a trimeric coiled-coil structure. By way of demonstration, a trimeric coiled-coil structure that is covalently-stabilized via chemoselective reactions can comprise a single CC-chimeric N-peptide (*e.g.*, CCCIZN17; SEQ ID NO:76) and two derivatized-chimeric N-peptides (*e.g.*, Bromoacetyl-GGGIZN17; SEQ ID NO:77), each having either identical or substantially identical α-helical core domains (*e.g.*, IZN17; SEQ ID NO:1), wherein covalent thioether bonds are generated among said chimeric peptides. Epitopes within the scaffold portions of each of these three chimeric peptides can be selectively shielded by attachment of a small molecular weight polymer moiety (*e.g.*, a SMW PEG) to one or more amino acids within said scaffold domain. The covalently-stabilized trimeric coiled-coil designated "NH₂-C(Fm)(thioIZNI7)₃" represents one example of a thioether bond-stabilized coiled-coil (see in Figure 8 and U.S. Provisional Patent Applications 60/576,062 and 60/636,724, *supra)* that can be polymer-modified as per the methods described as part of the present invention. NH₂-C(Fm)(thioIZN17)₃ is comprised of one CC-chimeric N-peptide, CCCIZN17 peptide (SEQ ID NO:76), and two derivatized-chimeric N-peptides, Bromoacetyl-GGGIZN17 (SEQ IDNO:77), wherein each bromoacetyl moiety, located at the NH₂-terminus of the derivatized-chimeric N-peptides, forms a thioether bond with a cysteine residue located in the "cysteine portion" of the CC-chimeric N-peptide. In this particular coiled-coil, the NH₂-terminal cysteine residue is initially protected by an Fm group, causing the two interior cysteine residues to participate in thioether bond formation with the bromo moieties. As demonstrated with the derivatized-chimeric N-peptide, Bromoacetyl-GGGIZN17 (SEQ ID NO:77), the electrophilic moiety of the derivatized-chimeric N-peptides may be separated from the α-helical portion of the chimeric N-peptide by a flexible linker region *(e.g.,* two or more consecutive glycine residues) to help minimize interference between coiled-coil formation of the chimeric peptides and covalent bond formation to stabilize the resulting coiled-coil.

Additionally, IQN17(*RMKQIEDKIEEIESKQKKIENEIARIKK*LLQLTVWGIKOLOARIL; SEQ ID NO:11) is a chimeric N-peptide with a similar design as IZN17; however, the N17 domain (underlined) is fused in helical phase to an alternative, trimeric coiled-coil motif derived from the yeast transcription activator, GCN4 (the "IQ" domain; SEQ ID NO:17; italicized above). Covalently-stabilized trimeric coiled-coil similar to those described above comprising core IQN17 peptides can be generated and polymer-modified as part of the present invention.

The "scaffold domain," used interchangeably with "scaffold portion" herein, of the CC-and derivatized-chimeric N-peptides that are selectively shielded as part of the present invention comprise non-HIV amino acid residues and represent a soluble, trimeric form of a coiled-coil. The scaffold domain can be fused to the NH₂-terminus or COOH-terminus of the N-peptide region described above, or divided such that portions of said domain are located at both the NH₂- and COOH-termini of the N-peptide region. If the scaffold domain is divided such that a portion is located at both ends of the N-peptide portion, in order to properly shield said divided scaffold domain and any epitopes contained within one or both of the divided portions, at least one polymer moiety will be attached to an amino acid residue residing in each portion (*i.e.*, the NH₂-terminal portion and the COOH-terminal portion) of the divided scaffold region. Coiled-coil proteins consist of a characteristic heptad repeat of amino acids designated by the letters "a" through "g", wherein the first and fourth positions of the heptad repeat, the "a" and "d" positions, respectively, form the interior of the interacting strands of the coiled-coil and are generally hydrophobic. Coiled-coil motifs representing scaffold domains of the polymer-modified, trimeric coiled-coil structures described herein can be isolated from a variety of sources, including, but not limited to, the isoleucine zipper motif disclosed in Suzuki et al. (1998, *supra;* hereinafter "Suzuki-IZ"; YGGIEKKIEAIEKKIEAIEKKIEAIEKKIEA; SEQ ID NO:12) and the GCN4-pI_{Q}I coiled-coil motif disclosed in Eckert et al. (1998, J. Mol. Biol. 284:859-865 and PCT International Application PCT/US01/29637, International publication number WO 02/24735; RMKQIEDKIEEILSKQYHIENEIARIKKLIGER (SEQ ID NO:13)), and truncated and/or modified versions thereof. The Suzuki-IZ or GCN4-pI_{Q}I coiled-coil motifs can be changed by the addition, substitution, modification and/or deletion of one amino acid residues. For example, an amino acid residue within said scaffold protein may be modified to facilitate the selective attachment of a SMW polymer at that particular location. While the scaffold domain can be shortened, modified or both, it is important that the resulting CC- or derivatized-chimeric N-peptide retains the ability to properly present the N-peptide coiled-coil region (*i.e.*, retains the ability to generate a stable, faithful mimetic of the N-helix trimeric coiled-coil). This can be determined as per experiments disclosed in U.S. Provisional Patent Applications 60/576,062 and 60/636,724, *supra.* Many different examples of truncated and/or modified Suzuki-IZ-like and GCN4-pI_{Q}I-like scaffold domains are disclosed in U.S. Provisional Patent Applications 60/576,062 and 60/636,724, *supra;* each of which are herein incorporated by reference. Examples of said Suzuki-IZ-like and GCN4-pI_{Q}I-like scaffold proteins are listed in Table 1; however, this recitation in no way limits the present invention to methods of selectively shielding the scaffold proteins, as contained within a CC- or derivatized-chimeric N-peptide, listed in Table 1.

**Table 1: Representative "scaffold" proteins.**

| **Name** | **SEQ ID NO:** | **Sequence** |
|---|---|---|
| IZ | 14 | IKKEIEAIKKEQEAIKKKIEAIEK |
| EZ | 15 | IEKKIEEIEKKIEEIEKKIEEIEK |
| E17 | 83 | IEKKIEEIEKKIEEIEK |
| E17Glu | 84 | IEKKIEEIEEKIEEIEK |
| I10 | 16 | IKKKIEAIEK |
| IQ | 17 | RMKQIEDKIEEIESKQKKIENEIARIKK |
| 117 | 18 | IKKEIEAIKKEQEAIKK |
| SZ | 19 | IEKKIEAIEKKIEAIEKKIEAIEK |
| S17 | 20 | IEKKIEAIEKKIEAIEK |
| S10 | 21 | IEKKIEAIEK |
| E10 | 22 | IEEKIEEIEE |
| IQ15a | 25 | KQKKIENEIAAIKKL |
| IQ15b | 26 | KIKKIENEIARIKKL |
| IQ21a | 27 | KIEEIESKQKKIENEIARIKKL |
| IQ21b | 28 | KIEEIESKIKKIENEIARIKK |

In one embodiment of the present invention, the scaffold domain of the CC- and derivatized-chimeric N-peptides described herein may be specifically modified to facilitate their conjugation, and/or the conjugation of resulting coiled-coiled structures, to an immunogenic carrier or an affinity resin. For example, the "EZ" scaffold, consisting of the amino acid sequence IEKKIEEIEKKIEEEEKKIEEIEK (SEQ ID NO:32; "a" positions are underlined), was designed to facilitate conjugation of covalently-stabilized, trimeric coiled-coils described in U.S. Provisional Patent Applications 60/576,062 and 60/636,724 (*supra*) to *Neisseria meningitidis* Outer Membrane Proteosome Complex ("OMPC") particles, an immunogenic carrier. The covalent addition of a peptide to a carrier (*i.e.,* to prepare a carrier-peptide conjugate) frequently results in irreversible precipitation of the conjugate, as is the case with OMPC-peptide conjugates. To prevent this, the maximum number of peptide moles that can be incorporated on a mole of OMPC (usually referred to as peptide "load") has to be carefully controlled. A strategy was recently developed that enables the straightforward conjugation of a peptide of any sequence to OMPC and ensures that the peptide load will be above a certain threshold, critical to obtaining a satisfactory immune response against the peptide epitope (see Bianchi et al., U.S. Provisional Patent Application, Serial No. 60/530,867, "A Method to Make a Peptide-Carrier Conjugate with a High Immunogenicity," filed on December 18, 2003). The method entails manipulation of the pI (isoelectric point) of the peptide sequence, adjusting its value to, optimally, between approximately 3.5-5.0. In order to achieve a pI within this range, the peptide can be modified outside of its active, immunogenic domain, ensuring maintenance of the peptide's immunological properties. For example, IZN17 (SEQ ID NO: 1) has a very high pI of 10.7, likely due to the large number of basic amino acid residues present within the IZ scaffold. The EZ scaffold was designed to have a more acidic character, facilitating the conjugation of the chimeric peptides comprising the scaffold, as well as the coiled-coil structures containing them, to OMPC. The Suzuki-IZ scaffold which was used to generated the IZ scaffold has a heptad repeat sequence of (IEKKIEA)ₙ (SEQ ID NO:99), as designed by Suzuki et al., 1998, *supra.* This heptad repeat sequence was modified in the EZ scaffold to (IEKKIEE)ₙ (SEQ ID NO:100) by mutating the alanine residue at position "c" of the Suzuki-IZ heptad repeat to glutamic acid residues. With a pI of 5.2, the resulting chimeric N-peptide, EZN17
(IEKKIEEIEKKIEEIEKKIEEIEKLLQLTVWGIKQLQARIL; SEQ ID NO:98), is close to the most favorable range for efficient OMPC conjugation.

As mentioned, it is critical that proper helical structure is maintained when generating the various chimeric peptides that can be polymer-modified as per methods described as part of the present invention, described above. For example, IZN-chimeric peptides, and CCIZN-derivatives thereof, having HIV N-peptide sequence segments longer than N17 *(e.g.,* "N23" or "N36") may comprise a modified IZ domain, extended by from one to a few amino acids, to ensure that the α-helical heptad repeat is maintained during the fusion event joining the IZ and longer HIV N-peptides. IZN23 and IZN36 are chimeric N-peptides also disclosed in Eckert and Kim, 2001 (*supra*) which can be covalently-stabilized within a trimeric coiled-coil and polymer-modified to selectively mask epitopes within the scaffold domain as described by the present invention. The amino acid sequences of IZN23 and IZN36 are as follows: *IKKEIEAIKKEQEAIKKKIEAIEKE*IEAQQHLLQLTVWGIKQLQARIL(SEQ ID NO:23) and *IKKEIEAIKKEQEAIKKKIEAIEKEI*SGIVQQQNNLLRAIEAQQHLLQLTVWGIKQLQARIL (SEQ ID NO:24), respectively. The scaffold domains of IZN23 and IZN36 are in italics, and the "a" positions of the peptides are underlined. In comparison to the IZ scaffold used to make IZN17 and its CCIZN-derivative, CCIZN17, the scaffold domains of IZN23 and IZN36 are extended by one (IZN23) or two (IZN36) amino acid residues to maintain proper "a" through "g" spacing, facilitating generation of a stable, α-helical conformation. The amino acids chosen to extend the scaffold domain in this manner should enable electrostatic interaction between adjacent helices (see Suzuki et al., 1998, *supra*).

The present invention relates to vaccine constructs having a core structure of a covalently-stabilized, trimeric coiled-coil as described in detail previously (see U.S. Provisional Patent Applications 60/576,062 and 60/636,72) that are modified by the selective attachment of one or more small molecular weight polymers, including but not limited SMW PEG moieties, in order to mask one or more epitopes within said protein structure and, thus, refocus and/or concentrate immune responses produced against said construct toward the unshielded region. As described above, in one embodiment of the present invention, said vaccine constructs are comprised of three chimeric peptides, each comprising a soluble scaffold protein fused in helical phase to all or a portion of the N-helix heptad region of the HIV gp41 ectodomain (the "N-peptide" portion). The HIV-1 gp41 ectodomain represents, approximately, 169 amino acid residues (residues 512-681 as numbered according to their position in the HIV-1 gpl60 envelope protein). Two 4-3 heptad repeat regions located within the NH₂- and COOH-terminal portions of the ectodomain are predicted to form α-helices, designated as the N- and C-helix regions, respectively. The N-helix and C-helix domains are located, approximately, at amino acid positions 541-592 and 623-663 of gp160, respectively (see, *e.g.*, Caffrey et al., 1998, EMBO J. 17:4572-4584). A core fusion-active structure (*i.e.*, "trimer-of-hairpins") of the gp41 ectodomain consists of a trimer of two interacting peptides, referred to as N36
(SGIVQQQNNLLRAIEAQQHLLQLTVWGIKQLQARIL; SEQ ID NO:29) and C34 (WMEWDREINNYTSLIHSLIEESQNQQEKNEQELL; SEQ ID NO:30), located within the N-helix and C-helix regions, respectively (Chan et al., 1997, Cell 89:263-273; see also U.S. Patent No. 6,506,554). N36 includes amino acid residue 546 through and including amino acid residue 581, numbered according to position in HIV-1 gp160. Other groups have crystallized helical domains of RV-1 gp41 that form stable, six-helix bundles slightly larger than those disclosed by Chan et al. For example, Weissenhorn et al. (1997, Nature 387:426-430) identified a N-peptide region slightly longer than N36 consisting of five additional amino acid residues at the NH₂-terminus and seven additional amino acid residues at the COOH-terminus of the N36 sequence:
ARQLLSGIVQQQNNLLRAIEAQQHLLQLTVWGIKQLQARILAVERYLK (SEQ ID NO:31).

Therefore, the N-peptide region of the CC- and derivatized-chimeric N-peptides that may be polymer-modified as per the present invention is not necessarily limited to all or a portion of the N36 region disclosed above. Thus, in one embodiment, the CC- and derivatized-chimeric N-peptides of which the scaffold domain is polymer-modified as described herein may comprise at least the 17 amino acids located at the COOH-terminal half of N36 ("N17"), corresponding to residues 565-581 of the HIV-1 gp160 sequence (LLQLTVWGIKQLQARIL; SEQ ID NO:32). In a further embodiment, longer or shorter segments of the N-peptide region, in comparison to N17, may be fused to a polymer-modified scaffold domain as described above (*e.g.*, "N23" - IEAQQHLLQLTVWGIKQLQARIL; SEQ ID NO:33). Extending the N-peptide domain may be useful in overcoming a putative immunogenic edge effect identified within N17 (see U.S. Provisional Patent Applications 60/576,062 and 60/636,724; *supra).* The trimer-of-hairpins structure is also punctuated by deep cavities, called hydrophobic pockets. One such cavity is located at the base (*i.e.*, the COOH-terminal half) of each groove of the N36 α-helix and is filled by a knob-like protrusion from a juxtaposed C34 helix, creating a ball-and-socket arrangement (see Cao et al., 1993, J. Virol. 67:2747-2755). Thus, the CC- and derivatized-chimeric N-peptides may comprise a portion of the gp41 N-helix that includes the amino acid residues which form the hydrophobic pocket or cavity of the central, N-helix coiled-coil which lies in the COOH-terminal half of the N-helix α-helical domain.

The HIV N-peptide region of the vaccine constructs described herein may also be modified in comparison to the wild-type amino acid sequence. For example, an immunodominant, non-neutralizing epitope located within the COOH-terminus of N17 was identified by alanine-scanning of the IZN17 peptide (see U.S. Provisional Patent Applications 60/576,062 and 60/636,724). This undesirable immunodominant response may be overcome by mutating one or more of the amino acid residues of N17 thought to contribute to the non-neutralizing epitope (leucine-581, arginine-579, glutamine-577, and/or glutamine-575). In one example, N17 can be modified such that each of the four amino acid residues believed to contribute to the immunodominant epitope are replaced with alanine residues, generating "N17Ala4" consisting of the following amino acid sequence: LLQLTVWGIKALAAAI (SEQ ID NO:34). Alternatively, said non-neutralizing, immunodominant region can be truncated or removed entirely in efforts to eliminate the unfavorable antigenic response *(e.g.,* "N13" - LLQLTVWGIKQLQ; SEQ ID NO:35). The N-peptide portion of the chimeric peptides of the present invention can also be modified to further stabilize the peptide as a whole. For example, the N-peptide domain can be modified to incorporate more stabilizing isoleucine residues into the sequence (*e.g.*, "N17Ile" - LIQLIVWGIKQIQARIL; SEQ ID NO:36).

As described *supra,* a preferred embodiment of the trimeric coiled-coil vaccine constructs that are polymer-modified as per the methods described herein are drawn to mimetics of HIV fusion intermediates having a portion of the HIV gp41 N-helix region fused in helical phase to an α-helical stabilizing scaffold domain. Said N-helix portion of HIV gp41 can be isolated from HIV-1, HIV-2, another HIV strain or a strain from another lentiviral species (*e.g.*, simian immunodeficiency virus (SIV), feline immunodeficiency virus (FIV) or Visna virus). To this end, the corresponding N-peptide sequences in similar HIV strains and/or immunodeficiency viruses of other species can be easily identified and are known in the art. For example, the amino acid sequences of other lentiviral NH₂-terminal α-helical domains that align to the N36 N-peptide sequence of HIV-1 gp41 are listed in Table 2. Additionally, α-helical, coiled-coil domains have been identified in the membrane-fusion proteins of other enveloped viruses (see Singh et al., 1999, *supra).*

**Table 2: Lentivirus NH₂-terminal heptad domain.**

| **Virus** | **Sequence** | **GenBank Acc. No.** |
|---|---|---|
| Simian immunodeficiency virus (SIV) | AGIVQQQQQLLDVVKRQQELLRLTVWGTKNLQTRVS (SEQ ID NO:37) | P05885 |
| Visna virus | QSLANATAAQQNVLEATYAMVQHVAKGVRILEARVA (SEQ ID NO:38) | JQ1165 |
| Caprine arthritis encephaliti virus (CAEV) | QTLANATAAQQDALEATYAMVQHVAKGVRILEARVA (SEQ ID NO:39) | P31627 |
| Feline immunodeficiency virus (FIV) | ATHQETIEKVTEALKINNLRLVTLEHQVLVIGLKVE (SEQ ID NO:40) | Q04995 |
| Equine infectious anemia virus (EIAV) | NHTFEVENSTLNGMDLIERQIKILYAMILQTHADVQ (SEQ ID NO:41) | P22427 |
| Bovine immunodeficiency virus (BIV) | ERVVQNVSYIAQTQDQFTHLFRNINNRLNVLHRRVS (SEQ ID NO:42) | P19556 |

The production of chimeric N-peptides, as defined herein (*e.g*., IZN17), are described in detail above and even more specifically in U.S. Provisional Patent Applications 60/576,062 and 60/636,724, *supra.* Said chimeric N-peptides can be modified with the addition of either at least two cysteine residues (*i.e.,* "CC-chimeric N-peptide") or an electrophilic moiety (*i.e.,* "derivatized-chimeric N-peptide") at the NH₂- or COOH-terminus of the core chimeric peptide sequence to impart the ability for said peptides to generate stabilizing disulfide and/or thioether bonds, respectively, resulting in covalently-stabilized, trimeric coiled-coil structures. In one embodiment, at least two cysteine amino acid residues are added outside of the core α-helical domain of the chimeric N-peptides, generating a "CC-chimeric N-peptide," which can form disulfide bonds with adjacent CC-chimeric N-peptides under oxidizing conditions. The extra-helical placement of the additional cysteine residues minimizes interference between the coiled-coil conformation of said peptides and the stabilizing disulfide bonds. When three CC-chimeric N-peptides physically associate in a parallel orientation, a result of the α-helical structure of the individual polypeptide monomers, the cysteine residues are in close approximation. Under oxidizing conditions, these juxtaposed cysteine residues will spontaneously form disulfide bridges among the three chains. In another embodiment, the thiol-reactive functional group present in the engineered cysteine amino acids residues of a CC-chimeric N-peptide can form covalent, chemoselective bonds with electrophilic moieties (*e.g.*, an alkyl halide moiety or a Michael acceptor) on adjacent derivatized N-peptides. Said electrophilic moieties are added to a terminus of a chimeric N-peptide and outside of its α-helical domain. Said cysteine residues or electrophilic moiety may be added to the NH₂-terminus or COOH-terminus of a core chimeric N-peptide. The added cysteine residues or electrophilic moieties of the CC- and derivatized-chimeric N-peptides, respectively, may be separated from the α-helical portion of the chimeric peptides by a linker or spacer region. The spacer region acts to break the helical structure of the chimeric N-peptide, giving the terminal cysteine residues or electrophilic moiety higher conformational freedom to participate in covalent bond formation. The spacer/linker region can comprise a short amino acid sequence known to have the ability to break α-helical secondary structure (*e.g.*, a glycine or proline residue; or any single D-amino acid, the dextrorotatory isomer of amino acids). It is preferred that a minimal number of residues required to break the α-helical structure is used as a spacer. Alternatively, the spacer/linker region can comprise a chemical or synthetic linker, *e.g.*, aminopentanoic acid, aminohexanoic acid, or any amino acid without a C-alpha side chain.

A preferred covalently-stabilized, trimeric coiled-coil that can be polymer-modified via the attachment of one or more small molecular weight polymer molecules to one or more amino acid residues located within the scaffold domain, effectively shielding one or more epitopes within said scaffold domain, and focusing the immune response to the unshielded portion (e.g., HIV portion) of the chimeric peptides, is (CCIZN17)₃ (SEQ ID NO:2)₃; see Figure 1. This coiled-coil structure is comprised of three, identical CCIZNN17 chimeric peptides having the following amino acid sequence: CCGGIKKEIEAIKKEQEAIKKKIEAIEKLLQLTVWGIKQLQARIL (SEQ ID NO:2). As described earlier, CCIZN17 represents a CC-chimeric N-peptide wherein the core chimeric peptide sequence, "IZN17," consists of the IZ scaffold domain (underlined above) fused to the NH₂-terminus of N17 (SEQ ID NO:32). CCIZN17 further comprises a stabilizing Cys-Cys-Gly-Gly (SEQ ID NO:10) sequence added to the NH₂-terminus of the core chimeric peptide. The two, consecutive cysteine residues participate in disulfide bond linkages with juxtaposed cysteine residues on closely associated CC-chimeric N-peptides that are formed upon oxidation of the peptides. The two, consecutive glycine residues represent a spacer region, separating the cysteine residues from the a-helical domain of the core chimeric peptide sequence. The glycine spacer region ensures that the cysteine residues are not embroiled in the helical secondary structure of the core peptide sequence, helping to free said cysteine residues to participate in disulfide linkages.

Thus, one embodiment of the present invention is a covalently-stabilized, trimeric coiled-coil having a core structure of (CCIZN17)₃, wherein the scaffold domain of said coiled-coil structure, and in turn the epitopes contained within it, is selectively shielded as a result of the covalent attachment of one or more small molecular weight polymers to one or more amino acid residues located within or near said domain. In the exemplified vaccine construct described in detail in Example 1, *infra,* two lysine amino acid residues located within the scaffold domain are PEGylated with a commercially available PEG 236 moiety, generating what is herein designated as "CCIPN17." CCIPN17 has the same amino acid sequence as CCIZN17 (CCGG*IKKEIEAIKKEQEAIKKKIEAIEK*LLQLTVWGIKQLQARIL; SEQ ID NO:2); however, as stated, two lysine residues (underlined) located within the scaffold domain (italicized) are covalently modified with PEG 236 (see Figure 2). Thus, the scaffold domain of the resulting (CCIPN17)₃ vaccine construct is shielded with six PEG 236 moieties, two on each peptide monomer. Similar, small molecular weight, polymer-modified (including but not limited to PEG-modified) coiled-coil structures which represent mimetics of HIV gp41 fusion intermediates may be generated using the various chimeric peptides described herein, as well as those disclosed in U.S. Provisional Patent Applications 60/576,062 and 60/636,724 *(supra).* Examples of trimeric coiled-coil structure, each comprising three CC-chimeric N-peptides that are covalently-stabilized via disulfide bonds formed between said peptides, that can be polymer-modified as per the methods described as part of the present invention are disclosed in Table 3. Again, the recitation of the covalently-stabilized, trimeric coiled coil structures in Table 3 in no way limits the present invention to methods of selectively shielding the scaffold domains contained only within said examples.

**Table 3: Examples of covalently-stabilized mimetics of HIV fusion intermediates.**

| **Name** | **Peptide Sequence¹** |
|---|---|
| (CCIZ-N17Ala4)₃ | (*CCGGIKKEIEAIKKEQEAIKKKIEAIEK*LLQLTVWGIKALAAAIA)₃ (SEQ ID NO:43)₃ |
| (CCN17IZa)₃ | *(CCGG*LLQLTVWGIKQLQARILKKEIEA*IKKEQEAIKKKIEAI*)₃ (SEQ ID NO:74)₃ |
| (CCIZN13)₃ | (*CCGGIKKEIEAIKKEQEAIKKKIEAIEK*LLQLTVWGIKQLQ)₃ (SEQ ID NO:75)₃ |
| (CCEZN17)₃ | (*CCGGIEKKIEEIEKKIEEIEKKIEEIEK*LLQLTVWGIKQLQARIL)₃ (SEQ ID NO:44)₃ |
| (CCEZ-N17Ala4)₃ | (*CCGGIEKKIEEIEKKIEEIEKKIEEIEK*LLQLTVWGIKALAAAIL)₃ (SEQ ID NO:45)₃ |
| (CCIZN23)₃ | (*CCGGIKKEIEAIKKEQEAlKKKIEAIEKE*TEAQQHLLQLTVWGIKQLQARIL)₃ (SEQ ID NO:46)₃ |
| (CCEZN23)₃ | (*CCGGIEKKIEElEKKIEEIEKKlEEIEEK*IEAQQHLLQLTVWGIKQLQARIL)₃ (SEQ ID NO:47)₃ |
| (CCIZN36)₃ | (CCGGIKKEIEAIKKEQEAIKKKIEAIEKEISGIVQQQNNLLRAIEAQQHLLQLTVWGIKQLQ ARIL)3 (SEQ ID NO:48)3 |
| (CCEZN36)₃ | (CCGGIEKKIEEIEKKIEEIEKKIEEIEEKISGIVQQQNNLLRAIEAQQHLLQLTVWGIKQLQ ARIL)3 (SEQ ID NO:49)3 |
| (CCI10N17)₃ | (*CCGGIKKKIEAIEK*LLQLTVWGIKQLQARLR)₃ (SEQ ID NO:50)₃ |
| (SCCIZN17)₃ | (*SGGCCGGIKKEIEAIKKEQEAIKKKIEAIEK*LLQLTVWGIKQLQARIL)₃ (SEQ ID NO:51)₃ |
| (CCIQN17)₃ | (*CCGGRMKQIEDKIEEIESKQKKIENEIARIKK*LLQLTVWGIKQLQARIL)₃ (SEQ ID NO:52)₃ |
| (CCIQN17-Ala4)₃ | (*CCGGRMKQIEDKIEEIESKQKKIENEIARIKK*LLQLTVWGIKALAAAIA)₃ (SEQ ID NO:53)₃ |
| (CCIQN23)₃ | (*CCGGRMKQIEDKIEEIESKQKKIENEIARIKKL*IEAQQHLLQLTVWGIKQLQARIL)₃ (SEQ ID NO:69)₃ |
| (CCIQN36)₃ | (CCGGRMKQIEDKIEEIESKQKKIENEIARIKKLISGIVQQQNNLLRAIEAQQHLLQLTVWGI KQLQARIL)3 (SEQ ID NO:70)3 |
| (CCI17N17)₃ | (*CCGGIKKEIEAIKKEQEAIKK*LLQLTVWGIKQLQARIL)₃ (SEQ ID NO:54)₃ |
| (CCIZ-N17+7)₃ | (*CCGGIKKEIEAIKKEQEAIKKKIEAIEK*LLQLTVWGIKQLQARILAVERYLK)₃ (SEQ ID NO:55)₃ |
| (CCE-N17+7)₃ | (*CCGGIEKKIEEIEKKIEEIEKKIEEIEK*LLQLTVWGIKQLQARILAVERYLK)₃ (SEQ ID NO:71)₃ |
| (CCIQ-N17+7)₃ | (*CCGGRMKQIEDKIEEIESKQKKIENEIARIKK*LLQLTVWGIKQLQARILAVERYLK)₃ (SEQ ID NO:72)₃ |
| (CCI17N23)₃ | (*CCCGIKKEIEALKKEQEAIKKE*TEAQQHLLQLTVWGIKQLQARIL)₃ (SEQ ID NO:56)₃ |
| (CCI17N36)₃ | (*CCGGIKKEIEAIKKEQEAIKKEI*SGIVQQQNNLLRAEEAQQHLLQLTVWGIKQLQARIL)₃ (SEQ ID NO:57)₃ |
| (CCI17 -N17+7)₃ | (*CCGGIKKEIEAIKKEQEAIKK*LLQLTVWGIKQLQARELAVERYLK)₃ (SEQ ID NO:58)₃ |
| (CCII7-N17Ala4)₃ | (*CCGGIKKEAIKKEQEAIKK*LLQLTVWGIKALAAAIA)₃ (SEQ ID NO:59)₃ |
| (I10N17CC)₃ | (*IKKKIEAIEK*LLQLTVWGIKQLQARIL*GGCC*)₃ (SEQ ID NO:60)₃ |
| (CCI10-N17Ile)₃ | (*CCGGIKKKIEAIEK*LIQLIVWGIKQIQARIL)₃ (SEQ ID NO:61)₃ |
| (110-N17IleCC)₃ | (*IKKKIEAIEK*LIQLIVWGIKQIQARIL*GGCC*)₃ (SEQ ID NO:62)₃ |
| (CCI10-N17+7)₃ | (*CCGGIKKKIEALEK*LLQLTVWGIKQLQARILAVERY)₃ (SEQ ID NO:73)₃ |
| (CCI17-N17Ile)₃ | (*CCGGIKKEIEAIKKEQEAIKK*LIQLIVWGIKQIQARIL)₃ (SEQ ID NO:63)₃ |
| (CCN17IZb)₃ | (*CCGG*LLQLTVWGIKQLQARIL*AIKKEIEAKKEQEAIKKKIEAI*)₃ (SEQ ID NO:64)₃ |
| (CCSZN17)₃ | (*CCGGIEKKIEAIEKKIEAIEKKIEAIEK*LLQLTVWGIKQLQARIL)₃ (SEQ ID NO:65)₃ |
| (CCS17N17)₃ | (*CCGGIEKKIEAIEKKIEAIEK*LLQLTVWGIKQLQARIL)₃ (SEQ ID NO:66)₃ |
| (CCS10N17)₃ | (*CCGGIEKKIEAIEK*LLQLTVWGIKQLQARIL)₃ (SEQ ID NO:67)₃ |
| (CCE10-N17)₃ | (*CCGGIEEKIEEIEE*LLQLTVWGIKQLQARIL)₃ (SEQ ID NO:68)₃ |
| (CCE17N17)₃ | (*CCGGIEKKIEEIEKKIEEIEK*LLQLTVWGIKQLQARIL)₃ (SEQ ID NO:85)₃ |
| (CCE17Gtu-N17)₃ | (*CCGGIEKKIEEIEEKIEEIEK*LLQLTVWGIKQLQARL)₃ (SEQ ID NO:86)₃ |

| | |
|---|---|
| ¹ in italics, non-HIV residues; underlined, "cysteine" portion (including glycine linker); NH₂-terrninus acetyl (except with biotinylated and SCC-peptides), COOH-terminus amide | |

As stated above, each of the covalently-stabilized, trimeric coiled-coil structures in Table 3 is comprised of three CC-chimeric N-peptides that are covalently-stabilized via the formation of disulfide bonds between the cysteine amino acid residues located outside the a-helical domain of the core peptide structure. In the alternative, and as described previously, the trimeric coiled-coil vaccine structures which can be polymer-modified as per the present invention can also be covalently-stabilized via the formation of chemoselective bonds. For example, two chemoselective bonds can be formed between the electrophilic moieties of two derivatized-chimeric N-peptides and a thiol-functional group of each of two added cysteine amino acid residues of one CC-chimeric N-peptide, forming two thioether, covalent bonds. For example, a trimeric coiled-coil mimetic designated NH₂-C(Fm)(thioI2N17)₃, described in detail in U.S. Provisional Patent Applications 60/576,062 and 60/636,724 *(supra),* can be polymer-modified as per the present invention. This trimeric coiled-coil is comprised of the following peptide monomers:
1) NH₂-C(Fm)Ttds-CCIZN17 (peptide 1 in Table 4): a peptide having two cysteine residues separated from the scaffold domain through a flexible spacer (-GG-; other flexible spacers are suitable) and a third cysteine residue with a protected thiol group for later conjugation (a fluorenylmethoxy (Fm) protecting group is used, but other protecting groups are suitable); and,
2) bromoacetyl-GGGIZN17 (peptide 2 in Table 4): a peptide having a bromoacetyl moiety separated from the scaffold domain through a flexible linker (-GGG-; other flexible spacers are suitable, provided they are compatible with the formation of two thioether bonds between two derivatized-chimeric N-peptide chains and one CC-chimeric N-peptide).

The two peptide precursors, NH₂-C(Fm)Ttds-CCIZN17 (peptide 1 in Table 4) and Br-acetyl-GGGIZN17 (peptide 2 in Table 4), can be incubated at an approximate concentration ratio of 1:2, respectively, to generate a trimeric coiled-coil having one CC-chimeric N-peptide (peptide 1) and two derivatized N-peptides (peptide 2). The chemoselective ligation reaction proceeds with formation of two thioether bonds, wherein each bromoderivative of the two Br-acetyl-GGGIZN17 peptides reacts with one of the two, unprotected cysteine residues of the NH₂-C(Fm)Ttds-CCIZN17 peptide (see Figure 8). Once the covalently-stabilized trimeric coiled-coil has formed, the protecting group can be removed to facilitate conjugation of said trimeric coiled-coil (e.g., to a purification resin or OMPC).

For demonstrative purposes only, additional peptide monomers that may be used to generate covalently-stabilized, trimeric coiled-coil structures via the formation of chemoselective bonds which can be polymer-modified as per the present invention are listed in Table 4. For example, a stabilized coiled-coil designated "ac-C(thioIZN17)₃" may be synthesized which comprises one ac-C(Acm)Ttds-CCIZN17 peptide (peptide 3 of Table 4) and two Br-acetyl-GGGIZN17 peptides (peptide 2 of Table 4). The resulting trimeric coiled-coil has Acm (acetamidomethyl group) as the protecting group for the NH₂-terminal cysteine residue, which can be removed to facilitate later conjugation.

**Table 4: Examples of IZN17-, S17-, E10-, EZ-, E17-, and El7Glu-based chimeric peptides for participation in chemoselective ligation reactions generating thioether bonds between said peptides.**

| **Peptide No.¹** | **Peptide Name** | **Peptide characteristics²** |
|---|---|---|
| 1 | NH₂-C(Fm)Ttds-CCIZN17 | NH2-C(Fm)Ttds-CCGGIKKEIEAIKKEQEAIKKKIEAIEKLL QLTVWGIKQLQARIL (SEQ ID NO:76) |
| 2 | Br-acetyl-GGGIZN17 | Br-acetyl-GGGIKKEIEAIKKEQEAIKKKIEAIEKLLQLTVW GIKQLQARIL (SEQ ID NO:77) |
| 3 | ac-C(Acm)Ttds-CCIZN17 | Acetyl-C(Acm)Ttds-CCGGIKKEIEAIKKEQEAIKKKIEAIEK LLQLTVWGIKQLQARIL (SEQ ID NO:76) |
| 4 | ac-C(Acm)Ttds-CCS 17N 17 | Acetyl-C(Acm)Ttds-CCGGIEKKIEAIEKKIEAIEKLLQLTV WGIKQLQARIL (SEQ ID NO:78) |
| 5 | Br-acetyl-GGGS17N17 | Br-acetyl-GGGIEKKIEAIEKKIEAIEKLLQLTVWGIKQLQA RIL (SEQ ID NO:79) |
| 6 | Br-acetyl-GGGE10N17 | Br-acetyl-*GGGIEEKIEEIEE*LLQLTVWGIKQLQARIL (SEQ ID NO:80) |
| 7 | ac-C(Acm)Ttds-CCE10N17 | Acetyl-C(Acm)Ttds-CCGGIEEKIEEIEELLQLTVWGIKQL QARIL (SEQ ID NO:81) |
| 8 | Br-acetyl-GGGE17GluN17 | Br-acetyl-GGGIEKKIEEIEEKIEEIEKLLQLTVWGIKQLQA RIL (SEQ ID NO:87) |
| 9 | ac-C(Acm)Tds-CCE17GluN17 | Acetyl-C(Acm)Ttds-CCGGIEKKIEEIEEKIEEIEKLLQLTV WGIKQLQARIL (SEQ ID NO:88) |
| 10 | Br-acetyl-GGGEZNl7 | Br-acetyl-GGGIEKKIEEIEKKIEEIEKKIEEIEKLLQLTVW GIKQLQARIL (SEQ ID NO:89) |
| 11 | ac-C(Acm)Tds-CCEZN17 | Acetyl-C(Acm)Ttds-CCGGIEKKIEEIEKKIEEIEKKIEEIEK LLQLTVWGIKQLQARIL (SEQ ID NO:90) |
| 12 | Br-acetyl-GGGE17N17 | Br-acetyl-GGGIEKKIEEIEKKIEEIEKLLQLTVWGIKQLQA RIL (SEQ ID NO:91) |
| 13 | ac-C(Acm)Tds-CCE 17N 17 | Acetyl-C(Acm)Ttds-CCGGIEKKIEEIEKKIEEIEKLLQLTV WGIKQLQARIL (SEQ ID NO:92) |

| | | |
|---|---|---|
| ¹ Peptide sequence 1 has a free amino group on the NH₂-terminal cysteine. ² in italics, non-HIV residues; single underlined, "cysteine" portion of CC-chimeric N-peptides (including cysteine residues and glycine linker); double-underlined, linker region of derivatized-chimeric N-peptides (glycine residues) or CC-chimeric N-peptides (synthetic Ttds linker) | | |

A variety of means have been used to attach polymer moieties, such as PEG and related polymers, to reactive groups found on proteins; see, *e.g.,* U.S. Patent No. 4,179,337, issued to Davis et al., and U.S. Patent No. 4,002,531, issued to Royer. For a review, see Abuchowski et al., in Enzymes as Drugs, Eds. J. S. Holcerberg and J. Roberts, 1998, pp. 367-383 and Zalipsky, S, 1995, Bioconjugate Chem. 6:150-165. The use of PEG and other polymers to modify proteins is also discussed by Cheng, T.-L. et al., 1999, Bioconjugate Chem. 10:520-528; Belcheva, N. et al., 1999, Bioconjugate Chem. 10:932-937; Bettinger, T. et al., Bioconjugate Chem. 9:842-846; Huang, S.-Y. et al., 1998, Bioconjugate Chem. 9:612-617; Schwarz, J.B. et al., 1999, J. Amer. Chem. Soc. 121:2662-2673; Reuter, J.D. et al., 1999, Bioconjugate Chem. 10:271-278; and, Chan, T.-H. et al., 1997, J. Org. Chem. 62:3500-3504. As mentioned previously, typical polymer attachment sites in proteins include primary amino groups, such as those on lysine residues or at the NH₂-terminus; thiol groups, such as those on cysteine side-chains; and carboxyl groups, such as those on glutamate or aspartate residues or at the COOH-terminus. The attachment of water-soluble polymers, such as PEG, to polypeptides of interest (*e.g.*, to improve circulating half-life, reduce proteolysis and immunogenicity) has been met with mixed results given the difficulty of attaching the polymer moieties in a controlled manner and with user-defined precision. In the past, because of both the stochastic nature of attachment and the hetero-disperse nature of the water-soluble polymers, purification and analytical characterization of polymer-protein conjugates was difficult.

As a preferred method for producing the SMW polymer-modified polypeptides as described herein, one or more SMW polymers are attached to site-specific locations within the protein of interest. This is especially important when, as in the present invention, a specific biologically active site(s) within the protein is intended to be shielded by the attached polymer, while leaving alternative sites free to induce or facilitate an intended a biological response (*e.g.*, an immune response). Preparation of small molecular weight polymer-derivatized proteins (*e.g.*, SMW PEGylated proteins) wherein said polymer is site-specifically attached to the protein can be achieved by various methods, allowing for full control over the number and location of the attached chains. For example, in an attempt to reduce the random attachment of polymers, proteins have been made in which natural lysine or cysteine amino acids are removed in conjunction with the adding back of lysine/cysteine amino acids at desired sites for polymer attachment (see, *e.g.,* U.S. Patent No. 4,904,584). Alternatively, the vaccine construct exemplified in Example 1 *infra,* (CCIPN17)₃, was prepared via Solid-Phase Peptide Synthesis ("SPPS"). Thus, the synthesis of peptides and/or polypeptides to be modified as per the present invention may employ a stepwise standard Boc and/or Fmoc solid phase peptide synthesis using standard automated peptide synthesizers, or manually, following standard protocols, or ordered and purchased from commercial vendors. See, *e.g.,* Synthetic Peptides, A User's Guide, Ed. G.A., New York:W. H. Freeman & Company, 1992; Principles of Peptide Synthesis, 2nd ed., Ed. M. Bodanszky, Springer-Verlag, 1993; The Practice of Peptide Synthesis, 2nd ed., Eds. M. Bodanszlky and A. Bodanszky, Springer-Verlag, 1994; Protecting Groups, Ed. P. J. Kocienski, Stuttgart, Germany:Georg Thieme Verlag, 1994; and, Fmoc Solid Phase Peptide Synthesis, A Practical Approach, Eds. W.C. Chan and P.D. White, Oxford University Press, 2000.

Using chemoselective methods, such as native chemical ligation (Dawson, P.E. and S.B. Kent, 2000, Ann. Rev. Biochem. 69:923-60), larger polymer-modified proteins are also easily accessible via total chemical synthesis. An excellent illustrative example of this is demonstrated by Kochendoerfer, G.G. et al. (2003, Science 299:884-87), with further description in Kochendoerfer, G.G. et al., U.S. Non-Provisional Application Serial No. 10/332,385, US Publication No. US2004/0115774, wherein the authors/inventors prepared a 166-amino acid protein comprising two, site-specific, HWM branched PEG molecules in a cumulative yield of 25-40%. The chemistry described in this work for the attachment of HMW branched PEG is easily adaptable to the introduction of SMW PEG moieties, although many more alternatives are also available, see for example (Harris, J.M. and R.B. Chess, 2003, Nat. Rev. Drug Discov. 2:214-221), and are known to the skilled artisan. The size of the protein synthesized in Kochendoerfer et al, 2003 *(supra),* SEP, is comparable to the size of the gp 120 subunit of the HIV envelope glycoprotein, for which a strategy of epitope masking based on the selective addition of sugar residues has been proposed. It is thus evident that an HIV vaccine based on gp120 that is selectively derivatized with SMW PEG in order to diminish or entirely remove a particular immune reactivity in favor of another is within the reach of a total chemical synthesis method.

An alternative method for the production of polymer-modified proteins as described herein is to attach the SMW polymer (*e.g.*, PEG) chemoselectively to a suitable precursor protein produced by recombinant DNA methods. More specifically, Bertozzi and co-workers developed a generally applicable strategy for the incorporation of an azide group into any position within a recombinant protein (Kiick, K.L. et al., 2002, Proc. Natl. Acad. Sci. U.S.A. 99:19-24). The method consists of utilizing a non-natural amino acid which is processed by the cell's native translational apparatus. Azidohomoalanine (a methionine surrogate) is activated by the methionyl-tRNA synthetase of *Escherichia coli,* and replaces methionine in proteins expressed in methionine-depleted bacterial cultures. Azides can participate in an exquisitely chemoselective reaction, termed Staudinger ligation, with phosphanes, ultimately resulting in the formation of a stable amide bond joining the protein and the moiety attached to the phosphane (see Kohn, M. and R. Breinbauer, 2004, Angew Chem. Int. Ed. Engl. 43:3106-16; Saxon, E. et al., 2000, Org. Lett. 2:2141-3; Saxon, E. and C.R. Bertozzi, 2000, Science 287:2007-10). For example, Staudinger ligation of PEG 236, the SMW PEG utilized in CCIPN17 (see Example 1), can be carried out with the corresponding phosphinothioester 1, which would ligate to azidohomoalanine according to Scheme 1 below:

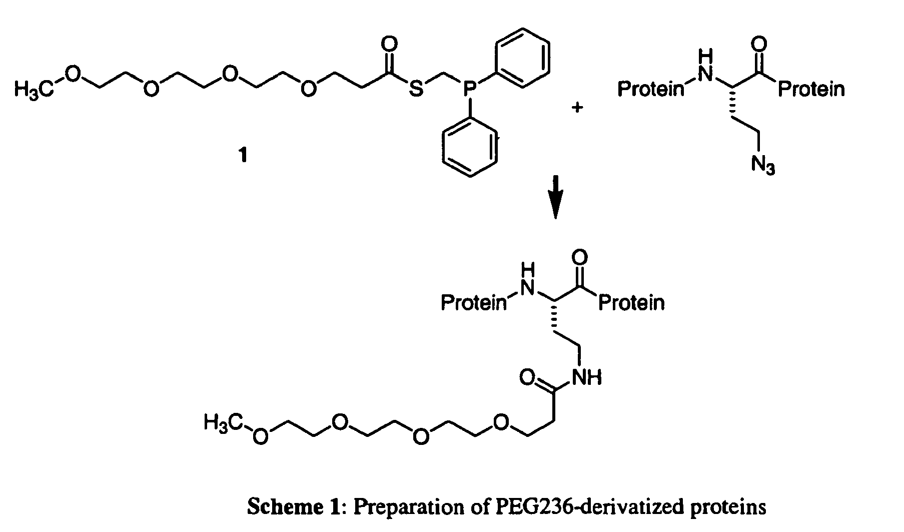

Synthesis of phosphinothioesters of longer chain PEG has been described (Soellner, M.B. et al., 2003, Am. Chem. Soc. 125:11790-1). Since no limitation in the protein size exists *a priori* for the production of recombinant proteins containing azidohomoalanine, methods exist in the prior art to prepare proteins of any length with SMW polymer chains attached at any selective location within the primary sequence.

In designing the polymer-modified polypeptides of the invention, the amino acid sequence of a target protein is initially obtained, typically, from any number of various sources including databases such as gene and/or protein databases, or de novo, for instance, through proteomic identification of a novel protein target of interest. Examples of such databases include, but are not limited to, GeneBank (Benson, et al., 1998, Nucleic Acids Res. 26:1-7; USA National Center for Biotechnology Information, National Library of Medicine, National Institutes of Health, Bethesda, Md., USA), TIGR Database (The Institute for Genomic Research, Rockville, Md., USA), Protein Data Bank (Brookhaven National Laboratory, USA), and the ExPASy and Swiss-Protein database (Swiss Institute of Bioinformatics, Geneve, Switzerland). The polypeptide backbone employed for the design strategy can represent a portion (*e.g.*, a domain) or all of a target sequence (*e.g.*, a mature form of the polypeptide chain). The desired, polymer-modified polypeptide structure is then designed for a given target protein. This design strategy includes the choice of water-soluble polymer with which to modify the polypeptide, the site(s) of polymer attachment within the polypeptide, and the attachment strategy to be used. In particular to the present invention, the polypeptide amino acid sequence is first reviewed for the presence of one or more functional sites (*e.g.*, glycosylation sites, immunogenic sites, or protease sensitive site) in order to identify and assess which of said sites should/could be specifically shielded by attachment of a SMW polymer. Biologically active sites often represent regions that are substantially devoid of helix or beta-sheet secondary structure; typically loop and turn regions. Thus, the identification of functional sites to be masked via attachment of a SMW polymer as described herein can be determined using modeling approaches. Modeling includes bioinformatic approaches, such as the use of homology modeling software algorithms or programs that can compare either portions or up to all of a given nucleic acid or amino acid sequence of a target protein, two-dimensional structure information for a polypeptide of interest, and three-dimensional structure information for a protein of interest, or a combination thereof. Any number of software programs and databases are well known and available for this purpose. An example of a software program that integrates these methods is "MAXVECTOR" (Oxford Molecular Group), which contains multiple protein analysis tools for predicting secondary structure (Chou-Fasman and Robson-Garnier methods); hydrophilicity (Hopp-Woods, Kyte-Doolittle, Goldman-Engelman-Steitz (GES), and von-Heijne methods); hydrophobicity (Fauchere-Pliska, Janin, Manavalan, Sweet-Eisenberg methods); antigenicity (Hopp-Woods, Parker, Protrusion Index (Thornton), Welling methods); surface probability (Karplus & Schultz's methods); flexibility (Janin and Emini methods); amphiphilicity (Eisenberg method); PROSITE-based subsequence database and user-defined subsequences; customized subsequence sets; and, protease site and proteolytic digestion predictions. Thus, it may be helpful to take into account the three dimensional structure and chemistry of the target natural protein or mimetic when selecting the location of polymer attachment.

For example, to predict immunogenic sites in proteins, the following approach can be used. The secondary structure of the protein sequence is first predicted, and disordered regions, particularly turns, are identified. Chou-Fasman parameters are particularly useful for this purpose (Chou et al., 1978, Advanced Enzymology 47:45-148; Qian et al., 1988, J. Mol. Biol. 202:865-884; and, "PEPEPLOT" program from Genetics Computer Group, Inc.). The protein sequence is also scanned for hydrophobicity, typically using a running average of six residues following standard methods (*e.g.,* Fauchere-Pliska or Hopp-Woods hydrophobicity prediction methods; or Bull-Breese). The more hydrophilic regions are identified with particular attention to those that are turns. In conjunction, glycosylation sites, including N-linked sites and O-linked sites are identified. The most hydrophilic site in the protein, if it is in a turn and not predicted to be glycosylated, may have a high probability of immunogenicity and, thus, serve as a site for polymer modification. The same basic rules can be applied to successively less hydrophilic regions to identify secondary sites for polymer modification.

Biological and chemical analysis also may be used to identify specific locations that may benefit from polymer attachment in as per the present invention. For example, alanine scanning can be used to identify immunogenic sites, protease sensitive sites or other sites suitable for polymer modification, and is particularly useful for identifying invariant residues required for biological activity. Alanine scanning is also useful when used in combination with other protein analysis tools, such as those described above. Another approach is to systematically prepare a library of synthetic proteins of interest with the modifying water-soluble polymer moiety being located at a different site in the polypeptide chain of each molecule. After folding the library of polymer-modified polypeptide chains, a functional selection/assay can be performed to separate, for example, folded from non-folded molecules, or receptor-binding from non-binding molecules. Then the preferable location of the sites of polymer modification can be determined for the functional molecules. Such an approach can make use of the principles of the "protein signature analysis" method to facilitate identification of non-interfering sites of polymer modification (Muir et al., 1996, Chem. Biol. 3:817-825). Given the various attachment chemistries described above, the bond formed between the water-soluble polymer and a target peptide or polypeptide can comprise a bond selected from carbonate, ester, urethane, orthoester, amide, amine, oxime, imide, urea, thiourea, thioether, thiourethane, thioester, ether, thiazolidine, hydrazone, oxazolidine and the like.

One aspect of the present invention relates to a vaccine that can be administered to a human or non-human mammalian subject to cause immunoprotection in the subject against a particular disease or condition (including but not limited to HIV infection/AIDS) and/or reduction of pathogenesis contributing to such a condition in said subject. In general, the vaccines of the present invention can confer immunoprotection against a number of pathogenic organisms, including but not limited to fungi, protozoa, bacteria, and viruses (*e.g.*, influenza virus and HIV, in particular). In one embodiment of the present invention, the vaccine comprises a polymer-modified form of a covalently-stabilized, trimeric coiled-coil structure described herein and in further detail in U.S. Provisional Patent Applications 60/576,062 and 60/636,724 *(supra)* which represents a mimetic of fusion intermediates of enveloped viral proteins, including but not limited to HIV gp41. In one embodiment of the present invention, one or more of the individual chimeric peptides comprised within the covalently-stabilized coiled-coil mimetics are modified with a small molecular weight, water-soluble polymer, including but not limited to PEG, such that all or a portion of the stabilizing scaffold domain of the coiled-coil, and thus one or more epitopes contained within, are selectively is shielded from interacting with immune-derived receptors/cells by the polymer. Thus, the present invention is related to a method of immunizing a mammal or inducing a protective immune response against a pathogenic organism by administering a therapeutically effective amount (as defined *infra*) of the polymer-modified vaccine constructs described herein. This method includes the step of administering to a mammal a vaccine that comprises a polymer-modified form (*e.g*., modified with one or more SMW PEG moieties) of all or a portion of a native pathogenic antigen either existing alone or coupled (*e.g.*, as a fusion peptide/protein) with non-native polypeptide components (*e.g.*, the scaffold coiled-coil as described *supra*). The addition of the polymer will immunodampen the region shielded by the presence of said polymer, thus focusing the immune response to the unmasked portions of the antigen. Thus, one embodiment of the present invention relates to HIV pharmaceutical products, as well as the production and use thereof (as described herein) which, when directly introduced into living vertebrate tissue, preferably a mammalian host such as a human or a non-human mammal of commercial or domestic veterinary importance, induce a cellular immune response which specifically recognizes human immunodeficiency virus-1 (HIV-1).

Thus, a preferred use of the polymer-modified polypeptides of the present invention is in the treatment of disease conditions in mammals, *e.g.*, HIV infection, via administering a therapeutically effective amount of said polypeptides in the form of a vaccine to said mammal. This includes any treatment of a disease in a human or non-human mammalian subject, such as: (i) preventing the disease from occurring in a subject which may be predisposed to the disease but has not yet been diagnosed with it; (ii) inhibiting and/or arresting development of the disease; or, (iii) relieving the disease, *i.e.*, causing regression of the disease. As used herein, the term "therapeutically effective amount" refers to that amount of a polymer-modified polypeptide and/or a vaccine composition comprising said polypeptide of the invention which, when administered to a mammal, is sufficient to affect treatment, for example, to inhibit HIV-1 infection in mammals. The amount that constitutes a "therapeutically effective amount" will vary depending on the polypeptide, the condition or disease and its severity, and the mammal to be treated, *e.g.*, its weight, age, but may be determined routinely by one of ordinary skill in the art with regard to contemporary knowledge and to this disclosure.

Small molecules such as peptides can be poorly immunogenic and, thus, it is often necessary to prepare peptides conjugates to larger macromolecules (carriers) in order to elicit a substantive immune response to said molecules. Therefore, the polymer-modified polypeptides of the present invention may be further conjugated to an immunogenic carrier. For the coiled-coil structures described herein, individual polymer-modified, a-helical chimeric peptides (*e.g.*, CCIPN17) may first be conjugated to an immunogenic carrier and then subjected to conditions leading to the formation of covalently-stabilized homotrimeric or heterotrimeric coiled-coil structures comprised of three, individually-conjugated peptides. Alternatively, a covalently-stabilized, polymer-modified homotrimeric or heterotrimeric coiled-coil structure comprised of the chimeric peptides of the present invention can be conjugated to the immunogenic carrier. The carrier molecule, usually a heterologous protein, can help to evoke and/or elevate an immune response to the unmasked portions (*e.g.*, HIV portions) of the coiled-coil. The polymer-modified polypeptides can be linked to the immunogenic carrier by non-specific cross-linking agents, monogeneric spacers or bigeneric spacers. There are a number of immunogenic carrier molecules known in the art to which the polymer-modified polypeptides of the present invention can be conjugated (see, *e.g.*, Shodel et al., 1996, J. Biotechnol. 44:91-96; Lang and Korhonen, 1997, Behring Inst. Mitt. 98:400-409; Brennan et al 2001, Mol. Biotechnol. 17:15-26; Pumpens and Grens, 2201, Intervirology 44:98-114; and Simpson et al., 1999, Cell Mol. Life Sci. 56:47-61). Said potential immunogenic molecules include but are not limited to *Neisseria meningitidis* OMPC particles, keyhole limpet hemocyanin (KLH), bovine serum albumin (BSA), ovalbumin (OVA), thyroglobulin (TG), HBV-core antigen, HBV-surface antigen, immunogenic proteins such as tetanus or diphtheria toxoid or rotavirus VP6, and HIV capsid particles comprised of p24.

Thus, in one embodiment of the present invention, the polymer-modified, HIV-specific peptide mimetics described herein can be used as components of a preventative or protective (*e.g.*, either prophylactic or therapeutic) vaccine for HIV infection, resulting in the generation of HIV-specific neutralizing antibodies for active immunity against HIV. The vaccine could be formulated with adjuvants known in the art, such as MPL-A, and adsorbed onto either Alum or aluminum phosphate. The antigenic conjugates may also include T-cell helper epitopes to effectuate a stronger helper T cell response, including but not limited to a synthetic, non-natural pan HLA DR-binding epitope (PADRE) (see, *e.g.*, Alexander et al., 2000, J. Immunol., 164:1625-1633 and del Guercio et al., 1997, Vaccine 15:441-448). Immunogenic conjugates of the polymer-modified polypeptides described herein may be prepared by isolating, synthesizing and purifying their component parts (polymer-modified polypeptides and carrier) and then conjugating the two components. Subsequent purification of conjugate mixtures may be performed as desired. Antigenic conjugates of the polymer-modified polypeptides and a suitable immunogenic carrier have at least one covalent linkage between the component parts (*i.e.*, polymer-modified polypeptides and carrier) and, typically, have more than one polymer-modified polypeptide molecule covalently bound to each carrier molecule. If the components are prepared separately, they can be subsequently linked by non-specific cross-linking agents, monogeneric spacers or bigeneric spacers. Methods for non-specific cross-linking are well known in the art and include, but are not limited to, the following: reaction with glutaraldehyde; reaction with N-ethyl-N'-(3-dimethylaminopropyl) carbodiimide, with or without admixture of a succinylated carrier; periodate oxidation of glycosylated substituents followed by coupling to free amino groups of a protein carrier in the presence of sodium borohydride or sodium cyanoborohydride; diazotization of aromatic amino groups followed by coupling on tyrosine side chain residues of the protein; reaction with isocyanates; or reaction of mixed anhydrides. See, generally, Briand et al., 1985, J. Imm. Meth. 78:59-69.

In order to facilitate the conjugation of the chimeric peptides described herein to, for example, a resin or a carrier, a polymer-modified polypeptide of the present invention, *e.g.*, CCIPN17, can be further modified to include an additional cysteine residue. For CCIZN17/CCIPN17, this may include incorporating an extra cysteine amino acid residue at the NH₂-terminus of the peptide (*e.g.,* "CCCIZNI7" - CCCGGIKKEIEAIKKEQEAIKKKIEAIEKLLQLTVWGIKQLQARIL; SEQ ID NO: 76). The odd-numbered, terminal cysteine residue can be optionally separated from the remaining contiguous cysteine residues by means of a flexible, chemical linker, *e.g.*, Ttds (1-amino-4,7,10-trioxa-13-tridecamine succinic acid; -NH-(M₂)₃-O-(CH₂)₂-O-(CH₂)₂-O-(CH₂)₃-NH-CO-CH₂)₂-CO-; see U.S. Provisional Patent Applications 60/576,062 and 60/636,724, *supra.* The odd number of cysteine residues assures that after formation of a covalently-stabilized trimer comprising polymer-modified CC- and/or derivatized-chimeric N-peptides as described herein, at least one reactive thiol group per covalently-stabilized trimer is available for further reaction with thiol-reactive groups, such as maleimidyl. The linker provides flexibility, solubility and spacing between the disulfide bridges of the trimerization domain and the cysteine residues suitable for conjugation/derivatization. The additional cysteine residue may have a protected thiol group in order to prevent the participation of said cysteine residue in either disulfide or chemoselective bond formation (*e.g.*, thioether bond formation) and/or polymer attachment. The protecting group (including, but not limited to, a fluorenylmethoxy ("Fm") or an acetamidomethyl ("Acm") group) may be removed after the formation of the covalently-stabilized, trimeric coiled-coil so that said newly exposed thiol group is available for conjugation reactions.

The vaccines of the present invention may be formulated in any pharmaceutically effective formulation for host administration. Any such formulation may be, for example, a saline solution such as phosphate buffered saline (PBS). It will be useful to utilize pharmaceutically acceptable formulations which also provide long-term stability of the vaccine constructs of the present invention. A variety of storage conditions (*e.g.*, pH, buffer type, salt concentration, light exposure, high temperature, low ionic strength) may be controlled in the formulation to optimize the stability of the peptide vaccine. Said vaccines may be administered with or without the addition of adjuvant to the respective vaccine formulation. To this end, the peptide vaccines of the present invention may also be formulated with an adjuvant or adjuvants which may increase immunogenicity of the vaccine constructs of the present invention. A number of these adjuvants are known in the art. Two examples of adjuvants for use in the vaccines of the present invention are one or more forms of an aluminum phosphate-based adjuvant and the saponin adjuvant QS21 (a purified form of saponin and 3D-monophosphoryl lipid A (MPL), a nontoxic derivative of lipopolysaccharide (LPS)), each showing effective humoral and cellular responses to vaccine in a number of animal models. The artisan may alter the ratio of peptide vaccine to adjuvant to provide for an optimal immune response. Another adjuvant is a non-ionic block copolymer comprising blocks of polyoxyethylene (POE) and polyoxypropylene (POP) such as a POE-POP-POE block copolymer (Newman et al., 1998, Critical Reviews in Therapeutic Drug Carrier Systems 15(2):89-142). The basic structure comprises blocks of polyoxyethylene (POE) and polyoxypropylene (POP) such as a POE-POP-POE block copolymer. Newman et al. *id*., disclose that certain POE-POP-POE block copolymers may be useful as adjuvants to an influenza protein-based vaccine, namely higher molecular weight POE-POP-POE block copolymers containing a central POP block having a molecular weight of over about 9000 daltons to about 20,000 daltons and flanking POE blocks which comprise up to about 20% of the total molecular weight of the copolymer (see also U.S. Reissue Patent No. 36,665, U.S. Patent No. 5,567,859, U.S. Patent No. 5,691;387, U.S. Patent No. 5,696,298 and U.S. Patent No. 5,990,241, all issued to Emanuele, et al., regarding these POE-POP-POE block copolymers). WO 96/04932 further discloses higher molecular weight POE/POP block copolymers which have surfactant characteristics and show biological efficacy as vaccine adjuvants. The above cited references within this paragraph are hereby incorporated by reference in their entirety. It is therefore within the purview of the skilled artisan to utilize available adjuvants which may increase the immune response of the vaccine constructs of the present invention in comparison to administration of a non-adjuvanted vaccine.

The vaccine constructs of the present invention can be administered to the host by any means known in the art, such as enteral and parenteral routes. These routes of delivery include but are not limited to intramuscular injection, intraperitoneal injection, intravenous injection, inhalation or intranasal delivery, oral delivery, sublingual administration, subcutaneous administration, transdermal administration, transcutaneous administration, percutaneous administration or any form of particle bombardment or by any available needle-free injection device. The amount of polymer-modified polypeptide constructs to be introduced to a vaccine recipient will depend on the immunogenicity of the polypeptides. It is also contemplated that booster vaccinations are to be provided in a fashion which optimizes the overall immune response to vaccines of the present invention.

All publications mentioned herein are included for the purpose of describing and disclosing methodologies and materials that might be used in connection with the present invention. Nothing herein is to be construed as an admission that the invention is not entitled to antedate such disclosure by virtue of prior invention.

Having described preferred embodiments of the invention with reference to the accompanying figures, it is to be understood that the invention is not limited to those precise embodiments, and that various changes and modifications may be effected therein by one skilled in the art without departing from the scope or spirit of the invention as defined in the appended claims. Thus, the following examples are provided to illustrate the present invention without, however, limiting the same hereto:

### EXAMPLE 1

### Use of SMW PEG to Shield Undesired Epitopes: Design of a SMW PEG-Derivatized HIV Vaccine

This example provides one application of the present invention contributing to the field of HIV vaccines. Engineered peptides were previously designed for use as subunit vaccines that represent mimetics of the trimeric coiled-coil of the N-helix of HIV gp41 (see U.S. Provisional Patent Application No. 60/576,062 and 60/636,724; *supra*). These subunit vaccines are chimeric peptides comprising a soluble designed trimeric coiled-coil ("scaffold" region), a portion of the N-helix coiled-coil of HIV gp41 and a covalent stabilization moiety for the formation of interchain covalent bonds. An example of one such mimetic is schematically drawn in Figure 1A, representing the covalently-stabilized, trimeric coiled-coil designated "(CCIZN17)₃," The chemical, structural representation of the disulfide bonds covalently-stabilizing the three chimeric CCIZN17 peptides within (CC1ZN17)₃ [SEQ ID NO:2]₃ is shown in Figure IB. The three disulfide bonds are located between thiol (-SH) groups of NH₂-terminal cysteine residues. The contiguous amino acids located at the carboxy terminus of the cysteine residue are in bold with single letter nomenclature (Figure 1B). Both the scaffold region and the covalent interchain bonds are essential for the stable presentation of the HIV portion (N-peptide portion; *e.g.*, "N17" in (CCIZN17)₃) of a trimeric coiled-coil which mimics the pre-hairpin complex of gp41. Antibodies that target a conformation epitope generated by the N 17 region of gp41, when constrained within an N17-containing trimeric coiled-coil, *i.e*., the hydrophobic pocket, show antiviral activity against HIV-1 in a single-cycle infectivity assay (see U.S. Provisional Patent Application No. 60/576,012 filed June 1, 2004). In order to facilitate the use of peptide vaccines of this class to raise antibodies which specifically target the trimeric coiled-coil N-peptide portion of HIV gp4l, the present inventors have devised a strategy which generates a selectively focused immune response towards the HIV portion of the peptide mimetics while shielding the portion comprising the scaffold and the covalent interchain bonds from immune recognition.

*Synthesis of CCIZN17 (SEQ ID NO:2) -* The peptide CCIZN17 (SEQ ID NO:2) was synthesized by solid phase using Fmoc/t-Bu chemistry on a Pioneer Peptide Synthesizer (Applied Biosystems). The resin used was the Fmoc-Linker AM-Champion, 1% cross-linked (Biosearch Technologies, Inc.), a PEG-PS based resin derivatized with a modified Rink linker p-[(R,S)-α-[9H-Fluoren-9-yl-methoxyformamido]-2,4-dimethoxybenzyl]-phenoxyacetic acid (Rink, H., 1987, Tetrahedron Lett. 28:3787-3789; Bernatowicz, M. S. et al., 1989, Tetrahedron Lett. 30:4645-4667). All the acylation reactions were performed for 60-120 minutes with 4-fold excess of activated amino acid over the resin free amino groups. Amino acids were activated with equimolar amounts of HBTU (2-(1H-benzotriazole-1-yl)-1,1,3,3-tetramethyluronium hexafluorophosphate) and a 2-fold molar excess of DIEA (N,N-diisopropylethylamine). The side chain protecting groups were as follows: tert-butyl for glutamic acid (Glu) and threonine (Thr); trityl for cysteine (Cys) and glutamine (Gln); tert-butoxy-arbonyl for lysine (Lys) and tryptophan (Trp); and, 2,2,4,6,7-pentamethyldihydrobenzofuran-5-sulfonyl for arginine (Arg). The acetylation reaction was performed at the end of the peptide assembly by reaction with a 10-fold excess of acetic anhydride in N,N-dimethylformamide (DMF). At the end of the synthesis, the dry peptide-resin was treated with 88% trifluoroacetic acid (TFA), 5% phenol, 2% triisopropylsilane and 5% water (Sole, N. A. and G. Barany, 1992, *supra*) for 1.5 hours at room temperature. The resin was filtered and the solution was added to cold methyl-t-butyl ether in order to precipitate the peptide. After centrifugation, the peptide pellets were washed with fresh, cold methyl-t-butyl ether to remove the organic scavengers. The process was repeated twice. The final pellets were dried, resuspended in H₂O, 20% acetonitrile and lyophilized.

The crude peptide CCIZN17 (SEQ ID NO:2) was purified by gel permeation chromatography (GPC) on a 700 x 26 mm column packed with a Toyopearl HW-50S resin, using 30% acetonitrile in water, 0.1 % TFA as eluent. In a typical run, 300 mg of crude peptide was dissolved in 12 mL of eluent and directly loaded on the column, flow rate 1 mL/min. Analytical HPLC of eluted fractions was performed on a Beckman HPLC with a Phenomenex, Jupiter C₄ column (150 x 4.6 mm, 5 µm) with the following gradient of eluent B (above): 40%-60% B (in 20 min)-80% (in 3 min), flow rate 1 mL/min. On the basis of the purity, the fractions were pooled and further purified by reverse phase HPLC using semi-preparative Waters RCM Delta-Pak™ C₋₄ cartridges (40 x 200 mm, 15 µm) and using as eluents (A) 0.1% TFA in water and (B) 0.1% TFA in acetonitrile. The following gradient of eluent B was used: 35%-50% over 20 min, flow rate 80 mL/min. The purified peptide was characterized by electrospray mass spectrometry on a Micromass LCZ platform spectrometer. The theoretical, average molecular weight (MW)) is 5175 Da; while the found MW is 5175 Da.

*Oxidation of CCIZN17 to (CCIZN17)₃ -* The purified peptide precursor (12 mg), CCIZN17 (SEQ ID NO:2), was dissolved in 0.1 M Hepes, pH 7.3 (USB Corp.) at a concentration of 1 mg/mL. Under this condition, CCIZN17 is slowly oxidized by the air to the covalent trimer (CCIZN17)₃ ([SEQ ID NO:2]₃), see Figure 1B. The oxidation reaction was monitored by LC-MS analysis using a Waters-Micromass LCZ Platform with a Phenomenex, Jupiter C₄ column (150 x 4.6 mm, 5 µm) using as eluents (A) 0.1% TFA in water and (B) 0.1% TFA in acetonitrile. The following gradient of eluent B was used: 40%-60% B (in 20 min)-80% (in 3 min), flow rate 1 mL/min. Under these chromatographic conditions, CCIZN17 elutes at t_{R}= 13.25'. The oxidation reaction proceeds smoothly in 3 hours with formation of one main product eluting at t_{R}= 15' whose mass corresponds to that of one molecule comprising three CCIZN17 peptide chains having a reduction of mass consistent with formation of three disulfide bridges ([CCIZN17]₃). The overall yield of the oxidation reaction is more than 80%. To the solution (12 mL) was added 24 µL of TFA, and the solution was directly loaded on a 700 x 26 mm column, packed with a TSKgel Toyopearl HW-50S resin, using as eluent H₂O/acetonitrile, 70/30, 0.1 % TFA, flow rate 1 mL/min. The eluted fractions were analyzed by HPLC with a Phenomenex, Jupiter C₄ column (150 x 4.6 mm, 5 µm) using as eluents: (A) 0.1% TFA in water and (B) 0.1% TFA in acetonitrile. The following gradient of eluent B was used: 40%-60% B (in 20 min)-80% (in 3 min), flow rate 1 mL/min on the LC-MS Waters-Micromass LCZ Platform. The pooled fractions corresponding to the covalent trimer were further analyzed by mass spectrometry on an ESI-QqToF (Applied Biosystems) in a positive mode (ES⁺), nanospray 1 µl injection. The expected MW is 15520.2 Da; while the found MW is 15520.1 Da.

*Synthesis of CCIPN17 -* The peptide CCIPN17 (see Figure 2B) was synthesized following the same protocol as described for CCIZN17 (SEQ ID NO:2). In the sequence assembly on solid phase, two lysine residues were introduced in the IZ portion (underlined in the sequence in Figure 2B and below) with the ε-NH protection of allyloxycarbonyl (aloc) while all the other lysine residues were protected with Boc as for standard Fmoc/tBu chemistry:
CCGG*IKKEIEAIKKEQEAIKKKIEAIEK*LLQLTVWGIKQLQARIL (SEQ ID NO:2; scaffold domain is italicized). The pegylated CCIZN17 peptide is referred to as "CCIPN17," although the amino acid sequence of CCIPN17 is the same as that of CCIZN17. At the end of the peptide assembly, the NH₂-terminus was acetylated by reaction with a 10-fold excess of acetic anhydride in DMF. The protected-peptide-resin was further treated with tetrakis(triphenylphospine)palladium, Pd(PPh₃)₄, 0.25 equivalents and 24 equivalents of Phenylsilane for 30 minutes to remove the Aloc protecting groups from the two lysine residues while leaving all the other residue side chains protected. The two deprotected lysine chains were further derivatized with polyethylene glycol acid derivative of MW 236 Da (m-d PEG acid, Quanta BioDesign) by reaction with DIPC (diisopropylcarbodiimide) and HOBt (hydroxybenzotriazole) for 3 hours.

The crude peptide, CCIPN17, was purified by gel permeation chromatography (GPC) as described for purification of CCIZN17 (above). The pooled fractions (purity 70%) obtained by GPC were further purified to >95% purity by reverse-phase HPLC as described for CCIZN17 purification. The following gradient of eluent B was used: 40%-55% over 25 min, flow rate 80 mL/min. Analysis of the crude peptide, the eluted fractions and the purified pool was carried out by analytical HPLC on Jupiter C₄ column (150 x 4.6 mm, 5 µm, 300A, Phenomenex), as described above for CCIZN17. The purified peptide was characterized by mass spectrometry on a Micromass LCZ platform ES⁺. The found MW is 5612.0 Da, and the calculated average MW is 5611.8 Da.

*Oxidation of CCIPN17 to (CCIPN17)₃ -* The purified peptide precursor (26 mg), CCIPN17, was dissolved in buffer and slowly oxidized by the air to the covalent trimer (CCIPN17)₃ (as described for the oxidation of CCIZN17). The oxidation reaction was monitored by LC-MS analysis using a Waters-Micromass LCZ Platform as described previously. Under these chromatographic conditions, CCIPN17 elutes at t_{R}= 15.6'. The oxidation reaction proceeds smoothly overnight with formation of one main product eluting at t_{R}= 18.7' whose mass corresponds to that of one molecule comprising three CCIPN17 peptide chains having a reduction of mass consistent with formation of three disulfide bridges, (CCIPN17)₃. The overall yield of the oxidation reaction is more than 80%. To the solution was added 45 µL of TFA, and the solution was directly loaded on a 700 x 26 mm column, packed with a TSKgel Toyopearl HW-50S resin, using as eluent H₂O/acetonitrile 40/60, 0.1 % TFA, flow rate 1 mL/min. The eluted fractions were analyzed by HPLC with a Phenomenex, Jupiter C₄ column (150 x 4.6 mm, 5 µm) using as eluents: (A) 0.1% TFA in water and (B) 0.1% TFA in acetonitrile. The following gradient of eluent B was used: 35%-55% B (in 20 min)-80% (in 3 min), flow rate 1 mLmin on the LC-MS Waters-Micromass LCZ Platform. in a positive ion mode (ES⁺). The expected MW is 16829.57 Da; while the found MW is 16830 Da.

*Synthesis of Biotin-CCIZ (Biotin-SEQ ID NO:3) -* The peptide biotin-CCIZ (Biotin-GCCGGIKKEIEAIKKEQEAIKKKIEAIEKE; SEQ ID NO:3) was synthesized following the same protocol as described for CCEZN17 (SEQ ID NO:2). The reaction with Biotin was performed at the end of the peptide assembly process by reaction with a 4-fold excess of Biotin activated with an equimolar amount of DIPC (N,N'-diisopropylcarbodiimide) and HOAt (7-aza-1-hydroxybenzotriazole), overnight. The crude peptide, Biotin-CCIZ (SEQ ID NO:3), was purified by reverse-phase HPLC on semi-preparative Waters RCM Delta-Pak™ C₋₁₈ cartridges (40 x 200 mm, 15 µm) flow rate 80 mL/min, using as eluents (A) 0.1 % TFA in water and (B) 0.1 % TFA in acetonitrile. The following gradient of eluent B was used: 25%-35% over 30 min, flow rate 80 mL/min. Analysis of the crude peptide, the eluted fractions and the purified pool was carried out by analytical HPLC on Ultrasphere C₁₈ column (250 x 4.6 mm, 5 µm, 80A, Beckman), flow rate 1 mL/min, using as eluents: (A) 0.1% TFA in water and (B) 0.1% TFA in acetonitrile. The following gradient of eluent B was used: 25%-40% over 20 min-80% over 3 minutes. The expected MW is 3570.0 Da; while the found MW is 3570.5 Da.

*Oxidation of Biotin-CCIZ to (Biotin-CCIZ)₃ -* The purified peptide precursor (15 mg), Biotin-CCIZ (Biotin-SEQ ID NO:3), was dissolved in 0.1 M Hepes, pH 7.3 (USB Corp.) at a concentration of 5 mg/mL. Biotin-CCIZ is oxidized overnight by the air to the covalent trimer (Biotin-CCIZ)₃. The oxidation reaction was monitored by LC-MS analysis using a Waters-Micromass LCZ Platform as described previously. Under these chromatographic conditions, Biotin-CCIZ elutes at t_{R}= 16.4'. The oxidation reaction proceeds smoothly overnight with formation of one main product eluting at t_{R}= 16.9' whose mass corresponds to that of one molecule comprising three Biotin-CCIZ peptide chains having a reduction of mass consistent with formation of three disulfide bridges ([Biotin-CCIZ)₃). The overall yield of the oxidation reaction is more than 80%. The solution was dialyzed against water and then freeze-dried. The product was analyzed by HPLC with a Phenomenex, Jupiter C₄ column (150 x 4.6 mm, 5 µm) using as eluents: (A) 0.1% TFA in water and (B) 0.1% TFA in acetonitrile on the LC-MS Waters-Micromass LCZ Platform. in a positive mode (ES⁺). The expected MW is 10704.94 Da; while the found MW is 10704.0 Da.

*Synthesis of Biotin-CCIZN17 (Biotin-SEQ ID NO:82) -* Biotin-CCIZN17 (Biotin-GCCGGIKKEIEAIKKEQEAIKKKIEAIEKLLQLTVWGIKQLQARIL; SEQ ID NO:82) was synthesized following the same protocol as described for CCIZN17 (SEQ ID NO:2). The reaction with Biotin was performed at the end of the peptide assembly process as described above for Biotin-CCIZ. The crude peptide, Biotin-CCIZN17, was purified by gel permeation chromatography (GPC) as described above for purification of CCIZN17. The pooled fractions (purity 70%) obtained by GPC were further purified to >95% purity by reverse-phase HPLC as described previously. The following gradient of eluent B was used: 35%-55% over 25 min, flow rate 80 mL/min. Analysis of the crude peptide, the eluted fractions and the purified pool was carried out by analytical HPLC on Jupiter C₄ column (150 x 4.6 mm, 5 µm, 300A, Phenomenex), flow rate 1 mL/min, using as eluents: (A) 0.1% TFA in water and (B) 0.1% TFA in acetonitrile. The following gradient of eluent B was used: 40%-60% over 20 min-80% over 3 minutes. The purified peptide was characterized by mass spectrometry on a Micromass LCZ platform ES⁺. The found MW is 5416.0 Da, and the calculated average MW is 5416 Da.

*Oxidation of Biotin-CCIZN17 to (Biotin-CCIZIV17)₃. -* The purified peptide precursor (20 mg), Biotin-CCIZN17, was dissolved in 0.1 M Hepes, pH 7.3 (USB Corp.) at a concentration of 2 mg/mL. Under this condition, Biotin-CCIZN17 is slowly oxidized by the air to the covalent trimer (Biotin-CCIZN17)₃. The oxidation reaction was monitored by LC-MS analysis using a Waters-Micromass LCZ Platform as described above with the following gradient of eluent B: 40%-60% B (in 20 min)-80% (in 3 min), flow rate 1 mL/min. Under these chromatographic conditions, Biotin-CCIZN17 elutes at t_{R}= 12.87'. The oxidation reaction proceeds smoothly overnight with formation of one main product eluting at t_{R}= 14.9' whose mass corresponds to that of one molecule comprising three Biotin-CCIZN17 peptide chains having a reduction of mass consistent with formation of three disulfide bridges, (Biotin-CCIZN17)₃. The overall yield of the oxidation reaction is more than 80%. To the solution was added 45 µL of TFA, and the solution was directly loaded on a 700 x 26 mm column, packed with a TSKgel Toyopearl HW-50S resin, using as eluent H₂O/acetonitrile, 40/60, 0.1 % TFA, flow rate 1 mL/min. The pooled fractions were further purified by reverse-phase HPLC on semi-preparative Phenomenex C-₄ column (10 x 250 mm, 15 µm) flow rate 20 mL/min, using as eluents (A) 0.1% TFA in water and (B) 0.1% TFA in acetonitrile. The following gradient of eluent B was used: 35%-50% over 20 min, flow rate 20 mL/min. The eluted fractions were analyzed by HPLC with a Phenomenex, Jupiter C₄ column (150 x 4.6 mm, 5 µm) using as eluents: (A) 0.1% TFA in water and (B) 0.1% TFA in acetonitrile. The following gradient of eluent B was used: 40%-60% B (in 20 min)-80% (in 3 min), flow rate 1 mL/min on the LC-MS Waters-Micromass LCZ Platform in a positive mode (ES⁺). The expected MW is 16244 Da; while the found MW is 16245 Da.

*Synthesis of Biotin-CCIPN17 -* The peptide was synthesized following the same protocol as described for Biotin-CCIZN17 (SEQ ID NO:82). In the sequence assembly on solid phase, two lysine residues were introduced in the IZ portion (underlined in the sequence below) with the ε-NH protection of allyloxycarbonyl (aloc) while all the other lysine were protected with Boc as for standard Fmoc/tBu chemistry: Biotin-GCCGG*IKKEIEAIKKEQEAIKKKIEAIEK*LLQLTVWGIKQLQARIL (SEQ ID NO:82; scaffold domain is italicized). The pegylated Biotin-CC1Z1V17 peptide is referred to as "Biotin-CCIPN17," although the amino acid sequence of Biotin-CCIPN17 is the same as that of Biotin-CCIZN17. At the end of the peptide assembly, the reaction with Biotin was performed as described above for Biotin-CCIZ. The protected-peptide-resin was further treated with tetrakis(triphenylphospine)palladium, Pd(PPh₃)₄, 0.25 equivalents and 24 equivalents of Phenylsilane for 30 minutes to remove the Aloc protecting groups from the two lysine residues while leaving all the other residue side chains protected. The two deprotected lysine chains were further derivatized with polyethylene glycol acid derivative of MW 236 Da (m-d PEG acid, Quanta BioDesign) by reaction with DIPC (diisopropylcarbodiimide) and HOBt (hydroxybenzotriazole) for 3 hours.

The crude peptide, Biotin-CCIPN17, was purified by gel permeation chromatography (GPC) on TSK-gel Toyopearl HW-50S resin (700 x 26 mm column) as described previously. The pooled fractions (purity 70%) obtained by GPC were further purified to >95% purity by reverse-phase HPLC on semi-preparative Waters RCM Delta-Pak™ C₋₄ cartridges as described above, using the following gradient of eluent B: 35%-55% over 25 min, flow rate 80 mL/min. Analysis of the crude peptide, the eluted fractions and the purified pool was carried out by analytical HPLC on Jupiter C₄ column (150 x 4.6 mm, 5 µm, 300A, Phenomenex) as described previously. The purified peptide was characterized by mass spectrometry on a Micromass LCZ platform **ES⁺.** The found MW is 5796 Da, and the calculated average MW is 5796 Da.

*Oxidation of Biotin-CCIPN17 to (Biotin-CCIPN17)₃ -* The purified peptide precursor (20 mg), Biotin-CCIPN17, was dissolved in 0.1 M Hepes, pH 7.3 (USB Corp.) at a concentration of 2 mg/mL. Under this condition, Biotin-CCIPN17 is slowly oxidized overnight by the air to the covalent trimer (Biotin-CCIPN17)₃. The oxidation reaction was monitored by LC-MS analysis using a Waters-Micromass LCZ Platform with a Phenomenex, Jupiter C₄ column (150 x 4.6 mm, 5 µm) using as eluents (A) 0.1% TFA in water and (B) 0.1% TFA in acetonitrile. The following gradient of eluent B was used: 45%-60% B (in 20 min)-80% (in 3 min), flow rate 1 mL/min. Under these chromatographic conditions, Biotin-CCIPN17 elutes at t_{R}= 11.9'. The oxidation reaction proceeds smoothly overnight with formation of one main product eluting at t_{R}= 17.8' whose mass corresponds to that of one molecule comprising three Biotin-CCIPN17 peptide chains having a reduction of mass consistent with formation of three disulfide bridges ([Biotin-CCIPN17)₃). The overall yield of the oxidation reaction is more than 80%. To the solution was added guanidinium chloride to a final concentration of 6M, and the solution was directly loaded on a 700 x 26 mm column, packed with a TSKgel Toyopearl HW-50S resin, using as eluent H₂O/acetonitrile 40/60, 0.1% TFA, flow rate 1 mL/min. The eluted fractions were analyzed by HPLC with a Phenomenex, Jupiter C₄ column (150 x 4.6 mm, 5 µm) using as eluents: (A) 0.1% TFA in water and (B) 0.1% TFA in acetonitrile. The following gradient of eluent B was used: 45%-60% B (in 20 min)-80% (in 3 min), flow rate 1 mL/min on the LC-MS Waters-Micromass LCZ Platform. in a positive mode (ES⁺). The expected MW is 17383 Da; while the found MW is 17384 Da.

*Synthesis of Biotin-CCIP -* The peptide was synthesized following the same protocol as described for Biotin-CCIZ (SEQ ID NO:3). In the sequence assembly on solid phase, two lysine residues were introduced in the IZ portion (underlined in the sequence below) with the ε-NH protection of allyloxycarbonyl (aloc) while all the other lysine were protected with Boc as for standard Fmoc/tBu chemistry: Biotin-GCCGG*IKKEIEAIKKEQEAIKKKIEAIEK* (SEQ ID NO:3 scaffold domain is italicized). The pegylated Biotin-CCIZ peptide is referred to as "Biotin-CCIP," although the amino acid sequence of Biotin-CCIP is the same as that of Biotin-CCIZ. At the end of the peptide assembly, the reaction with Biotin was performed as described above for Biotin-CCIZ. The protected-peptide-resin was further treated with tetrakis(triphenylphospine)palladium, Pd(PPh₃)₄, 0.25 equivalents and 24 equivalents of Phenylsilane for 30 minutes to remove the Aloc protecting groups from the two lysine residues while leaving all the other residue side chains protected. The two deprotected lysine chains were further derivatized with polyethylene glycol acid derivative of MW 236 Da (m-d PEG acid, Quanta BioDesign) by reaction with DIPC (diisopropylcarbodiimide) and HOBt (hydroxybenzotriazole) for 3 hours.

The crude peptide, Biotin-CCIP, was purified by gel permeation chromatography (GPC) on TSK-gel Toyopearl HW-50S resin (700 x 26 mm column), using 30% acetonitrile in water as eluent, 0.1% TFA, flow rate 1 mL/min. The pooled fractions (purity 70%) obtained by GPC were further purified by reverse-phase HPLC on semi-preparative Waters RCM Delta-Pak™ C₋₄ cartridges (40 x 200 mm, 15 µm), using as eluents (A) 0.1% TFA in water and (B) 0.1% TFA in acetonitrile. The following gradient of eluent B was used: 20%-35% over 30 min, flow rate 80 mL/min. Analysis of the crude peptide, the eluted fractions and the purified pool was carried out by analytical HPLC on Jupiter C₄ column (150 x 4.6 mm, 5 µm, 300A, Phenomenex), flow rate 1 mL/min, using as eluents: (A) 0.1% TFA in water and (B) 0.1% TFA in acetonitrile. The following gradient of eluent B was used: 20%-40% over 20 min-80% over 3 minutes. The expected MW is 3821 Da; while the found MW is 3821 Da.

*Oxidation of Biotin-CCIP to (Biotin-CCIP)₃ -* The purified peptide precursor (20 mg), Biotin-CCIPN17, was dissolved in 0.1 M Hepes, pH 7.3 (USB Corp.) at a concentration of 2 mg/mL. Under this condition, Biotin-CCIP is slowly oxidized overnight by the air to the covalent trimer (Biotin-CCIP)₃ ([SEQ ID NO:6]₃). The oxidation reaction was monitored by LC-MS analysis using a Waters-Micromass LCZ Platform with a Phenomenex, Jupiter C₄ column (150 x 4.6 mm, 5 µm) using as eluents (A) 0.1% TFA in water and (B) 0.1% TFA in acetonitrile. The following gradient of eluent B was used: 25%-40% B (in 20 min)-80% (in 3 min), flow rate 1 mL/min. Under these chromatographic conditions, Biotin-CCIP elutes at t_{R}= 15.5'. The oxidation reaction proceeds smoothly overnight with formation of one main product eluting at t_{R}= 18.4' whose mass corresponds to that of one molecule comprising three Biotin-CCIP peptide chains having a reduction of mass consistent with formation of three disulfide bridges [(Biotin-CCIP)₃]. The overall yield of the oxidation reaction is more than 80%. To the solution was added trifluoroacetic acid to a final concentration of 0.2% and guanidinium chloride to a final concentration of 6M, and the solution was directly loaded on a 700 x 26 mm column, packed with a TSKgel Toyopearl HW-50S resin, using as eluent H₂O/acetonitrile, 40/60, 0.1% TFA, flow rate 1 mL/min. The eluted fractions were analyzed by HPLC with a Phenomenex, Jupiter C₄ column (150 x 4.6 mm, 5 µm) using as eluents: (A) 0.1 % TFA in water and (B) 0.1 % TFA in acetonitrile. The following gradient of eluent B was used: 25%-40% B (in 20 min)-80% (in 3 min), flow rate 1 mL/min on the LC-MS Waters-Micromass LCZ Platform. in a positive mode (ES⁺). The expected MW is 11456 Da; while the found MW is 11456 Da.

*Vaccine design -* Polyethylene glycol moieties of the nominal size of about 15.6 Å were strategically attached to the scaffold domain of CCIZN17 (SEQ ID NO:2) in such a manner that said PEG moieties would only minimally interfere, if at all, with trimeric coiled-coil formation. This small polyethylene glycol moiety, m-dPEG^{™} acid (MW=236) (Quanta BIODESIGN, Powell, Ohio) has a molecular weight of approximately 236 Da and the structure as follows: 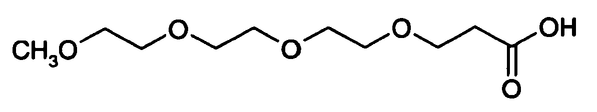 Initially, the epsilon amines of three lysine residues located at the more external "f" positions of the heptad repeat of the trimeric coiled-coil were identified as suitable positions for the attachment of the polyethylene glycol moiety on the IZ scaffold (see Figure 2A). Two of these lysine residues were ultimately selected for derivatization with m-dPEG^{™} acid (MW=236) (see Figure 2B with derivatized lysine ("K") residues underlined). As depicted in Figure 2B, the nomenclature of this polymer-modified CC-chimeric N-peptide changes from CCIZN17 to CCIPN17, capable of generating a covalently-stabilized (CCIPN17)₃ trimeric coiled-coil that comprises three CCIPN17 chimeric, polymer-modified peptides. A schematic model of this vaccine construct, (CCIPN17)₃, is shown in Figure 3. The helices that form the trimeric coiled-coil are represented as cylinders of approximately 62 Å in length. The dark gray portion of each cylinder represents the IZ scaffold domain, about 36 Å long, while the light gray portion represents the N17 region of HIV gp41, about 25 Å long. The m-dPEG^{™} acid (MW=236) moieties are represented as rectangles of about 15 Å in length. The two indicated lysine residues were selected for polymer modification in order to shield only the IZ scaffold. In fact, the lysine residue located more proximal to the N17 domain of the coiled-coil is about 22 Å from the N17 coiled-coil portion, ensuring that a m-dPEG^{™} acid moiety of 15 Å should not shield any part of this domain.

*Immunogenicity of the SMW-PEGylated vaccine -* Animal studies were conducted in guinea pigs to compare the antibody response elicited by the non-PEGylated vaccine (CCIZN17)₃ and the PEGylated vaccine (CCIPN17)₃. The latter was used both in a homologous modality (*i.e*., using a priming injection with the antigen, followed by two boost injections with the same antigen) and a heterologous modality (*i.e*., using a priming injection with the non-PEGylated antigen (CCIZN17)_{3,} followed by two boost injections with the PEGylated antigen (CCIPN17)₃) (see Figure 4). The PEGylated antigens were administered to the animals along with QS21 as an adjuvant (Antigenics; New York, NY). Analysis of the immune response was performed by ELISA. To eliminate any bias due to differences in the absorption capacity of the antigens on the ELISA plate, all the capture antigens were biotinylated, and the ELISA plate was coated with streptavidin prior to the addition of the capture biotinylated molecules. This way, the ELISA geometric mean titers (GMT) against each antigen are a true reflection of the relative amount of the antibodies with the corresponding specificity present in the serum. The biotinylated version of immunizing molecules, (Biotin-CCIZN17)₃ and (Biotin-CCIPN17)₃, and of the scaffold moieties alone, (Biotin-CCIZ)₃ and (Biotin-CCIP)₃, were used as capture antigens for the ELISA assay. In the serum of animals vaccinated with the non-PEGylated vaccine, (CCIZN17)₃, antibodies are present in comparable amounts against the whole antigen and the scaffold moiety alone. However, immunization with the PEGylated vaccine, both in the homologous and heterologous modality, generates a significantly reduced_response against the scaffold (neither (CCIZ)₃, nor as its PEGylated (CCIP)₃version), while very high titers are induced against the N-peptide moiety. This is identical in (CCIZN17)₃ and (CCIPN17)₃ and, accordingly, the measured GMT are identical when measured against either antigen.

This experiment proves that attachment of a SMW PEG moiety to selectively mask particular regions of a protein antigen is able to eliminate the immune reactivity to said regions without shielding the immunogenicity of the rest of the peptide/protein.

### EXAMPLE 2

### Immunotherapy of Alzheimer Disease

*Synthesis of PEG236-A*β*1-42 (SEQ ID NO:4; see* *Figure 5C**) -* The peptide was synthesized by solid phase on a ABI433 Synthesizer (Applied Biosystems) by using standard Fmoc/t-Bu chemistry. The resin used was a prederivatized Fmoc-Ala -ALH-Champion, 1% cross-linked (Biosearch Technologies, Inc.), a PEG-PS based resin derivatized that yields a carboxylated alanine. All the acylation reactions were performed for 60 minutes with 8-fold excess of activated amino acid over the resin free amino groups. Amino acids were activated with equimolar amounts of HATU (O-(7-azabenzotriazole-1-yl)-1,1,3,3-tetramethyluronium hexafluorophosphate) and a 2-fold molar excess of DIEA (N,N-diisopropylethylamine). The side chain protecting groups were as follows: tert-butyl for glutamic acid (E), aspartic acid (A), tyrosine (Y) and serine (S); trityl for histidine (H), asparagine (N) and glutamine (Q); tert-butoxy-carbonyl for lysine (K); and, 2,2,4,6,7-pentamethyldihydrobenzofuran-5-sulfonyl for arginine (R). For removal of the Fmoc protecting group, a solution of 2%DBU (diaza-bicyclo-undecene) in DMF was used for the (8-42) residue assembly and a solution of 20% piperidine in dimethylformamide (DMF) for the (1-8) residue assembly. At the end of the amino acid assembly the NH₂-terminus was further derivatized with polyethylene glycol acid derivative of MW 236 Da (m-d PEG acid, Quanta BioDesign) by reaction with DIPC (diisopropylcarbodiimide) and HOBt (hydroxybenzotriazole) for 3 hours.

The dry peptide-resin was treated with 88% trifluoroacetic acid (TFA), 5% phenol, 2% triisopropylsilane and 5% water (Sole, N. A. and G. Barany, 1992, *supra*) for 1.5 hours at room temperature. The resin was filtered and the solution was added to cold methyl-t-butyl ether in order to precipitate the peptide. After centrifugation, the peptide pellets were washed with fresh, cold methyl-t-butyl ether to remove the organic scavengers. The process was repeated twice. The final pellets were dried, resuspended in H₂O, 20% acetonitrile and lyophilized.

The crude peptide *PEG236-Aβ1-42* (DAEFRHDSGYEVHHQKLVFFAEDVGSNKGAIIGLMVGGVVIA; SEQ ID NO:4) was purified by reverse phase HPLC using semi-preparative Waters RCM Delta-Pak^{™} C₋₄ cartridges (40 x 200 mm, 15 µm) and using as eluents (A) water and (B) 0.1% NH4OH in acetonitrile. The following gradient of eluent B was used: 10%-30% over 20 min, flow rate 80 mL/min. Analytical HPLC of eluted fractions was performed on a Beckman HPLC with a Phenomenex, Jupiter C₄ column (150 x 4.6 mm, 5 µm) with the following gradient of eluent B (above): 15%-35% B (in 20 min)-80% (in 3 min), flow rate 1 mL/min. The purified peptide was characterized HPLC-MS on a Micromass LCZ platform spectrometer. The theoretical, average molecular weight (MW) is 4731.0 Da; while the found MW is 4731.0 Da.

*Results -* One specific application of the present invention is the development of an improved vaccine for the prophylactic and therapeutic treatment of Alzheimer disease (AD). Recent reports suggest that the severe brain inflammation problems which led to discontinuation of the clinical trial of the Elan/Wyeth AN-1792 Aβ 1-42 vaccine (see Figure 5B) are due to a T-cell response (Monsonego, A. and H.L. Weiner, 2003, Science 302:834-8; Nicoll, J.A. et al., 2003, Nat. Med. 9:448-52), specifically directed to the COOH-terminal region of the Aβ peptide (Monsogeo, A. et al., 2003, J. Clin. Invest. 112:415-22). Interestingly, however, the same clinical trial had shown a reduction of plaque and some limited stabilization of memory in the patients with high titers of Aβ antibodies, indicating that the vaccine was exerting a therapeutic effect. In particular, it was found that antibodies specific for the pathological form of Aβ were induced in the majority of patients vaccinated with Aβ 1-42 (Hock, C. et al., 2002, Nat. Med. 8:1270-5). These therapeutic antibodies recognize an epitope in the NH₂-terminal region of the peptide, *i.e.*, residues 4-10 (McLaurin, J. et al., 2002, Nat. Med. 8:1263-9; see Figure 5A). These finding were also confirmed in an animal model of the disease (Bard, F. et al., 2003, Proc. Natl. Acad. Sci. U.S.A. 100:2023-8). Accordingly, Elan/Wyeth are proceeding to clinical testing of a second-generation vaccine, ACC-001, based on a conjugated N-terminal (1-16) Aβ peptide (see Figure 5B).

The use of an Aβ peptide, free or in conjugated form, where the COOH-terminal region is not deleted, but shielded with a SMW polymer (e.g., SMW PEG), is a viable alternative to the Elan/Wyeth vaccine (see Figure 5C). SMW PEG, for example, can be attached in single or multiple copies within or in close proximity to the COOH-terminal T-cell epitope of Aβ thought to contribute to the severe side effects noted above, located from amino acids 16-30 (Monsonego, A. et al., 2003, *supra*), leaving intact the immunogenicity of the NH₂-terminal region, amino acids 1-16, or the even smaller region, amino acids 4-10, both believed to contain a favorable epitope (McLaurin, J.R. et al., 2002, *supra*)*.* The same molecule(s) could be used to develop antibodies for passive immunotherapy, either by classical hybridoma technology following immunization with the SMW PEGylated Aβ peptides, or by panning of phage display libraries on immobilized SMW PEGylated Aβ peptides.

Another specific application of this invention is the development of therapeutic antibodies that have the potential to distinguish between the different aggregation states of the Aβ protein, for example antibodies which are specific for the small oligomers which are thought to be responsible for the Aβ-induced pathology (Cleary, J.P. et al., 2005, Nat. Neurosci. 8:79-84). While the NH₂-temiinal region of Aβ is not involved in aggregation of Aβ to low molecular weight oligomers, high molecular weight oligomers, and fibrils, the same NH₂-temiinal region is immunodominant, and dictates the prevalent formation *in vivo* of antibodies which, being specific for an epitope common to the various forms, are unable to distinguish between them. Antibodies with the desired specificity can be obtained by using an Aβ peptide, free or in conjugated form, where the NH₂-terminal region is not deleted, but shielded with a SMW polymer (*e.g.*, SMW PEG). This peptide maintains the aggregation potential of normal Aβ, but the immune response against the N-terminal region is suppressed by PEG shielding.

### EXAMPLE 3

### Small MW PEGylated Vaccines Targeting the 4E10/Trp-rich Region of HIV-1

The structure of the Trp-rich region within HIV-1 gp41 which binds the neutralizing antibody 4E10 (Salzwedel, K. et al., 1999, J. Virol. 73:2469-80) has been determined within a lipid-mimetic environment by Schibli, D.J. et al. (2001, Biochemistry 40:9570-8). The amino acid residues that contact the 4E10 antibody have also recently been disclosed (Cardoso, R. et al., 2004, *supra*). These contact residues define the water-facing side of the peptide in the structure (see Figure 6A). In Figure 6A, the 4E10 binding residues are underlined and include Trp residues at position 672 and 680, while the membrane-facing Trp residues are double-underlined and located at positions 666, 670 and 678.

A desired a-helical conformation of the 4E10 epitope can be obtained by grafting the 4E10 epitope onto the surface of a GCN4 leucine zipper (O'Shea, E.K. et al., 1989, Science 245:646-8), in analogy to Sia, S.K. and P.S. Kim (2003, Proc. Natl. Acad. Sci. U.S.A. 100:9756-61). Based on the structure of the Trp-rich region as shown in Figure 6A, mutations can be introduced in some of the membrane-facing residues (underlined amino acids as part of the "4E10 coil" in Figure 6B), to produce a 4E10 peptide ("4E10 coil") with a dimerization surface compatible with a GCN4 peptide ("GCN4 coil") (see Figure 6B). This coiled-coil will constrain the 4E10 region in the appropriate helical structure. The stability of the GCN4/4E10 coiled-coil can be further enhanced by the addition of a disulfide bond (Sia, S.K. and P.S. Kim, 2003, *supra*) and by mutation of the residues in the "a" and "d" positions of both GCN4 and 4E10 to leucine or isoleucine (Lu, S.M. and R.S. Hodges, 2002, J. Biol. Chem. 277:23515-24). To avoid an immune response directed against the scaffolding part of the structure (*i.e*., the GCN4 coil), a suitable lysine residue (*e.g.*, double-underlined residue in Figure 7) along the surface of the GCN4 coil can be derivatized with PEG 236 (Figure 7).

### EXAMPLE 4

### PEGylated Chimeric N17 Peptides with Acidic Scaffold for Conjugation to OMPC

*Synthesis of Br-acetyl-GGGEP17GluN17 (peptide no. 1 in Table 5) -* The peptide was synthesized by solid phase on a Pioneer Peptide Synthesizer (Applied Biosystems). The resin used was the Fmoc-Linker AM-Champion, 1% cross-linked (Biosearch Technologies, Inc.), a PEG-PS based resin derivatized with a modified Rink linker p-[(R,S)-α-[9H-Fluoren-9-yl-methoxyformamido]-2,4-dimethoxybenzyl]-phenoxyacetic acid (Rink, H., 1987, Tetrahedron Lett. 28:3787-3789; Bernatowicz, M. S. et al., 1989, Tetrahedron Lett. 30:4645-4667). All the acylation reactions were performed for 90 minutes with 4-fold excess of activated amino acid over the resin free amino groups. Amino acids were activated with equimolar amounts of HBTU (2-(1H-benzotriazole-1-yl)-1,1,3,3-tetramethyluronium hexafluorophosphate) and a 2-fold molar excess of DIEA (N,N-diisopropylethylamine). For the first 31 residues portion of the peptide sequence: *KIEEIEEKIEEIEK*LLQLTVWGIKQLQARIL (SEQ ID NO:93; non-HIV residues are italicized), standard Fmoc/t-Bu chemistry was used. The side chain protecting groups were as follows: tert-butyl for glutamic acid (Glu) and threonine (Thr); trityl for cysteine (Cys) and glutamine (Gln); tert-butoxy-carbonyl for lysine (Lys) and tryptophan (Trp); and, 2,2,4,6,7-pentamethyldihydrobenzofuran-5-sulfonyl for arginine (Arg). After assembly of the first 31 residues, Dde-Lys(Fmoc)-OH, N-α-1-(4,4-Dimethyl-2,6-dioxocyclohex-1-ylidene)ethyl-N-ε-Fmoc-L-lysine, was acylated as the 32^{nd} residue. The Fmoc- protecting group on the ε-amino group was selectively removed by treatment with a solution of 20% piperidine in dimethylformamide (DMF). Under this condition, the Dde- protecting group is completely stable. The deprotected lysine side chain was further derivatized with polyethylene glycol acid derivative of MW 236 Da (m-d PEG acid, Quanta BioDesign) by reaction with DIPC (diisopropylcarbodiimide) and HOBt (hydroxybenzotriazole) for 3 hours. The peptide resin was then treated with a solution of 1% hydrazine in DMF for removing the Dde-group from the α-amino group of the lysine. The assembly was continued to obtain the peptide resin *GGIEKKIEEIEEKIEEIEK*LLQLTVWGIKQLQARIL (SEQ ID NO:94; non-HIV residues are italicized; pegylated lysine residue is underlined). Then the peptide resin was separated into two portions. The assembly was continued using the larger portion, 2/3 of the total, to incorporate another glycine residue which was further bromoacetylated by reaction with a 10-fold excess of bromoacetic anhydride in DMF. The remaining 1/3 of the *GGIEKKIEEIEEKIEEIEK*LLQLTVWGIKQLQARIL (SEQ ID NO:94)-resin, was used to continue with the synthesis of Ac-C(Acm)-TtdsCCEP17GluN17 (see below).

At the end of the synthesis of Br-acetyl-EP17GluN17, the dry peptide-resin was treated with 88% trifluoroacetic acid (TFA), 5% phenol, 2% triisopropylsilane and 5% water (Sole, N. A. and G. Barany, 1992, *supra*) for 1.5 hours at room temperature. The resin was filtered and the solution was added to cold methyl-t-butyl ether in order to precipitate the peptide. After centrifugation, the peptide pellets were washed with fresh, cold methyl-t-butyl ether to remove the organic scavengers. The process was repeated twice. The final pellets were dried, resuspended in H₂O, 20% acetonitrile and lyophilized.

The crude peptide *Br-acetyl-GGGEP17GluN17* (peptide no. 1 in Table 5) was purified by gel permeation chromatography (GPC) on a 700 x 26 mm column packed with a Toyopearl HW-50S resin, using 60% acetonitrile in water, 0.1 % TFA as eluent. In a typical run, 900 mg of crude peptide was dissolved in 25 mL of eluent and directly loaded on the column, flow rate 1 mL/min. Analytical HPLC of eluted fractions was performed on a Beckman HPLC with a Phenomenex, Jupiter C₄ column (150 x 4.6 mm, 5 µm) with the following gradient of eluent B (above): 45%-65% B (in 20 min)-80% (in 3 min), flow rate 1 mL/min. On the basis of the purity, the fractions were pooled and further purified by reverse phase HPLC using semi-preparative Waters RCM Delta-Pak^{™} C₋₄ cartridges (40 x 200 mm, 15 µm) and using as eluents (A) 0.1% TFA in water and (B) 0.1% TFA in acetonitrile. The following gradient of eluent B was used: 45%-60% over 20 min, flow rate 80 mL/min. The purified peptide was characterized by electrospray mass spectrometry on a Micromass LCZ platform spectrometer. The theoretical, average molecular weight (MW) is 4613.3 Da; while the found MW is 4613.0 Da.

*Synthesis of Ac-C(Acm)-Ttds-CCEP17GluN17 (peptide no. 2 in Table 5) -* From the previous assembly, a third of the *GGIEKKIEEIEEKIEEIEK*LLQLTVWGIKQLQARIL (SEQ ID NO:94-resin (generated as described above) was used to continue the synthesis of the Ac-C(Acm)Ttds-CC-portion. The first (sequence carboxy terminal) two cysteines, with the tritryl protection on the side chain, were assembled by standard Fmoc/tBu chemistry as previously described for Br-acetyl-GGGEP17GluN17 (peptide no. 1 in Table 5). The reaction with Ttds (1-amino-4,7,10-trioxa-13-tridecamine succinic acid (-NH-(CH₂)₃-O-(CH₂)₂-O-(CH₂)₂-O-(CH₂)₃-NH-CO-CH₂)₂-CO-) was performed by reaction with a 4-fold excess of Ttds activated with an equimolar amount of HBTU and a 2-fold molar excess of DIEA for two hours. The N-terminal residue, C(Acm), was acylated under the same conditions as with Fmoc-Cys(Acm)-OH (above) and then acetylated after Fmoc deprotection by reaction with a 10-fold excess of acetic anhydride in DMF. After cleavage from resin as described for previous example, the crude peptide, Ac-C(Acm)-TtdsCCEP17G1uN17 (peptide no. 2 in Table 5), was purified by gel permeation chromatography (GPC) on TSK-gel Toyopearl HW-50S resin (700 x 26 mm column), using 60% acetonitrile in water as eluent, 0.1% TFA, flow rate 1 mL/min. The pooled fractions (purity 70%) obtained by GPC were further purified to >95% purity by reverse-phase HPLC on semi-preparative Waters RCM Delta-Pak^{™} C₋₄ cartridges (40 x 200 mm, 15 µm) using as eluents (A) 0.1% TFA in water and (B) 0.1% TFA in acetonitrile. The following gradient of eluent B was used: 45%-60% over 20 min, flow rate 80 mL/min. Analysis of the crude peptide, the eluted fractions and the purified pool was carried out by analytical HPLC on Jupiter C₄ column (150 x 4.6 mm, 5 µm, 300A, Phenomenex), flow rate 1 mL/min, using as eluents: (A) 0.1% TFA in water and (B) 0.1% TFA in acetonitrile. The following gradient of eluent B was used: 45%-65% over 20 min-80% over 3 minutes. The purified peptide was characterized by electrospray mass spectrometry on a Micromass LCZ platform. The theoretical average MW is 5161.2 Da, and the found MW was 5161.0 Da.

*Synthesis of Ac-C(Acm)-(thioEP17GluN17)₃ - covalent trimerization by formation of thioether bonds -* The purified peptide precursor (10.1 mg), Br-acetyl-GGGEP17GluN17 (peptide no. 1 in Table 5), was dissolved in 6 M guanidinium chloride, 0.25M TrisHCl, pH 8.5, 2mM EDTA at a concentration of 5 mg/mL. To this solution was added the other peptide precursor, Ac-C(Acm)Ttds-CCEP17GluN17 (peptide no. 2 in Table 5) (5.3 mg; 1 mg/mL). The actual molar ratio of the two peptide precursors was 2.2:1, Br-acetyl-GGGEP17GluN17:Ac-C(Acm)Ttds-CCEP17GluN17. The trimerization reaction was monitored by LC-MS analysis using a Waters-Micromass LCZ Platform with a Phenomenex, Jupiter C₄ column (150 x 4.6 mm, 5 µm) using as eluents (A) 0.1% TFA in water and (B) 0.1% TFA in acetonitrile. The following gradient of eluent B was used: 45%-65% B (in 20 min)-80% (in 3 min), flow rate 1 mL/min. The thioether formation reaction proceeded smoothly. After two hours, one product was formed, whose mass corresponds to one molecule comprising one Ac-C(Acm)Ttds-CCEP17GluN17 peptide chain ligated via two thioether bonds to two Br-acetyl-GGGEP17GluN17 peptide chains. To the solution was added 20 µL of TFA, and the solution was directly purified by gel permeation chromatography (GPC) on TSK-gel Toyopearl HW-50S resin (700 x 26 mm column), using 60% acetonitrile in water as eluent, 0.1% TFA, flow rate 1 mL/min. The eluted fractions were analyzed by HPLC with a Phenomenex, Jupiter C₄ column (150 x 4.6 mm, 5 µm) using as eluents: (A) 0.1% TFA in water and (B) 0.1% TFA in acetonitrile. The following gradient of eluent B was used: 45%-65% B (in 20 min)-80% (in 3 min), flow rate 1 mL/min on the LC-MS Waters-Micromass LCZ Platform in a positive mode (ES⁺). The expected MW is 14225 Da; while the found MW was 14226 Da.

*Deprotection of the acetylated-cysteine of Ac-C(Acm)(thioEP17GluN17)₃ to form Ac-C(thioEP17GluN17)₃ -* The purified coiled-coil precursor ac-C(Acm)(thioEPl7GluN17)₃ was dissolved at a concentration of 20 mg/ml with TFA, anisole 2% and 50 eq. AgOTf (silver trifluoromethanesulfonate) at 4°C for 3 hours. Ice-cold, dry ether was added to the reaction mixture and the precipitate was isolated by centrifugation. The precipitate was washed twice with ice-cold, dry ether. The peptide was redissolved in H₂O/acetonitrile, 0.1%TFA and purified by gel permeation chromatography (GPC) on TSK-gel Toyopearl HW-50S resin (700 x 26 mm column), using 60% acetonitrile in water as eluent, 0.1% TFA, flow rate 1 mL/min. The purified peptide was characterized by electrospray mass spectrometry on a Micromass LCZ platform. The theoretical average MW was 14157 Da, and the found MW was 14157 Da.

*Synthesis of Br-acetyl-GGGEPN17 (peptide no. 3 in Table 5) -* The peptide was synthesized by solid phase on a Pioneer Peptide Synthesizer (Applied Biosystems). The resin used was the Fmoc-Linker AM-Champion, 1% cross-linked (Biosearch Technologies, Inc.), a PEG-PS based resin derivatized with a modified Rink linker p-[(R,S)-α-[9H-Fluoren-9-yl-methoxyformamido]-2,4-dimethoxybenzyl]-phenoxyacetic acid (Rink, H., 1987, Tetrahedron Lett. 28:3787-3789; Bernatowicz, M. S. et al., 1989, Tetrahedron Lett. 30:4645-4667). All the acylation reactions were performed for 90 minutes with 4-fold excess of activated amino acid over the resin free amino groups. Amino acids were activated with equimolar amounts of HBTU (2-(1H-benzotriazole-1-yl)-1,1,3,3-tetramethyluronium hexafluorophosphate) and a 2-fold molar excess of DIEA (N,N-diisopropylethylamine). For the first 31 residues portion of the peptide sequence, *KIEEIEKKIEEIEK*LLQLTVWGIKQLQARIL (SEQ ID NO:95; non-HIV residues are italicized), a standard Fmoc/t-Bu chemistry was followed. The side chain protecting groups were as follows: tert-butyl for glutamic acid (Glu) and threonine (Thr); trityl for cysteine (Cys) and glutamine (Gln); tert-butoxy-carbonyl for lysine (Lys) and tryptophan (Trp); and, 2,2,4,6,7-pentamethyldihydrobenzofuran-5-sulfonyl for arginine (Arg). After assembly of the first 31 residues, Dde-Lys(Fmoc)-OH, N-α-1-(4,4-Dimethyl-2,6-dioxocyclohex-1-ylidene)ethyl-N-ε-Fmoc-L-lysine, was acylated as the 32^{nd} residue. The Fmoc- protecting group on the ε-amino group was selectively removed by treatment with a solution of 20% piperidine in dimethylformamide (DMF). Under this condition, the Dde- protecting group is completely stable. The deprotected lysine side chain was further derivatized with polyethylene glycol acid derivative of MW 236 Da (m-d PEG acid, Quanta BioDesign) by reaction with DIPC (diisopropylcarbodiimide) and HOBt (hydroxybenzotriazole) for 3 hours. The peptide resin was then treated with a solution of 1% hydrazine in DMF for removing the Dde-group from the α-amino group of the lysine. The assembly was continued to incorporate six other residues of the scaffold, non-HIV domain, namely the sequence KIEEIE (SEQ ID NO:96), on the peptide resin. Then Dde-Lys(Fmoc)-OH, N-α-1-(4,4-Dimethyl-2,6-dioxocyclohex-1-ylidene)ethyl-N-ε-Fmoc-L-lysine, was acylated. The Fmoc- protecting group on the ε-amino group was selectively removed by treatment with a solution of 20% piperidine in dimethylformamide (DMF). Under this condition, the Dde-protecting group is completely stable. The deprotected lysine side chain was further derivatized with polyethylene glycol acid derivative of MW 236 Da (m-d PEG acid, Quanta BioDesign) by reaction with DIPC (diisopropylcarbodiimide) and HOBt (hydroxybenzotriazole) for 3 hours. The assembly was continued to obtain the peptide resin *GGIEKKIEEIEKKIEEIEKKIEEIEK*LLQLTVWGIKQLQARIL (SEQ ID NO:97; non-HIV residues are italicized; pegylated lysine residues are underlined). Then the peptide resin was separated into two portions. The assembly was continued on the larger portion, 2/3 of the total, to incorporate another glycine residue which was further bromoacetylated by reaction with a 10-fold excess of bromoacetic anhydride in DMF. Using the remaining 1/3 of the peptide-resin, the assembly was continued for the synthesis of Ac-C(Acm)-Ttds CCEPN17 (see below).

At the end of the synthesis of Br-acetyl-EPN17, the dry peptide-resin was treated with 88% trifluoroacetic acid (TFA), 5% phenol, 2% triisopropylsilane and 5% water (Sole, N. A. and G. Barany, 1992, *supra)* for 1.5 hours at room temperature. The resin was filtered and the solution was added to cold methyl-t-butyl ether in order to precipitate the peptide. After centrifugation, the peptide pellets were washed with fresh, cold methyl-t-butyl ether to remove the organic scavengers. The process was repeated twice. The final pellets were dried, resuspended in H₂O, 20% acetonitrile and lyophilized.

The crude peptide *Br-acetyl-GGGEPN17* (peptide no. 3 in Table 5) was purified by gel permeation chromatography (GPC) on a 700 x 26 mm column packed with a Toyopearl HW-50S resin, using 60% acetonitrile in water, 0.1 % TFA as eluent. In a typical run, 900 mg of crude peptide was dissolved in 25 mL of eluent and directly loaded on the column, flow rate 1 mL/min. Analytical HPLC of eluted fractions was performed on a Beckman HPLC with a Phenomenex, Jupiter C₄ column (150 x 4.6 mm, 5 µm) with the following gradient of eluent B (above): 45%-65% B (in 20 min)-80% (in 3 min), flow rate 1 mL/min. On the basis of the purity, the fractions were pooled and further purified by reverse phase HPLC using semi-preparative Waters RCM Delta-Pak^{™} C₋₄ cartridges (40 x 200 mm, 15 µm) and using as eluents (A) 0.1% TFA in water and (B) 0.1 % TFA in acetonitrile. The following gradient of eluent B was used: 45%-60% over 20 min, flow rate 80 mL/min. The purified peptide was characterized by electrospray mass spectrometry on a Micromass LCZ platform spectrometer. The theoretical, average molecular weight (MW)) is 5699 Da; while the found MW is 5700 Da.

*Synthesis of Ac-C(Acm)-TtdsCCEPN17 (peptide no. 4 in Table 5*) - From the previous assembly, a third of the *GGIEKKIEEIEKKIEEIEKKIEEIEK*LLQLTVWGIKQLQARIL (SEQ ID NO:97)-resin (see above for details) was used to continue the synthesis of the Ac-C(Acm)Ttds-CC-portion. The first (sequence carboxy terminal) two cysteines, with the trityl protection on the side chain, were assembled by standard Fmoc/tBu chemistry. The reaction with Ttds (1-amino-4,7,10-trioxa-13-tridecamine succinic acid (-NH-(CH₂)₃-O-(CH₂)₂-O-(CH₂)₂-O-(CH₂)₃-NH-CO-CH₂)₂-CO-) was performed by reaction with a 4-fold excess of Ttds activated with an equimolar amount of HBTU and a 2-fold molar excess of DIEA for two hours. The N-terminal residue, C(Acm), was first acylated under the same condition as used for Fmoc-Cys(Acm)-OH and then acetylated after Fmoc deprotection by reaction with a 10-fold excess of acetic anhydride in DMF.

After cleavage from resin as described for previous example, the crude peptide, acetyl-C(Acm)-Ttds-CCEPN17, was purified by gel permeation chromatography (GPC) on TSK-gel Toyopearl HW-50S resin (700 x 26 mm column), using 60% acetonitrile in water as eluent, 0.1% TFA, flow rate 1 mL/min. The pooled fractions (purity 70%) obtained by GPC were further purified to >95% purity by reverse-phase HPLC on semi-preparative Waters RCM Delta-Pak^{™} C₋₄ cartridges (40 x 200 mm, 15 µm) using as eluents (A) 0.1% TFA in water and (B) 0.1% TFA in acetonitrile. The following gradient of eluent B was used: 45%-60% over 20 min, flow rate 80 mL/min. Analysis of the crude peptide, the eluted fractions and the purified pool was carried out by analytical HPLC on Jupiter C₄ column (150 x 4.6 mm, 5 µm, 300A, Phenomenex), flow rate 1 mL/min, using as eluents: (A) 0.1% TFA in water and (B) 0.1% TFA in acetonitrile. The following gradient of eluent B was used: 45%-65% over 20 min-80% over 3 minutes. The purified peptide was characterized by electrospray mass spectrometry on a Micromass LCZ platform. The theoretical average MW is 6248 Da, and the found MW was 6247 Da.

*Synthesis of Acetyl-C(Acm)(thioEPN17)₃ - covalent trimerization by formation of thioether bonds -* The purified peptide precursor (12 mg), Br-acetyl-GGGEPN17 (peptide no. 3 in Table 5), was dissolved in 6 M guanidinium chloride, 0.25M TrisHCl, pH 8.5, 2mM EDTA at a concentration of 5 mg/mL. To this solution was added the other peptide precursor, Acetyl-C(Acm)Ttds-CCEPN17 (peptide no.4 in Table 5) (6 mg; 1 mg/mL). The actual molar ratio of the two peptide precursors was 2.2:1, Br-acetyl-GGGEPN17: Ac-C(Acm)Ttds-CCEPN17. The trimerization reaction was monitored by LC-MS analysis using a Waters-Micromass LCZ Platform with a Phenomenex, Jupiter C₄ column (150 x 4.6 mm, 5 µm) using as eluents (A) 0.1% TFA in water and (B) 0.1% TFA in acetonitrile. The following gradient of eluent B was used: 35%-70% B (in 20 min)-80% (in 3 min), flow rate 1 mL/min. The thioether formation reaction proceeded smoothly. After two hours, one product was formed, whose mass corresponds to one molecule comprising one Ac-C(Acm)Ttds-CCEPN17 peptide chain ligated via two thioether bonds to two Br-acetyl-GGGEPN17 peptide chains. To the solution was added 20 µL of TFA, and the solution was directly purified by gel permeation chromatography (GPC) on TSK-gel Toyopearl HW-50S resin (700 x 26 mm column), using 60% acetonitrile in water as eluent, 0.1% TFA, flow rate 1 mL/min. The eluted fractions were analyzed by HPLC with a Phenomenex, Jupiter C₄ column (150 x 4.6 mm, 5 µm) using as eluents: (A) 0.1% TFA in water and (B) 0.1% TFA in acetonitrile. The following gradient of eluent B was used: 45%-65% B (in 20 min)-80% (in 3 min), flow rate 1 mL/min on the LC-MS Waters-Micromass LCZ Platform in a positive mode (ES⁺). The expected MW is 17489 Da; while the found MW was 17489 Da.

*Deprotection of the acetylated-cysteine of ac-C(Acm)(thioEPN17)₃ to form ac-C(thioEPN17)₃ -* The purified coiled-coil precursor ac-C(Acm)(thioEPN17)₃ was dissolved at a concentration of 20 mg/ml with TFA, anisole 2% and 50 eq. AgOTf (silver trifluoromethanesulfonate) at 4°C for 3 hours. Ice-cold, dry ether was added to the reaction mixture and the precipitate was isolated by centrifugation. The precipitate was washed twice with ice-cold, dry ether. The peptide was redissolved in H₂O/acetonitrile, 0.1%TFA and purified by gel permeation chromatography (GPC) on TSK-gel Toyopearl HW-50S resin (700 x 26 mm column), using 60% acetonitrile in water as eluent, 0.1% TFA, flow rate 1 mL/min. The purified peptide was characterized by electrospray mass spectrometry on a Micromass LCZ platform. The theoretical average MW was 17415 Da, and the found MW was 17416 Da.

*Conjugation of ac-C(thioEP17GluN17)₃* and *ac-C(thioEPN17)₃ to OMPC -* Various methods of purifying OMPC from the gram-negative bacteria have been devised (Frasch et al., 1997, J Exp. Med 140:87; Frasch et al., 1978, J Exp. Med. 147:629; U.S. Pat. No. 4,707,543; Helting et al., 1981. Acta Path. Microbiol. Scand. Sect. C. 89:69; and, U.S. Pat. No. 4,271,147). *N. meningitidis* B improved Outer Membrane Protein Complex (iOMPC) can be obtained using techniques well known in the art such as those described by Fu, U.S. Patent No. 5,494,808.

To 1.36 mL of *Neisseria meningitidis* improved Outer Membrane Protein Complex (iOMPC) solution (7.13 mg/ml), 0.5 M NaHCO₃ (0.151 mL) was added to a final concentration of 50mM, pH 8.5. To this was added, drop-wise, 0.407 mL of a 20 µM solution of the heterobifunctional crosslinker sulfosuccinimidyl 4-(N-maleimidomethyl)cyclohexane-1-carboxylate (sSMCC, Pierce Chemical Co.) with a 2-fold excess (with respect to lysine residues of OMPC, 0.42 µmol lysine/mg OMPC protein). The pH was readjusted by further addition of 0.045 mL 0.5 M NaHCO₃. After aging the solution for 1 hour in the dark at 4°C, the pH was lowered to neutrality by adding a 1 M NaH₂PO₄ solution (16 µl). The solution was dialyzed at 4°C using 300K MWCO DispoDialyser (Spectrum Laboratories Inc., CA) with 6-buffer changes (every 2 h) of 2 L 20 mM HEPES pH 7.3 (4-(2-Hydroxyethyl)piperazine-1-ethanesulfonic acid), 2mM EDTA (Ethylenediaminetetracetic acid) to remove excess reagents. A total of 1.9 mL of activated OMPC (aOMPC) was recovered after dialysis.

A 0.5 mg/ml stock solutions of the two Cys-containing peptides, ac-C(thioEP17GluN17)₃ and ac-C(thioEPN17)3_{,} were prepared in degassed solution of 0.1 M HEPES, 2 mM EDTA pH 7.3. To define the maximum amount of peptide ligand that could be safely incorporated on aOMPC without causing precipitation, the conjugation reaction was first followed in small-scale trials where the aOMPC was incubated with increasing amounts of peptide ligand. The maximum number of maleimide groups that can be incorporated on the OMPC is limited by the total lysine residues on the OMPC, namely 0.42 µmoles lysine/mg OMPC. If one considers an average MW of 40 x 10⁶ Da for OMPC, this corresponds to 16,000 lysine moles/OMPCmol. Of these, only a portion can be activated with sSMCC (up to 35%), which corresponds to a maximum peptide load attainable of about 5000 moles. In this case, aOMPC was incubated with the following molar excesses of peptide ligand per OMPC mol: 1000, 2000, 3000. After one hour, the samples were compared with an aOMPC sample to check for the presence of any precipitation or enhancement of turbidity. In the case of ac-C(thioEP17GluN17)₃, no precipitation or increase of turbidity was visible up to the highest molar excess of ligand used, 3000 moles/OMPC mol; while in the case of ac-C(thioEPN17)₃, a slight precipitation was observed at 3000 moles/OMPC mol and was perfectly soluble at 2500 moles/OMPC mol.

On the basis of these observations, a large-scale reaction was performed: to 0.75 mL (3.8 mg) of aOMPC in 20 mM HEPES, 2 mM EDTA pH 7.3, was added 7 mL of the ac-C(thioEP17GluN17)₃ peptide stock solution, drop-wise, while gently vortexing, which corresponds to 3000 molar excess of peptide moles/OMPC mol. For ac-C(thioEPN17)_{3,} 0.45 mL (2.3 mg) of aOMPC in 20 mM HEPES, 2 mM EDTA pH 7.3, was added 9 mL of the ac-C(thioEPN17)₃ peptide stock solution, drop-wise while gently vortexing, which corresponds to 2500 molar excess of peptide moles/OMPC mol. A sample of maleimide-activated OMPC solution was retained as blank for the determination of the peptide loading of the final conjugate. The conjugation reaction mixture was allowed to age for 17 h at 4°C in the dark. Any residual maleimide groups on the OMPC were then quenched with µ-mercaptoethanol to a final concentration of 15 mM (10-12 µL total volume added) for 1 h at 4°C in the dark. The solution was dialyzed 4X, 4 hour/change, with 1 L of 20 mM HEPES pH 7.3 at 4°C with 300K MWCO DispoDialyser to remove unconjugated peptide and β-mercaptoethanol. The concentration was determined by Lowry assay (Lowry et al., 1951, J. Biol. Chem. 193:265), revealing 0.41 mg/mL for the OMPC-(thioEP17GluN17)₃ and 0.32 mg/mL for the OMPC-(thioEPN17)₃. The conjugate and aOMPC samples were hydrolyzed in evacuated, sealed glass tubes with azeotropic HCl for 70 h at 110°C. The amino acid composition was determined by amino acid analysis. The conjugation load of peptide to OMPC protein was determined by comparing the conjugate amino acid composition with both that of the OMPC carrier and that of peptide ligand and by multiple regression, least squares analysis of the data (Shuler et al., 1992, J. Immunol. Methods 156:137-149). For the conjugate OMPC-(thioEP17GluN17)₃, a molar ratio of peptide versus OMPC mole of 541 was obtained; while for OMPC-(thioEPN17)₃, a molar ratio of peptide versus OMPC mole of 443 was obtained.

*Results -* The design of two, thioether bond-stabilized chimeric peptides based on the CCEZN17 sequence (SEQ ID NO:44) is described in this example. The first trimeric coiled-coil, Ac-C(Acm)-(thioEP17GluN17)₃ is based a 17 amino acid EZ-like scaffold, while the second coiled-coil, Ac-C(Acm)-(thioEPN17)₃ is based on the 24 amino acid EZ scaffold. Both constructs are covalently-stabilized via the formation of thioether bonds between the individual chimeric peptides, as described in detail *supra.* Changes, i.e., mutations or side chain derivatizations, were introduced at various places within the scaffold sequence of the designed coiled-coils and are "underlined" in Table 5.

**Table 5: Chimeric peptides for participation in chemoselective ligation reactions generating thioether bonds between said peptides**

| Peptide No. | **Peptide Name** | **Peptide characteristics¹** |
|---|---|---|
| 1 | Br-acetyl-GGGEP17GluN17 | Br-acetyl-*GGGIEKKIEEIEEKIEEIEK*LLQLTVWGIKQLQARIL (SEQ ID NO:87) |
| 2 | Ac-C(Acm)Ttds CCEP17GluN17 | *Ac-C(Acm)Ttds-CCGGIEKKIEEIEEKIEEIEK*LLQLTVWGIKQLQARIL (SEQ ID NO:88) |
| 3 | Br-acetyl-GGGEPN17 | Br-acetyl-*GGGIEKKIEEIEKKIEEIEKKIEEIEK*LLQLTVWGIKQLQARIL (SEQ ID NO:89) |
| 4 | Ac-C(Acm)Ttds CCEPN17 | Ac-C(Acm)Ttds-CCGGIEKKIEEIEKKIEEIEKKIEEIEKLLQLTVWGIKQ LQARIL (SEQ ID NO:90) |

| | | |
|---|---|---|
| ¹italics, non-HIV residues or linker regions; underlined lysine residues ("K"), contains an attached PEG236 moiety; double-underlined glutamic acid residue, lysine to glutamic acid mutation; "Acm," acetamidomethyl protection group; "Ac," acetylated NH₂-terminus | | |

C(Acm)-(thioEP17GluN17)₃ is comprised of one CC-chimeric N-peptide, CCCE17N17 (SEQ ID NO:88; peptide no. 2 in Table 5) and two derivatized-chimeric N-peptides, Bromoacetyl-GGGE17GluN17 (SEQ ID NO:87; peptide no. 1 in Table 5). The NH₂-terminal cysteine residue of CCCE17GluN17 is protected by an Acm (acetamidomethyl) group, and the remaining unprotected cysteine residues each form a chemoselective, thioether bond with the bromo-acetyl moiety of each derivatized-chimeric N-peptide. The scaffold domain of each of the chimeric peptides comprised within C(Acm)-(thioEP17GluN17)₃ consists of a shortened EZ coiled-coil of 17 amino acids that further contains a lysine to glutamic acid mutation ("E17Glu" scaffold; SEQ ID NO:84), instead of the 24 amino acid residues of the EZ scaffold (SEQ ID NO:15). The scaffold domain of C(Acm)-(thioEP17GluN17)₃ is selectively-shielded by the attachment of one PEG236 moiety to a single lysine residue within the scaffold region of each individual peptide comprised within the trimeric coiled-coil (underlined in peptide nos. 1 and 2 of Table 5). The pegylated lysine residue of each peptide is located at an "f" position of the heptad repeat of the scaffold helix, and the PEG moiety is attached via the ε-amino group of said residue. Shortening of the scaffold from 24 to 17 amino acids facilitates the conjugation of the resulting coiled-coil, C(Acm)-(thioEP17GluN17)₃, to an OMPC carrier, as well as the solubilization of the peptide in the aqueous buffer used for conjugation, by lowering the isoelectric point ("pI") of the coiled-coil. Both the derivatization of the ε-amino group of the lysine residues with PEG236, eliminating a free amino group, and the Lys to Glu mutation helps to further lower the pI of the resulting coiled-coil. See Table 6 for a comparison of the isoelectric points for the various mimetics of HIV gp41 fusion intermediates described in this example. Conjugation of C(Acm)-(thioEP17GluN17)₃ to a maleimide-activated OMPC carrier can occur through the NH₂-terminal cysteine residue once it is deprotected to obtain thiolated peptides, forming C-(thioEP17GluN17)₃.

C(Acm)-(thioEPN17)₃ is comprised of one CC-chimeric N-peptide, CCCEZN17 (SEQ ID NO:90; peptide no. 4 in Table 5) and two derivatized-chimeric N-peptides, Bromoacetyl-GGGEZN17 (SEQ ID NO:89; peptide no. 3 in Table 5). The NH₂-terminal cysteine residue of CCCEZN17 is protected by an Acm (acetamidomethyl) group, and the remaining unprotected cysteine residues each form a chemoselective, thioether bond with the bromo-acetyl moiety of each derivatized-chimeric N-peptide. The scaffold domain of each of the chimeric peptides comprised within C(Acm)-(thioEPN17)₃ consists of the 24 amino acid residues of the EZ scaffold (SEQ ID NO: 15). The scaffold domain of C(Acm)-(thioEPN17)₃ is selectively-shielded by the attachment of a PEG236 moiety to two lysine residue within the scaffold region of each individual peptide comprised within the trimeric coiled-coil (underlined in peptide nos. 3 and 4 of Table 5). The pegylated lysine residues of each peptide are located at "f" positions of the heptad repeat of the scaffold helix, and the PEG moieties are attached via the ε-amino group of said residues. Derivatization of the ε-amino group of the lysine residues with PEG236, eliminating a free amino group, helps to lower the pI of the resulting coiled-coil (see Table 6). Conjugation of C(Acm)-(thioEPN17)₃ to a maleimide-activated OMPC carrier can occur through the NH₂-terminal cysteine residue once it is deprotected to obtain thiolated peptides , forming C-(thioEPN17)₃.

**Table 6: Comparison of the isoelectric point ("pI") of various mimetics of HIV gp41 fusion intermediates described as part of the present invention.**

| **Gp41 Mimetics¹** | **pI** |
|---|---|
| (CCEZN17)₃ | 5.30 |
| Ac-C(Acm)-(thioEPN17)₃ | 4.6 |
| Ac-C-(thioEPN17)₃ | 4.6 |
| Ac-C(Acm)-(thioEP17GluN17)₃ | 4.25 |
| Ac-C-(thioEP17GluN17)₃ | 4.25 |

| | |
|---|---|
| ¹Acm," acetamidomethyl protection group; "Ac," acetylated NH₂-terminus | |

## Claims

1. A method of shielding a binding site within a polypeptide comprising site-specifically attaching at least one small molecular weight, water-soluble polymer to said polypeptide such that the binding site is selectively masked by said polymer.

2. A method of claim 1, wherein said binding site is an epitope of a polypeptide antigen.

3. A method of claim 2, wherein said small molecular weight, water-soluble polymer is site-specifically attached to an amino acid residue of said polypeptide.

4. A method of claim 3, wherein said small molecular weight, water-soluble polymer is selected from the group consisting of polyalkylene oxide, polyamide alkylene oxide and derivatives thereof.

5. A method of claim 4, wherein said small molecular weight, water-soluble polymer is polyethylene glycol (PEG).

6. A method of claim 5, wherein said PEG polymer has a molecular weight of less than about 1000 Da.

7. A method of claim 6, wherein said epitope is located within a scaffold domain of a covalently-stabilized, trimeric coiled-coil structure that mimics all or a portion of an internal, trimeric coiled-coil motif of an enveloped virus membrane-fusion protein comprising three chimeric peptides covalently-stabilized by one or more covalent bonds between said peptides, each chimeric peptide comprising a scaffold domain which comprises a soluble, trimeric form of a coiled-coil fused in helical phase to all or a portion of a NH₂-terminal heptad repeat domain, or a modified form thereof, of said enveloped virus membrane-fusion protein.

8. A method of claim 7, wherein said enveloped virus membrane-fusion protein is HIV gp41.

9. A method of shielding an epitope of a polypeptide antigen comprising site-specifically attaching at least one small molecular weight, water-soluble polymer to at least one amino acid residue within said polypeptide such that the epitope is selectively masked by said polymer.

10. A method of claim 9, wherein said small molecular weight, water-soluble polymer is selected from the group consisting of polyalkylene oxide, polyamide alkylene oxide and derivatives thereof.

11. A method of claim 10, wherein said small molecular weight, water-soluble polymer is polyethylene glycol (PEG) with a molecular weight of less than about 1000 Da.

12. A method of shielding an epitope of a covalently-stabilized, trimeric coiled-coil structure that mimics all or a portion of an internal, trimeric coiled-coil motif of HIV gp41 comprising site-specifically attaching at least one small molecular weight, water-soluble polymer to at least one amino acid residue of said coiled-coil structure such that said epitope is selectively masked by said polymer, wherein said coiled-coil structure comprises three chimeric peptides covalently-stabilized by one or more covalent bonds between said peptides, each chimeric peptide comprising a scaffold domain which comprises a soluble, trimeric form of a coiled-coil fused in helical phase to all or a portion of a N-peptide of HIV gp41, or a modified form thereof, and wherein said selectively-masked epitope is located within the scaffold domain of said chimeric peptides.

13. A method of claim 12, wherein said small molecular weight, water-soluble polymer is selected from the group consisting of polyalkylene oxide, polyamide alkylene oxide and derivatives thereof.

14. A method of claim 13, wherein said small molecular weight, water-soluble polymer is polyethylene glycol (PEG) with a molecular weight of less than about 1000 Da.

15. A polymer-modified, covalently-stabilized, trimeric coiled-coil that mimics all or a portion of a HIV gp41 N-peptide coiled-coil comprising three chimeric peptides, each chimeric peptide comprising a scaffold domain which comprises a soluble, trimeric form of a coiled-coil fused in helical phase to all or a portion of a HIV gp41 N-peptide, or a modified form thereof, wherein an epitope located within the scaffold domain is selectively masked by at least one small molecular weight, water-soluble polymer site-specifically attached to at least one amino acid residue within said scaffold domain.

16. A coiled-coil of claim 15, wherein said small molecular weight, water-soluble polymer is selected from the group consisting of polyalkylene oxide, polyamide alkylene oxide and derivatives thereof.

17. A coiled-coil of claim 16, wherein said small molecular weight, water-soluble polymer is polyethylene glycol (PEG) with a molecular weight of less than about 1000 Da.

18. A coiled-coil of claim 17, wherein each of the three chimeric peptides within said coiled-coil further comprises at least two cysteine residues located outside of the helical domain of said peptide, and the coiled-coil is covalently-stabilized via disulfide bonds between said cysteine residues on juxtaposed peptides.

19. A coiled-coil of claim 17, wherein a first of the three chimeric peptides within said coiled-coil further comprises at least two cysteine residues located outside of the helical domain of said peptide, and a second and third of the three chimeric peptides within said coiled-coil further comprises an electrophilic moiety located outside of the helical domain of said peptide that is capable of forming a thioether bond, and the coiled-coil is covalently-stabilized via thioether bonds between a nucleophilic sulfhydryl of each cysteine residue of the first chimeric peptide and the electrophilic moiety of each second and third chimeric peptide.

20. A polymer-modified, covalently-stabilized, trimeric coiled-coil that mimics all or a portion of a HIV gp41 N-peptide coiled-coil selected from the group consisting of (CCIZN17)₃, C(thioEZN17)₃ and C(thioE17GluN17)₃, wherein an epitope located within the scaffold domain of said coiled-coil is selectively masked by at least one small molecular weight, water-soluble polymer site-specifically attached to at least one amino acid residue within said scaffold domain.

21. A coiled-coil of claim 20, wherein said small molecular weight, water-soluble polymer is selected from the group consisting of polyalkylene oxide, polyamide alkylene oxide and derivatives thereof.

22. A coiled-coil of claim 21, wherein said small molecular weight, water-soluble polymer is polyethylene glycol (PEG) with a molecular weight of less than about 1000 Da.

23. A polymer-modified, soluble chimeric peptide which comprises:
(a) a scaffold portion comprising a soluble, trimeric form of a coiled-coil;
(b) a N-peptide portion comprising all or a portion of a N-peptide of HIV gp41, or a modified form thereof; and,
(c) a cysteine portion comprising at least two cysteine residues;
wherein said scaffold portion in (a) is fused in helical phase to said N-peptide portion in (b), forming an α-helical domain; said cysteine portion in (c) is located outside of said α-helical domain; and an epitope located within said scaffold portion is selectively masked by at least one small molecular weight, water-soluble polymer site-specifically attached to at least one amino acid residue within said scaffold portion.

24. A chimeric peptide of claim 23, wherein said small molecular weight, water-soluble polymer is selected from the group consisting of polyalkylene oxide, polyamide alkylene oxide and derivatives thereof.

25. A chimeric peptide of claim 24, wherein said small molecular weight, water-soluble polymer is polyethylene glycol (PEG) with a molecular weight of less than about 1000 Da.

26. A chimeric peptide of claim 25, wherein said N-peptide portion comprises a sufficient portion of HIV gp41 N-peptide to include amino acid residues which form a N-helix hydrophobic pocket.

27. A polymer-modified chimeric peptide that is CCIPN17.

28. An antigenic conjugate of a soluble chimeric peptide of claim 23, wherein said peptide is covalently linked to an immunogenic carrier molecule.

29. An antigenic conjugate of claim 28, wherein said carrier molecule is OMPC.

30. An antigenic conjugate of a covalently-stabilized, trimeric coiled-coil of claim 15, wherein said trimeric coiled-coil is covalently linked to an immunogenic carrier molecule.

31. An antigenic conjugate of claim 30, wherein said carrier molecule is OMPC.

32. A pharmaceutical composition comprising an effective amount of an antigenic conjugate of claim 30 mixed with a biologically effective adjuvant, protein, or other agent capable of increasing the immune response, wherein said composition is useful as an immunogen capable of eliciting HIV-specific neutralizing antibodies in mammals.


SEQUENCE LISTING

<110> Istituto di Ricerche di Biologia Molecolare P. Angeletti S.p.A.

<120> Method for shielding functional sites or
  epitopes on proteins

<130> ITR0092PCT

<150> 60/668,354
<151> 2005-04-05

<160> 100

<170> FastSEQ for Windows Version 4.0

<210> 1
<211> 41
<212> PRT
<213> Artificial Sequence

<220>
<223> Peptide

<400> 1
Ile Lys Lys Glu Ile Glu Ala Ile Lys Lys Glu Gln Glu Ala Ile Lys
 1               5                   10                  15
Lys Lys Ile Glu Ala Ile Glu Lys Leu Leu Gln Leu Thr Val Trp Gly
            20                  25                  30
Ile Lys Gln Leu Gln Ala Arg Ile Leu
        35                  40

<210> 2
<211> 45
<212> PRT
<213> Artificial Sequence

<220>
<223> Peptide

<400> 2
Cys Cys Gly Gly Ile Lys Lys Glu Ile Glu Ala Ile Lys Lys Glu Gln
 1               5                   10                  15
Glu Ala Ile Lys Lys Lys Ile Glu Ala Ile Glu Lys Leu Leu Gln Leu
            20                  25                  30
Thr Val Trp Gly Ile Lys Gln Leu Gln Ala Arg Ile Leu
        35                  40                  45

<210> 3
<211> 30
<212> PRT
<213> Artificial Sequence

<220>
<223> Peptide

<400> 3
Gly Cys Cys Gly Gly Ile Lys Lys Glu Ile Glu Ala Ile Lys Lys Glu
1               5                   10                  15
Gln Glu Ala Ile Lys Lys Lys Ile Glu Ala Ile Glu Lys Glu
            20                  25                  30

<210> 4
<211> 42
<212> PRT
<213> Artificial Sequence

<220>
<223> Peptide

<400> 4

<210> 5
<211> 16
<212> PRT
<213> Artificial Sequence

<220>
<223> Peptide

<400> 5
Asp Ala Glu Phe Arg His Asp Ser Gly Tyr Glu Val His His Gln Lys
1               5                   10                  15

<210> 6
<211> 19
<212> PRT
<213> Artificial Sequence

<220>
<223> Peptide

<400> 6
Lys Trp Ala Ser Leu Trp Asn Trp Phe Asn Ile Thr Asn Trp Leu Trp
1               5                   10                  15
Tyr Ile Lys

<210> 7
<211> 24
<212> PRT
<213> Artificial Sequence

<220>

<223> Peptide

<400> 7
Cys Gly Gly Ile Lys Trp Leu Ser Leu Trp Ile Trp Phe Leu Ile Thr
1               5                   10                  15
Asn Ile Leu Trp Leu Ile Lys Lys
            20

<210> 8
<211> 24
<212> PRT
<213> Artificial Sequence

<220>
<223> Peptide

<400> 8
Cys Gly Gly Ile Lys Gln Leu Glu Asp Lys Ile Glu Glu Leu Leu Ser
1               5                   10                  15
Lys Ile Tyr His Leu Glu Asn Glu
            20

<210> 9
<211> 24
<212> PRT
<213> Artificial Sequence

<220>
<223> Peptide

<400> 9
Cys Gly Gly Ile Lys Gln Leu Glu Lys Lys Ile Glu Glu Leu Leu Ser
1               5                   10                  15
Lys Ile Tyr His Leu Glu Asn Glu
            20

<210> 10
<211> 4
<212> PRT
<213> Artificial Sequence

<220>
<223> Peptide

<400> 10
Cys Cys Gly Gly
1

<210> 11
<211> 45
<212> PRT
<213> Artificial Sequence

<220>
<223> Peptide

<400> 11
Arg Met Lys Gln Ile Glu Asp Lys Ile Glu Glu Ile Glu Ser Lys Gln
1               5                   10                  15
Lys Lys Ile Glu Asn Glu Ile Ala Arg Ile Lys Lys Leu Leu Gln Leu
            20                  25                  30
Thr Val Trp Gly Ile Lys Gln Leu Gln Ala Arg Ile Leu
        35                  40                  45

<210> 12
<211> 31
<212> PRT
<213> Artificial Sequence

<220>
<223> Peptide

<400> 12
Tyr Gly Gly Ile Glu Lys Lys Ile Glu Ala Ile Glu Lys Lys Ile Glu
1               5                   10                  15
Ala Ile Glu Lys Lys Ile Glu Ala Ile Glu Lys Lys Ile Glu Ala
            20                  25                  30

<210> 13
<211> 33
<212> PRT
<213> Artificial Sequence

<220>
<223> Peptide

<400> 13
Arg Met Lys Gln Ile Glu Asp Lys Ile Glu Glu Ile Leu Ser Lys Gln
1               5                   10                  15
Tyr His Ile Glu Asn Glu Ile Ala Arg Ile Lys Lys Leu Ile Gly Glu
            20                  25                  30
Arg

<210> 14
<211> 24
<212> PRT
<213> Artificial Sequence

<220>
<223> Peptide

<400> 14
Ile Lys Lys Glu Ile Glu Ala Ile Lys Lys Glu Gln Glu Ala Ile Lys
1               5                   10                  15
Lys Lys Ile Glu Ala Ile Glu Lys
            20

<210> 15
<211> 24

<212> PRT
<213> Artificial Sequence

<220>
<223> Peptide

<400> 15
Ile Glu Lys Lys Ile Glu Glu Ile Glu Lys Lys Ile Glu Glu Ile Glu
1               5                   10                  15
Lys Lys Ile Glu Glu Ile Glu Lys
            20

<210> 16
<211> 10
<212> PRT
<213> Artificial Sequence

<220>
<223> Peptide

<400> 16
Ile Lys Lys Lys Ile Glu Ala Ile Glu Lys
1               5                   10

<210> 17
<211> 28
<212> PRT
<213> Artificial Sequence

<220>
<223> Peptide

<400> 17
Arg Met Lys Gln Ile Glu Asp Lys Ile Glu Glu Ile Glu Ser Lys Gln
1               5                   10                  15
Lys Lys Ile Glu Asn Glu Ile Ala Arg Ile Lys Lys
            20                  25

<210> 18
<211> 17
<212> PRT
<213> Artificial Sequence

<220>

<223> Peptide

<400> 18
Ile Lys Lys Glu Ile Glu Ala Ile Lys Lys Glu Gln Glu Ala Ile Lys
1               5                   10                  15
Lys


<210> 19
<211> 24

<212> PRT
<213> Artificial Sequence

<220>
<223> Peptide

<400> 19
Ile Glu Lys Lys Ile Glu Ala Ile Glu Lys Lys Ile Glu Ala Ile Glu
1               5                   10                  15
Lys Lys Ile Glu Ala Ile Glu Lys
            20

<210> 20
<211> 17
<212> PRT
<213> Artificial Sequence

<220>
<223> Peptide

<400> 20
Ile Glu Lys Lys Ile Glu Ala Ile Glu Lys Lys Ile Glu Ala Ile Glu
1               5                   10                  15
Lys

<210> 21
<211> 10
<212> PRT
<213> Artificial Sequence

<220>
<223> Peptide

<400> 21
Ile Glu Lys Lys Ile Glu Ala Ile Glu Lys
1               5                   10

<210> 22
<211> 10
<212> PRT
<213> Artificial Sequence

<220>
<223> Peptide

<400> 22
Ile Glu Glu Lys Ile Glu Glu Ile Glu Glu
1               5                   10

<210> 23
<211> 48
<212> PRT
<213> Artificial Sequence

<220>
<223> Peptide

<400> 23
Ile Lys Lys Glu Ile Glu Ala Ile Lys Lys Glu Gln Glu Ala Ile Lys
1               5                   10                  15
Lys Lys Ile Glu Ala Ile Glu Lys Glu Ile Glu Ala Gln Gln His Leu
            20                  25                  30
Leu Gln Leu Thr Val Trp Gly Ile Lys Gln Leu Gln Ala Arg Ile Leu
        35                  40                  45

<210> 24
<211> 62
<212> PRT
<213> Artificial Sequence

<220>
<223> Peptide

<400> 24
Ile Lys Lys Glu Ile Glu Ala Ile Lys Lys Glu Gln Glu Ala Ile Lys
1               5                   10                  15
Lys Lys Ile Glu Ala Ile Glu Lys Glu Ile Ser Gly Ile Val Gln Gln
            20                  25                  30
Gln Asn Asn Leu Leu Arg Ala Ile Glu Ala Gln Gln His Leu Leu Gln
        35                  40                  45
Leu Thr Val Trp Gly Ile Lys Gln Leu Gln Ala Arg Ile Leu
    50                  55                  60

<210> 25
<211> 15
<212> PRT
<213> Artificial Sequence

<220>
<223> Peptide

<400> 25
Lys Gln Lys Lys Ile Glu Asn Glu Ile Ala Ala Ile Lys Lys Leu
1               5                   10                  15

<210> 26
<211> 15
<212> PRT
<213> Artificial Sequence

<220>
<223> Peptide

<400> 26
Lys Ile Lys Lys Ile Glu Asn Glu Ile Ala Arg Ile Lys Lys Leu
1               5                   10                  15

<210> 27
<211> 22

<212> PRT
<213> Artificial Sequence

<220>
<223> Peptide

<400> 27
Lys Ile Glu Glu Ile Glu Ser Lys Gln Lys Lys Ile Glu Asn Glu Ile
1               5                   10                  15
Ala Arg Ile Lys Lys Leu
            20

<210> 28
<211> 21
<212> PRT
<213> Artificial Sequence

<220>
<223> Peptide

<400> 28
Lys Ile Glu Glu Ile Glu Ser Lys Ile Lys Lys Ile Glu Asn Glu Ile
1               5                   10                  15
Ala Arg Ile Lys Lys
            20

<210> 29
<211> 36
<212> PRT
<213> Artificial Sequence

<220>
<223> Peptide

<400> 29
Ser Gly Ile Val Gln Gln Gln Asn Asn Leu Leu Arg Ala Ile Glu Ala
1               5                   10                  15
Gln Gln His Leu Leu Gln Leu Thr Val Trp Gly Ile Lys Gln Leu Gln
            20                  25                  30
Ala Arg Ile Leu
        35

<210> 30
<211> 34
<212> PRT
<213> Artificial Sequence

<220>
<223> Peptide

<400> 30
Trp Met Glu Trp Asp Arg Glu Ile Asn Asn Tyr Thr Ser Leu Ile His
1               5                   10                  15
Ser Leu Ile Glu Glu Ser Gln Asn Gln Gln Glu Lys Asn Glu Gln Glu
            20                  25                  30

Leu Leu

<210> 31
<211> 48
<212> PRT
<213> Artificial Sequence

<220>
<223> Peptide

<400> 31
Ala Arg Gln Leu Leu Ser Gly Ile Val Gln Gln Gln Asn Asn Leu Leu
1               5                   10                  15
Arg Ala Ile Glu Ala Gln Gln His Leu Leu Gln Leu Thr Val Trp Gly
            20                  25                  30
Ile Lys Gln Leu Gln Ala Arg Ile Leu Ala Val Glu Arg Tyr Leu Lys
        35                  40                  45

<210> 32
<211> 17
<212> PRT
<213> Artificial Sequence

<220>
<223> Peptide

<400> 32
Leu Leu Gln Leu Thr Val Trp Gly Ile Lys Gln Leu Gln Ala Arg Ile
1               5                   10                  15
Leu

<210> 33
<211> 23
<212> PRT
<213> Artificial Sequence

<220>
<223> Peptide

<400> 33
Ile Glu Ala Gln Gln His Leu Leu Gln Leu Thr Val Trp Gly Ile Lys
1               5                   10                  15
Gln Leu Gln Ala Arg Ile Leu
            20

<210> 34
<211> 16
<212> PRT
<213> Artificial Sequence

<220>
<223> Peptide

<400> 34
Leu Leu Gln Leu Thr Val Trp Gly Ile Lys Ala Leu Ala Ala Ala Ile
1               5                   10                  15

<210> 35
<211> 13
<212> PRT
<213> Artificial Sequence

<220>
<223> Peptide

<400> 35
Leu Leu Gln Leu Thr Val Trp Gly Ile Lys Gln Leu Gln
1               5                   10

<210> 36
<211> 17
<212> PRT
<213> Artificial Sequence

<220>
<223> Peptide

<400> 36
Leu Ile Gln Leu Ile Val Trp Gly Ile Lys Gln Ile Gln Ala Arg Ile
1               5                   10                  15

Leu


<210> 37
<211> 36
<212> PRT
<213> Artificial Sequence

<220>
<223> Peptide

<400> 37
Ala Gly Ile Val Gln Gln Gln Gln Gln Leu Leu Asp Val Val Lys Arg
1               5                   10                  15
Gln Gln Glu Leu Leu Arg Leu Thr Val Trp Gly Thr Lys Asn Leu Gln
            20                  25                  30
Thr Arg Val Ser
        35

<210> 38
<211> 36
<212> PRT
<213> Artificial Sequence

<220>
<223> Peptide

<400> 38
Gln Ser Leu Ala Asn Ala Thr Ala Ala Gln Gln Asn Val Leu Glu Ala
1               5                   10                  15
Thr Tyr Ala Met Val Gln His Val Ala Lys Gly Val Arg Ile Leu Glu
            20                  25                  30
Ala Arg Val Ala
        35

<210> 39
<211> 36
<212> PRT
<213> Artificial Sequence

<220>
<223> Peptide

<400> 39
Gln Thr Leu Ala Asn Ala Thr Ala Ala Gln Gln Asp Ala Leu Glu Ala
1               5                   10                  15
Thr Tyr Ala Met Val Gln His Val Ala Lys Gly Val Arg Ile Leu Glu
            20                  25                  30
Ala Arg Val Ala
        35

<210> 40
<211> 36
<212> PRT
<213> Artificial Sequence

<220>
<223> Peptide

<400> 40
Ala Thr His Gln Glu Thr Ile Glu Lys Val Thr Glu Ala Leu Lys Ile
1               5                   10                  15
Asn Asn Leu Arg Leu'Val Thr Leu Glu His Gln Val Leu Val Ile Gly
            20                  25                  30
Leu Lys Val Glu
        35

<210> 41
<211> 36
<212> PRT
<213> Artificial Sequence

<220>
<223> Peptide

<400> 41
Asn His Thr Phe Glu Val Glu Asn Ser Thr Leu Asn Gly Met Asp Leu
1               5                   10                  15
Ile Glu Arg Gln Ile Lys Ile Leu Tyr Ala Met Ile Leu Gln Thr His
            20                  25                  30
Ala Asp Val Gln
        35

<210> 42
<211> 36
<212> PRT
<213> Artificial Sequence

<220>
<223> Peptide

<400> 42
Glu Arg Val Val Gln Asn Val Ser Tyr Ile Ala Gln Thr Gln Asp Gln
1               5                   10                  15
Phe Thr His Leu Phe Arg Asn Ile Asn Asn Arg Leu Asn Val Leu His
            20                  25                  30
Arg Arg Val Ser
        35

<210> 43
<211> 45
<212> PRT
<213> Artificial Sequence

<220>
<223> Peptide

<400> 43
Cys Cys Gly Gly Ile Lys Lys Glu Ile Glu Ala Ile Lys Lys Glu Gln
1               5                   10                  15
Glu Ala Ile Lys Lys Lys Ile Glu Ala Ile Glu Lys Leu Leu Gln Leu
            20                  25                  30
Thr Val Trp Gly Ile Lys Ala Leu Ala Ala Ala Ile Ala
        35                  40                  45

<210> 44
<211> 45
<212> PRT
<213> Artificial Sequence

<220>
<223> Peptide

<400> 44
Cys Cys Gly Gly Ile Glu Lys Lys Ile Glu Glu Ile Glu Lys Lys Ile
1               5                   10                  15
Glu Glu Ile Glu Lys Lys Ile Glu Glu Ile Glu Lys Leu Leu Gln Leu
            20                  25                  30
Thr Val Trp Gly Ile Lys Gln Leu Gln Ala Arg Ile Leu
        35                  40                  45

<210> 45
<211> 45
<212> PRT
<213> Artificial Sequence

<220>

<223> Peptide

<400> 45
Cys Cys Gly Gly Ile Glu Lys Lys Ile Glu Glu Ile Glu Lys Lys Ile
1               5                   10                  15
Glu Glu Ile Glu Lys Lys Ile Glu Glu Ile Glu Lys Leu Leu Gln Leu
            20                  25                  30
Thr Val Trp Gly Ile Lys Ala Leu Ala Ala Ala Ile Ala
        35                  40                  45

<210> 46
<211> 52
<212> PRT
<213> Artificial Sequence

<220>
<223> Peptide

<400> 46
Cys Cys Gly Gly Ile Lys Lys Glu Ile Glu Ala Ile Lys Lys Glu Gln
1               5                   10                  15
Glu Ala Ile Lys Lys Lys Ile Glu Ala Ile Glu Lys Glu Ile Glu Ala
            20                  25                  30
Gln Gln His Leu Leu Gln Leu Thr Val Trp Gly Ile Lys Gln Leu Gln
        35                  40                  45
Ala Arg Ile Leu
    50

<210> 47
<211> 52
<212> PRT
<213> Artificial Sequence

<220>
<223> Peptide

<400> 47
Cys Cys Gly Gly Ile Glu Lys Lys Ile Glu Glu Ile Glu Lys Lys Ile
1               5                   10                  15
Glu Glu Ile Glu Lys Lys Ile Glu Glu Ile Glu Glu Lys Ile Glu Ala
            20                  25                  30
Gln Gln His Leu Leu Gln Leu Thr Val Trp Gly Ile Lys Gln Leu Gln
        35                  40                  45
Ala Arg Ile Leu
    50

<210> 48
<211> 66
<212> PRT
<213> Artificial Sequence

<220>
<223> Peptide

<400> 48

Cys Cys Gly Gly Ile Lys Lys Glu Ile Glu Ala Ile Lys Lys Glu Gln
1               5                   10                  15
Glu Ala Ile Lys Lys Lys Ile Glu Ala Ile Glu Lys Glu Ile Ser Gly
            20                  25                  30
Ile Val Gln Gln Gln Asn Asn Leu Leu Arg Ala Ile Glu Ala Gln Gln
        35                  40                  45
His Leu Leu Gln Leu Thr Val Trp Gly Ile Lys Gln Leu Gln Ala Arg
    50                  55                  60
Ile Leu
65

<210> 49
<211> 66
<212> PRT
<213> Artificial Sequence

<220>
<223> Peptide

<400> 49
Cys Cys Gly Gly Ile Glu Lys Lys Ile Glu Glu Ile Glu Lys Lys Ile
1               5                   10                  15
Glu Glu Ile Glu Lys Lys Ile Glu Glu Ile Glu Glu Lys Ile Ser Gly
            20                  25                  30
Ile Val Gln Gln Gln Asn Asn Leu Leu Arg Ala Ile Glu Ala Gln Gln
        35                  40                  45
His Leu Leu Gln Leu Thr Val Trp Gly Ile Lys Gln Leu Gln Ala Arg
    50                  55                  60
Ile Leu
65

<210> 50
<211> 31
<212> PRT
<213> Artificial Sequence

<220>
<223> Peptide

<400> 50
Cys Cys Gly Gly Ile Lys Lys Lys Ile Glu Ala Ile Glu Lys Leu Leu
1               5                   10                  15
Gln Leu Thr Val Trp Gly Ile Lys Gln Leu Gln Ala Arg Ile Leu
            20                  25                  30

<210> 51
<211> 48
<212> PRT
<213> Artificial Sequence

<220>
<223> Peptide

<400> 51

Ser Gly Gly Cys Cys Gly Gly Ile Lys Lys Glu Ile Glu Ala Ile Lys
1               5                   10                  15
Lys Glu Gln Glu Ala Ile Lys Lys Lys Ile Glu Ala Ile Glu Lys Leu
            20                  25                  30
Leu Gln Leu Thr Val Trp Gly Ile Lys Gln Leu Gln Ala Arg Ile Leu
        35                  40                  45

<210> 52
<211> 49
<212> PRT
<213> Artificial Sequence

<220>
<223> Peptide

<400> 52
Cys Cys Gly Gly Arg Met Lys Gln Ile Glu Asp Lys Ile Glu Glu Ile
1               5                   10                  15
Glu Ser Lys Gln Lys Lys Ile Glu Asn Glu Ile Ala Arg Ile Lys Lys
            20                  25                  30
Leu Leu Gln Leu Thr Val Trp Gly Ile Lys Gln Leu Gln Ala Arg Ile
        35                  40                  45
Leu

<210> 53
<211> 49
<212> PRT
<213> Artificial Sequence

<220>
<223> Peptide

<400> 53
Cys Cys Gly Gly Arg Met Lys Gln Ile Glu Asp Lys Ile Glu Glu Ile
1               5                   10                  15
Glu Ser Lys Gln Lys Lys Ile Glu Asn Glu Ile Ala Arg Ile Lys Lys
            20                  25                  30
Leu Leu Gln Leu Thr Val Trp Gly Ile Lys Ala Leu Ala Ala Ala Ile
        35                  40                  45
Ala

<210> 54
<211> 38
<212> PRT
<213> Artificial Sequence

<220>
<223> Peptide

<400> 54
Cys Cys Gly Gly Ile Lys Lys Glu Ile Glu Ala Ile Lys Lys Glu Gln
1               5                   10                  15

Glu Ala Ile Lys Lys Leu Leu Gln Leu Thr Val Trp Gly Ile Lys Gln
            20                  25                  30
Leu Gln Ala Arg Ile Leu
        35

<210> 55
<211> 52
<212> PRT
<213> Artificial Sequence

<220>
<223> Peptide

<400> 55
Cys Cys Gly Gly Ile Lys Lys Glu Ile Glu Ala Ile Lys Lys Glu Gln
 1               5                  10                  15
Glu Ala Ile Lys Lys Lys Ile Glu Ala Ile Glu Lys Leu Leu Gln Leu
            20                  25                  30
Thr Val Trp Gly Ile Lys Gln Leu Gln Ala Arg Ile Leu Ala Val Glu
        35                  40                  45
Arg Tyr Leu Lys
    50

<210> 56
<211> 45
<212> PRT
<213> Artificial Sequence

<220>
<223> Peptide

<400> 56
Cys Cys Gly Gly Ile Lys Lys Glu Ile Glu Ala Ile Lys Lys Glu Gln
 1               5                  10                  15
Glu Ala Ile Lys Lys Glu Ile Glu Ala Gln Gln His Leu Leu Gln Leu
            20                  25                  30
Thr Val Trp Gly Ile Lys Gln Leu Gln Ala Arg Ile Leu
        35                  40                  45

<210> 57
<211> 59
<212> PRT
<213> Artificial Sequence

<220>
<223> Peptide


<400> 57
Cys Cys Gly Gly Ile Lys Lys Glu Ile Glu Ala Ile Lys Lys Glu Gln
 1               5                  10                  15
Glu Ala Ile Lys Lys Glu Ile Ser Gly Ile Val Gln Gln Gln Asn Asn
            20                  25                  30
Leu Leu Arg Ala Ile Glu Ala Gln Gln His Leu Leu Gln Leu Thr Val
        35                  40                  45

Trp Gly Ile Lys Gln Leu Gln Ala Arg Ile Leu
    50                  55

<210> 58

<211> 45
<212> PRT
<213> Artificial Sequence

<220>
<223> Peptide

<400> 58
Cys Cys Gly Gly Ile Lys Lys Glu Ile Glu Ala Ile Lys Lys Glu Gln
 1               5                  10                  15
Glu Ala Ile Lys Lys Leu Leu Gln Leu Thr Val Trp Gly Ile Lys Gln
            20                  25                  30
Leu Gln Ala Arg Ile Leu Ala Val Glu Arg Tyr Leu Lys
        35                  40                  45

<210> 59
<211> 38
<212> PRT
<213> Artificial Sequence

<220>
<223> Peptide

<400> 59
Cys Cys Gly Gly Ile Lys Lys Glu Ile Glu Ala Ile Lys Lys Glu Gln
 1               5                  10                  15
Glu Ala Ile Lys Lys Leu Leu Gln Leu Thr Val Trp Gly Ile Lys Ala
            20                  25                  30
Leu Ala Ala Ala Ile Ala
        35

<210> 60
<211> 31
<212> PRT
<213> Artificial Sequence

<220>
<223> Peptide

<400> 60
Ile Lys Lys Lys Ile Glu Ala Ile Glu Lys Leu Leu Gln Leu Thr Val
 1               5                  10                  15
Trp Gly Ile Lys Gln Leu Gln Ala Arg Ile Leu Gly Gly Cys Cys
            20                  25                  30

<210> 61
<211> 31
<212> PRT
<213> Artificial Sequence

<220>
<223> Peptide

<400> 61
Cys Cys Gly Gly Ile Lys Lys Lys Ile Glu Ala Ile Glu Lys Leu Ile
1               5                   10                  15
Gln Leu Ile Val Trp Gly Ile Lys Gln Ile Gln Ala Arg Ile Leu
            20                  25                  30

<210> 62
<211> 31
<212> PRT
<213> Artificial Sequence

<220>
<223> Peptide

<400> 62
Ile Lys Lys Lys Ile Glu Ala Ile Glu Lys Leu Ile Gln Leu Ile Val
1               5                   10                  15
Trp Gly Ile Lys Gln Ile Gln Ala Arg Ile Leu Gly Gly Cys Cys
            20                  25                  30

<210> 63
<211> 38
<212> PRT
<213> Artificial Sequence

<220>
<223> Peptide

<400> 63
Cys Cys Gly Gly Ile Lys Lys Glu Ile Glu Ala Ile Lys Lys Glu Gln
1               5                   10                  15
Glu Ala Ile Lys Lys Leu Ile Gln Leu Ile Val Trp Gly Ile Lys Gln
            20                  25                  30
Ile Gln Ala Arg Ile Leu
        35

<210> 64
<211> 44
<212> PRT
<213> Artificial Sequence

<220>
<223> Peptide

<400> 64
Cys Cys Gly Gly Leu Leu Gln Leu Thr Val Trp Gly Ile Lys Gln Leu
1               5                   10                  15
Gln Ala Arg Ile Leu Ala Ile Lys Lys Glu Ile Glu Ala Ile Lys Lys
            20                  25                  30
Glu Gln Glu Ala Ile Lys Lys Lys Ile Glu Ala Ile
        35                  40

<210> 65
<211> 45
<212> PRT
<213> Artificial Sequence

<220>
<223> Peptide

<400> 65
Cys Cys Gly Gly Ile Glu Lys Lys Ile Glu Ala Ile Glu Lys Lys Ile
1               5                   10                  15
Glu Ala Ile Glu Lys Lys Ile Glu Ala Ile Glu Lys Leu Leu Gln Leu
            20                  25                  30
Thr Val Trp Gly Ile Lys Gln Leu Gln Ala Arg Ile Leu
        35                  40                  45

<210> 66
<211> 38
<212> PRT
<213> Artificial Sequence

<220>
<223> Peptide

<400> 66
Cys Cys Gly Gly Ile Glu Lys Lys Ile Glu Ala Ile Glu Lys Lys Ile
1               5                   10                  15
Glu Ala Ile Glu Lys Leu Leu Gln Leu Thr Val Trp Gly Ile Lys Gln
            20                  25                  30
Leu Gln Ala Arg Ile Leu
        35

<210> 67
<211> 31
<212> PRT
<213> Artificial Sequence

<220>
<223> Peptide

<400> 67
Cys Cys Gly Gly Ile Glu Lys Lys Ile Glu Ala Ile Glu Lys Leu Leu
1               5                   10                  15
Gln Leu Thr Val Trp Gly Ile Lys Gln Leu Gln Ala Arg Ile Leu
            20                  25                  30

<210> 68
<211> 31
<212> PRT
<213> Artificial Sequence

<220>
<223> Peptide

<400> 68

Cys Cys Gly Gly Ile Glu Glu Lys Ile Glu Glu Ile Glu Glu Leu Leu
1               5                   10                  15
Gln Leu Thr Val Trp Gly Ile Lys Gln Leu Gln Ala Arg Ile Leu
            20                  25                  30

<210> 69
<211> 56
<212> PRT
<213> Artificial Sequence

<220>
<223> Peptide

<400> 69
Cys Cys Gly Gly Arg Met Lys Gln Ile Glu Asp Lys Ile Glu Glu Ile
1               5                   10                  15
Glu Ser Lys Gln Lys Lys Ile Glu Asn Glu Ile Ala Arg Ile Lys Lys
            20                  25                  30
Leu Ile Glu Ala Gln Gln His Leu Leu Gln Leu Thr Val Trp Gly Ile
        35                  40                  45
Lys Gln Leu Gln Ala Arg Ile Leu
    50                  55

<210> 70
<211> 70
<212> PRT
<213> Artificial Sequence

<220>
<223> Peptide

<400> 70
Cys Cys Gly Gly Arg Met Lys Gln Ile Glu Asp Lys Ile Glu Glu Ile
1               5                   10                  15
Glu Ser Lys Gln Lys Lys Ile Glu Asn Glu Ile Ala Arg Ile Lys Lys
            20                  25                  30
Leu Ile Ser Gly Ile Val Gln Gln Gln Asn Asn Leu Leu Arg Ala Ile
        35                  40                  45
Glu Ala Gln Gln His Leu Leu Gln Leu Thr Val Trp Gly Ile Lys Gln
    50                  55                  60
Leu Gln Ala Arg Ile Leu
65                  70

<210> 71
<211> 52
<212> PRT
<213> Artificial Sequence

<220>
<223> Peptide

<400> 71
Cys Cys Gly Gly Ile Glu Lys Lys Ile Glu Glu Ile Glu Lys Lys Ile
1               5                   10                  15

Glu Glu Ile Glu Lys Lys Ile Glu Glu Ile Glu Lys Leu Leu Gln Leu
            20                  25                  30
Thr Val Trp Gly Ile Lys Gln Leu Gln Ala Arg Ile Leu Ala Val Glu
        35                  40                  45
Arg Tyr Leu Lys
    50

<210> 72
<211> 56
<212> PRT
<213> Artificial Sequence

<220>
<223> Peptide

<400> 72
Cys Cys Gly Gly Arg Met Lys Gln Ile Glu Asp Lys Ile Glu Glu Ile
 1               5                  10                  15
Glu Ser Lys Gln Lys Lys Ile Glu Asn Glu Ile Ala Arg Ile Lys Lys
            20                  25                  30
Leu Leu Gln Leu Thr Val Trp Gly Ile Lys Gln Leu Gln Ala Arg Ile
        35                  40                  45
Leu Ala Val Glu Arg Tyr Leu Lys
    50                  55

<210> 73
<211> 36
<212> PRT
<213> Artificial Sequence

<220>
<223> Peptide

<400> 73
Cys Cys Gly Gly Ile Lys Lys Lys Ile Glu Ala Ile Glu Lys Leu Leu
 1               5                  10                  15
Gln Leu Thr Val Trp Gly Ile Lys Gln Leu Gln Ala Arg Ile Leu Ala
            20                  25                  30
Val Glu Arg Tyr
        35

<210> 74
<211> 44
<212> PRT
<213> Artificial Sequence

<220>
<223> Peptide

<400> 74
Cys Cys Gly Gly Leu Leu Gln Leu Thr Val Trp Gly Ile Lys Gln Leu
 1               5                  10                  15
Gln Ala Arg Ile Leu Ala Ile Lys Lys Glu Ile Glu Ala Ile Lys Lys
            20                  25                  30

Glu Gln Glu Ala Ile Lys Lys Lys Ile Glu Ala Ile
        35                  40

<210> 75
<211> 41
<212> PRT
<213> Artificial Sequence

<220>
<223> Peptide

<400> 75
Cys Cys Gly Gly Ile Lys Lys Glu Ile Glu Ala Ile Lys Lys Glu Gln
 1               5                  10                  15
Glu Ala Ile Lys Lys Lys Ile Glu Ala Ile Glu Lys Leu Leu Gln Leu
            20                  25                  30
Thr Val Trp Gly Ile Lys Gln Leu Gln
        35                  40

<210> 76
<211> 46
<212> PRT
<213> Artificial Sequence

<220>
<223> Peptide

<400> 76
Cys Cys Cys Gly Gly Ile Lys Lys Glu Ile Glu Ala Ile Lys Lys Glu
 1               5                  10                  15
Gln Glu Ala Ile Lys Lys Lys Ile Glu Ala Ile Glu Lys Leu Leu Gln
            20                  25                  30
Leu Thr Val Trp Gly Ile Lys Gln Leu Gln Ala Arg Ile Leu
        35                  40                  45

<210> 77
<211> 44
<212> PRT
<213> Artificial Sequence

<220>
<223> Peptide

<400> 77
Gly Gly Gly Ile Lys Lys Glu Ile Glu Ala Ile Lys Lys Glu Gln Glu
 1               5                  10                  15
Ala Ile Lys Lys Lys Ile Glu Ala Ile Glu Lys Leu Leu Gln Leu Thr
            20                  25                  30
Val Trp Gly Ile Lys Gln Leu Gln Ala Arg Ile Leu
        35                  40

<210> 78
<211> 39
<212> PRT
<213> Artificial Sequence

<220>
<223> Peptide

<400> 78
Cys Cys Cys Gly Gly Ile Glu Lys Lys Ile Glu Ala Ile Glu Lys Lys
1               5                   10                  15
Ile Glu Ala Ile Glu Lys Leu Leu Gln Leu Thr Val Trp Gly Ile Lys
            20                  25                  30
Gln Leu Gln Ala Arg Ile Leu
        35

<210> 79
<211> 37
<212> PRT
<213> Artificial Sequence

<220>
<223> Peptide

<400> 79
Gly Gly Gly Ile Glu Lys Lys Ile Glu Ala Ile Glu Lys Lys Ile Glu
1               5                   10                  15
Ala Ile Glu Lys Leu Leu Gln Leu Thr Val Trp Gly Ile Lys Gln Leu
            20                  25                  30
Gln Ala Arg Ile Leu
        35

<210> 80
<211> 30
<212> PRT
<213> Artificial Sequence

<220>
<223> Peptide

<400> 80
Gly Gly Gly Ile Glu Glu Lys Ile Glu Glu Ile Glu Glu Leu Leu Gln
1               5                   10                  15
Leu Thr Val Trp Gly Ile Lys Gln Leu Gln Ala Arg Ile Leu
            20                  25                  30

<210> 81
<211> 32
<212> PRT
<213> Artificial Sequence

<220>
<223> Peptide

<400> 81
Cys Cys Cys Gly Gly Ile Glu Glu Lys Ile Glu Glu Ile Glu Glu Leu
1               5                   10                  15
Leu Gln Leu Thr Val Trp Gly Ile Lys Gln Leu Gln Ala Arg Ile Leu
            20                  25                  30

<210> 82
<211> 46
<212> PRT
<213> Artificial Sequence

<220>
<223> Peptide

<400> 82
Gly Cys Cys Gly Gly Ile Lys Lys Glu Ile Glu Ala Ile Lys Lys Glu
1               5                   10                  15
Gln Glu Ala Ile Lys Lys Lys Ile Glu Ala Ile Glu Lys Leu Leu Gln
            20                  25                  30
Leu Thr Val Trp Gly Ile Lys Gln Leu Gln Ala Arg Ile Leu
        35                  40                  45

<210> 83
<211> 17
<212> PRT
<213> Artificial Sequence

<220>

<223> Peptide

<400> 83
Ile Glu Lys Lys Ile Glu Glu Ile Glu Lys Lys Ile Glu Glu Ile Glu
1               5                   10                  15
Lys


<210> 84
<211> 17
<212> PRT
<213> Artificial Sequence

<220>
<223> Peptide

<400> 84
Ile Glu Lys Lys Ile Glu Glu Ile Glu Glu Lys Ile Glu Glu Ile Glu
1               5                   10                  15
Lys


<210> 85
<211> 38
<212> PRT
<213> Artificial Sequence

<220>
<223> Peptide

<400> 85

Cys Cys Gly Gly Ile Glu Lys Lys Ile Glu Glu Ile Glu Lys Lys Ile
1               5                   10                  15
Glu Glu Ile Glu Lys Leu Leu Gln Leu Thr Val Trp Gly Ile Lys Gln
            20                  25                  30
Leu Gln Ala Arg Ile Leu
        35

<210> 86
<211> 38
<212> PRT
<213> Artificial Sequence

<220>
<223> Peptide

<400> 86
Cys Cys Gly Gly Ile Glu Lys Lys Ile Glu Glu Ile Glu Glu Lys Ile
1               5                   10                  15
Glu Glu Ile Glu Lys Leu Leu Gln Leu Thr Val Trp Gly Ile Lys Gln
            20                  25                  30
Leu Gln Ala Arg Ile Leu
        35

<210> 87
<211> 37
<212> PRT
<213> Artificial Sequence

<220>
<223> Peptide

<400> 87
Gly Gly Gly Ile Glu Lys Lys Ile Glu Glu Ile Glu Glu Lys Ile Glu
1               5                   10                  15
Glu Ile Glu Lys Leu Leu Gln Leu Thr Val Trp Gly Ile Lys Gln Leu
            20                  25                  30
Gln Ala Arg Ile Leu
        35

<210> 88
<211> 39
<212> PRT
<213> Artificial Sequence

<220>
<223> Peptide

<400> 88
Cys Cys Cys Gly Gly Ile Glu Lys Lys Ile Glu Glu Ile Glu Glu Lys
1               5                   10                  15
Ile Glu Glu Ile Glu Lys Leu Leu Gln Leu Thr Val Trp Gly Ile Lys
            20                  25                  30
Gln Leu Gln Ala Arg Ile Leu
        35

<210> 89
<211> 44
<212> PRT
<213> Artificial Sequence

<220>
<223> Peptide

<400> 89
Gly Gly Gly Ile Glu Lys Lys Ile Glu Glu Ile Glu Lys Lys Ile Glu
1               5                   10                  15
Glu Ile Glu Lys Lys Ile Glu Glu Ile Glu Lys Leu Leu Gln Leu Thr
            20                  25                  30
Val Trp Gly Ile Lys Gln Leu Gln Ala Arg Ile Leu
        35                  40

<210> 90
<211> 46
<212> PRT
<213> Artificial Sequence

<220>
<223> Peptide

<400> 90
Cys Cys Cys Gly Gly Ile Glu Lys Lys Ile Glu Glu Ile Glu Lys Lys
1               5                   10                  15
Ile Glu Glu Ile Glu Lys Lys Ile Glu Glu Ile Glu Lys Leu Leu Gln
            20                  25                  30
Leu Thr Val Trp Gly Ile Lys Gln Leu Gln Ala Arg Ile Leu
        35                  40                  45

<210> 91
<211> 37
<212> PRT
<213> Artificial Sequence

<220>
<223> Peptide

<400> 91
Gly Gly Gly Ile Glu Lys Lys Ile Glu Glu Ile Glu Lys Lys Ile Glu
1               5                   10                  15
Glu Ile Glu Lys Leu Leu Gln Leu Thr Val Trp Gly Ile Lys Gln Leu
            20                  25                  30
Gln Ala Arg Ile Leu
        35

<210> 92
<211> 39
<212> PRT
<213> Artificial Sequence

<220>
<223> Peptide

<400> 92
Cys Cys Cys Gly Gly Ile Glu Lys Lys Ile Glu Glu Ile Glu Lys Lys
1               5                   10                  15
Ile Glu Glu Ile Glu Lys Leu Leu Gln Leu Thr Val Trp Gly Ile Lys
            20                  25                  30
Gln Leu Gln Ala Arg Ile Leu
        35

<210> 93
<211> 31
<212> PRT
<213> Artificial Sequence

<220>
<223> Peptide

<400> 93
Lys Ile Glu Glu Ile Glu Glu Lys Ile Glu Glu Ile Glu Lys Leu Leu
1               5                   10                  15
Gln Leu Thr Val Trp Gly Ile Lys Gln Leu Gln Ala Arg Ile Leu
            20                  25                  30

<210> 94
<211> 36
<212> PRT
<213> Artificial Sequence

<220>
<223> Peptide

<400> 94
Gly Gly Ile Glu Lys Lys Ile Glu Glu Ile Glu Glu Lys Ile Glu Glu
1               5                   10                  15
Ile Glu Lys Leu Leu Gln Leu Thr Val Trp Gly Ile Lys Gln Leu Gln
            20                  25                  30
Ala Arg Ile Leu
        35

<210> 95
<211> 31
<212> PRT
<213> Artificial Sequence

<220>
<223> Peptide

<400> 95
Lys Ile Glu Glu Ile Glu Lys Lys Ile Glu Glu Ile Glu Lys Leu Leu
1               5                   10                  15
Gln Leu Thr Val Trp Gly Ile Lys Gln Leu Gln Ala Arg Ile Leu
            20                  25                  30

<210> 96
<211> 6

<212> PRT
<213> Artificial Sequence

<220>
<223> Peptide

<400> 96
Lys Ile Glu Glu Ile Glu
1               5

<210> 97
<211> 43
<212> PRT
<213> Artificial Sequence

<220>
<223> Peptide

<400> 97
Gly Gly Ile Glu Lys Lys Ile Glu Glu Ile Glu Lys Lys Ile Glu Glu
1               5                   10                  15
Ile Glu Lys Lys Ile Glu Glu Ile Glu Lys Leu Leu Gln Leu Thr Val
            20                  25                  30
Trp Gly Ile Lys Gln Leu Gln Ala Arg Ile Leu
        35                  40

<210> 98
<211> 41
<212> PRT
<213> Artificial Sequence

<220>
<223> Peptide

<400> 98
Ile Glu Lys Lys Ile Glu Glu Ile Glu Lys Lys Ile Glu Glu Ile Glu
1               5                   10                  15
Lys Lys Ile Glu Glu Ile Glu Lys Leu Leu Gln Leu Thr Val Trp Gly
            20                  25                  30
Ile Lys Gln Leu Gln Ala Arg Ile Leu
        35                  40

<210> 99
<211> 7
<212> PRT
<213> Artificial Sequence

<220>
<223> Peptide
<400> 99
Ile Glu Lys Lys Ile Glu Ala
1               5

<210> 100
<211> 7

<212> PRT
<213> Artificial Sequence

<220>
<223> Peptide

<400> 100
Ile Glu Lys Lys Ile Glu Glu
1               5